# EUROPÄISCHE PATENTSCHRIFT

(11) **EP 4 242 286 B1**
(45) Veröffentlichungstag und Bekanntmachung des Hinweises auf die Patenterteilung: **29.04.2026**
(21) Anmeldenummer: 23176636.1
(22) Anmeldetag: 20.11.2018
(51) Int. Cl.: C09K 11/06, C08G 73/02, C07D 407/12, C07D 409/12, C07D 409/14, C07D 209/86, C07D 209/88, C07D 405/12, C07D 405/14, H10K 85/60

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 23.11.2017 EP 17203293
(43) Veröffentlichungstag der Anmeldung: 13.09.2023
(62) Teilanmeldung aus: 18807070.0
(73) Patentinhaber: Merck Patent GmbH, 64293 Darmstadt (DE)
(72) Erfinder: Wirges, Christian, 64293 DARMSTADT (DE); Mujica-Fernaud, Teresa, 64293 Darmstadt (DE); Montenegro, Elvira, 64293 Darmstadt (DE); Voges, Frank, 64293 Darmstadt (DE); Maier-Flaig, Florian, 64293 Darmstadt (DE); Eberle, Thomas, 64293 Darmstadt (DE)
(74) Vertreter: Merck Patent Association

(56) Entgegenhaltungen:
- EP-A1- 2 348 017
- WO-A1-2014/129764
- WO-A1-2017/043917
- WO-A1-2017/052212

## Beschreibung

Die vorliegende Anmeldung betrifft Triarylamin-Verbindungen gemäß einer weiter unten definierten Formel (I). Diese Verbindungen eignen sich zur Verwendung in elektronischen Vorrichtungen. Weiterhin betrifft die vorliegende Anmeldung Verfahren zur Herstellung der genannten Verbindungen, sowie elektronische Vorrichtungen enthaltend die genannten Verbindungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Emissionsschichten und Schichten mit lochtransportierender Funktion. Zur Verwendung in diesen Schichten werden weiterhin neue Verbindungen gesucht, insbesondere lochtransportierende Verbindungen und Verbindungen, die als Matrixmaterial, insbesondere für phosphoreszierende Emitter, in einer emittierenden Schicht dienen können.

Im Stand der Technik sind verschiedene Triarylamin-Verbindungen als Lochtransportmaterialien für elektronische Vorrichtungen bekannt.

Ebenfalls bekannt ist die Verwendung von bestimmten Triarylamin-Verbindungen als Matrixmaterialien in emittierenden Schichten.

EP 2 348 017 A1 offenbart Triarylamin-Verbindungen der allgemeinen Formel (1) 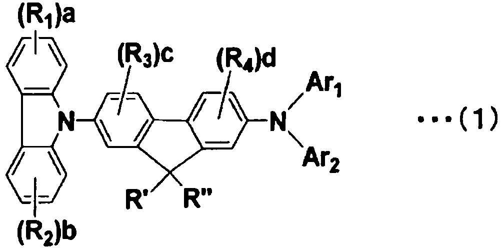 und, unter anderem, eine Verbindung der Formel [B-36]

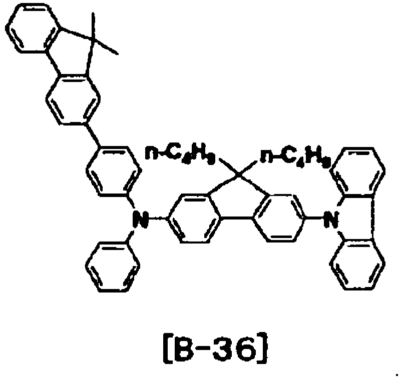

Es besteht jedoch weiter Bedarf an alternativen Verbindungen, die zur Verwendung in elektronischen Vorrichtungen geeignet sind.

Es besteht auch Verbesserungsbedarf bezüglich der Leistungsdaten bei Verwendung in elektronischen Vorrichtungen, insbesondere bezüglich der Betriebsspannung, der Lebensdauer und der Effizienz. Weiterhin besteht Verbesserungsbedarf bezüglich der Prozessierbarkeit der Materialien, ihrer Glasübergangstemperatur, ihrer Löslichkeit, ihrer Stabilität in Lösung, und ihrem Brechungsindex.

Nun wurde gefunden, dass sich bestimmte Triarylamin-Verbindungen hervorragend zur Verwendung in elektronischen Vorrichtungen eignen, insbesondere zur Verwendung in OLEDs, nochmals insbesondere darin zur Verwendung als Lochtransportmaterialien und zur Verwendung als Matrixmaterialien für phosphoreszierende Emitter.

Gegenstand der vorliegenden Anmeldung sind damit Verbindungen gemäß einer Formel (I) 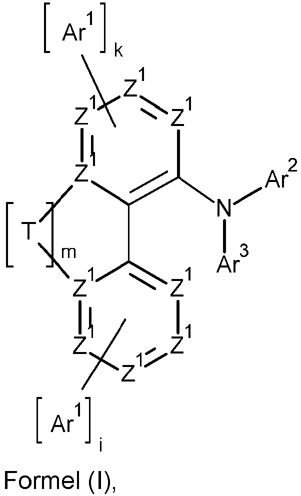
wobei die Untereinheit 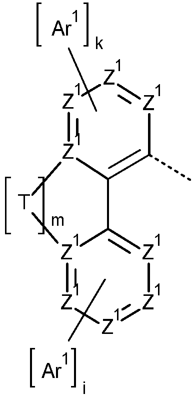
der Formel (I) gewählt ist aus den folgenden Strukturen:

| | | |
|---|---|---|
| 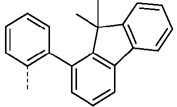 | | |
| Formel (I-A-45) | | |
| 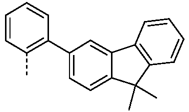 | 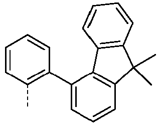 | |
| Formel (I-A-46) | Formel (I-A-47) | |
| 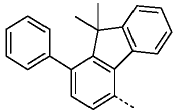 | 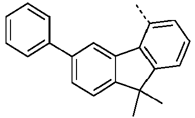 | 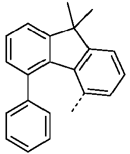 |
| Formel (I-A-49) | Formel (I-A-50) | Formel (I-A-51) |
| 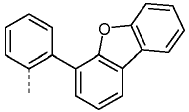 | | 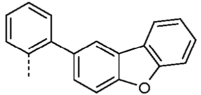 |
| Formel (I-A-53) | | Formel (I-A-54) |
| 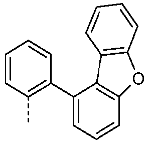 | | 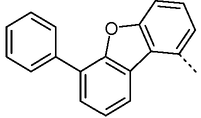 |
| Formel (I-A-55) | | Formel (I-A-57) |
| 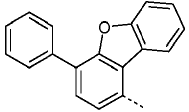 | 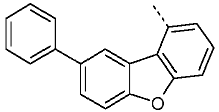 | 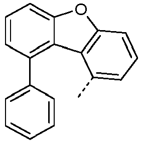 |
| Formel (I-A-58) | Formel (I-A-59) | Formel (I-A-60) |
| 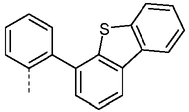 | | 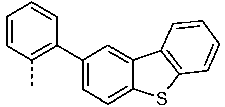 |
| Formel (I-A-62) | | Formel (I-A-63) |
| 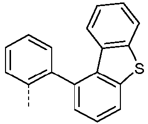 | | 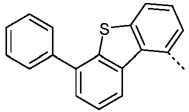 |
| Formel (I-A-64) | | Formel (I-A-66) |
| 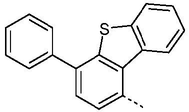 | 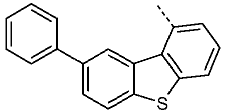 | 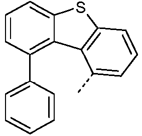 |
| Formel (I-A-67) | Formel (I-A-68) | Formel (I-A-69) |
| 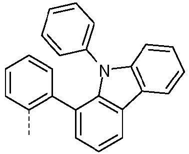 | | 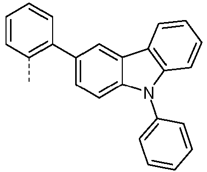 |
| Formel (I-A-71) | | Formel (I-A-72) |
| 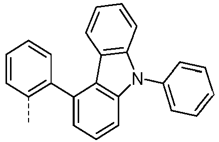 | | 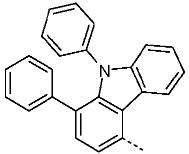 |
| Formel (I-A-73) | | Formel (I-A-75) |
| 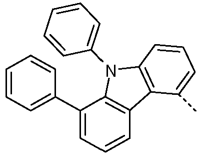 | 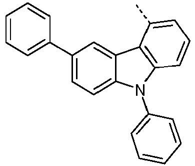 | 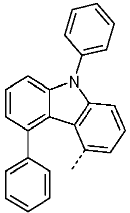 |
| Formel (I-A-76) | Formel (I-A-77) | Formel (I-A-78) |
| 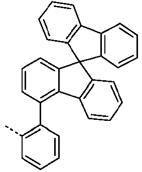 | | 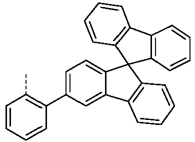 |
| Formel (I-A-80) | | Formel (I-A-81) |
| 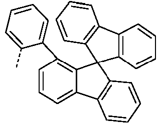 | | 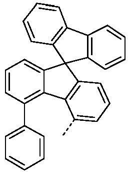 |
| Formel (I-A-82) | | Formel (I-A-84) |
| 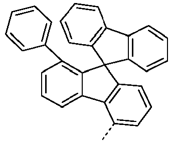 | 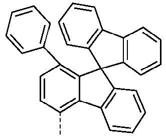 | 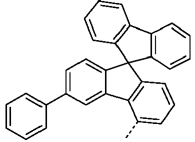 |
| Formel (I-A-85) | Formel (I-A-86) | Formel (I-A-87) |
| 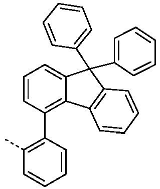 | | 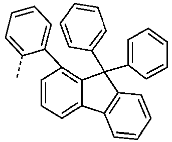 |
| Formel (I-A-88) | | Formel (I-A-90) |
| 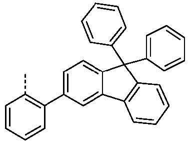 | | 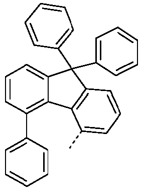 |
| Formel (I-A-91) | | Formel (I-A-93) |
| 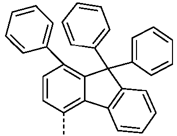 | 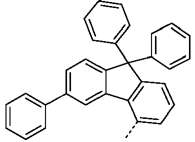 | 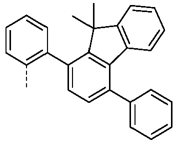 |
| Formel (I-A-94) | Formel (I-A-95) | Formel (I-A-96) |
| 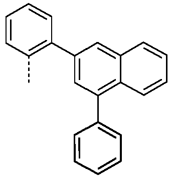 | | 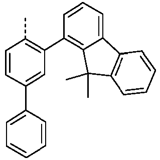 |
| Formel (I-A-109) | | Formel (I-A-111) |
| 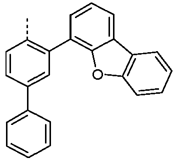 | 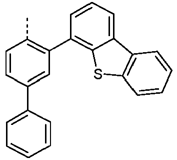 | 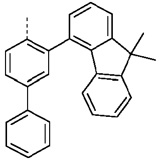 |
| Formel (I-A-112) | Formel (I-A-113) | Formel (I-A-114) |
| 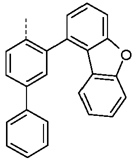 | 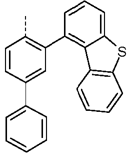 | |
| Formel (I-A-115) | Formel (I-A-116) | |
| 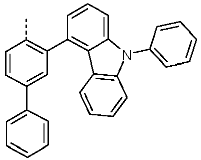 | 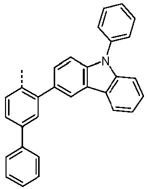 | |
| Formel (I-A-118) | Formel (I-A-119) | |
| 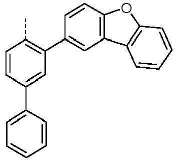 | 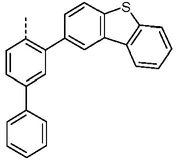 | |
| Formel (I-A-121) | Formel (I-A-122) | |

wobei die gestrichelte Linie die Bindung an das N-Atom in Formel (I) darstellt, und
wobei für die auftretenden Variablen gilt:
- Ar²: entspricht einer Formel (A) oder (B) 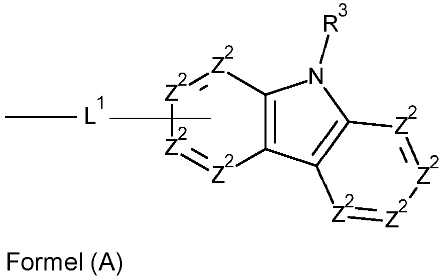 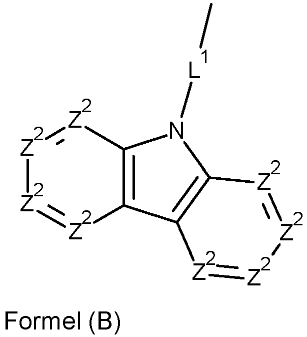
- Z²: ist bei jedem Auftreten gleich oder verschieden CR³ oder N, wobei Z² gleich C ist, wenn eine Gruppe L¹ daran gebunden ist;
- L¹: ist eine Einfachbindung, oder ein aromatisches Ringsystem mit 6 bis 30 aromatischen Ringatomen, das mit einem oder mehreren Resten R³ substituiert sein kann, oder ein heteroaromatisches Ringsystem mit 5 bis 30 aromatischen Ringatomen, das mit einem oder mehreren Resten R³ substituiert sein kann;
- Ar³: entspricht einer Formel (A), einer Formel (B) oder ist ein aromatisches Ringsystem mit 6 bis 30 aromatischen Ringatomen, das mit einem oder mehreren Resten R⁴ substituiert sein kann, oder ein heteroaromatisches Ringsystem mit 5 bis 30 aromatischen Ringatomen, das mit einem oder mehreren Resten R⁴ substituiert sein kann;
- R³, R⁴: sind bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ bzw. R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch-R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
- R⁵: ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C≡C-, Si(R⁶)2, C=O, C=NR⁶, -C(=O)O-, -C(=O)NR⁶-, NR⁶, P(=O)(R⁶), -O-, -S-, SO oder SO₂ ersetzt sein können;
- R⁶: ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugte Ausführungsformen der Formel (A) sind die Formeln (A-1) bis (A-10)

| | |
|---|---|
| 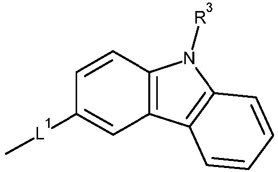 | 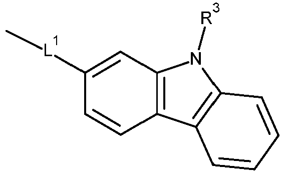 |
| Formel (A-1) | Formel (A-2) |
| 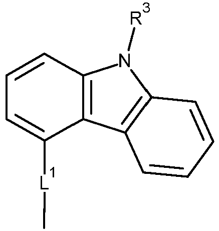 | 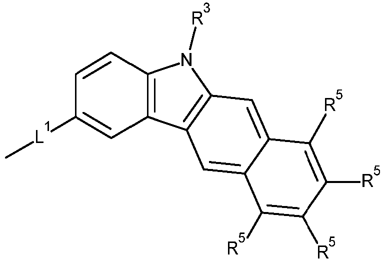 |
| Formel (A-3) | Formel (A-4) |
| 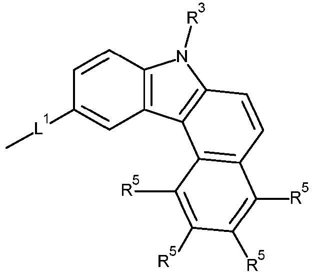 | 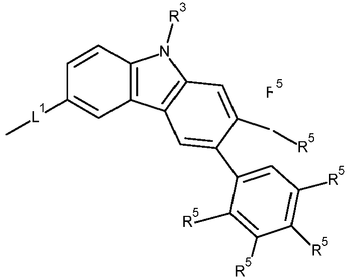 |
| Formel (A-5) | Formel (A-6) |
| 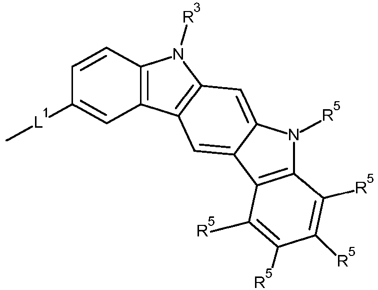 | 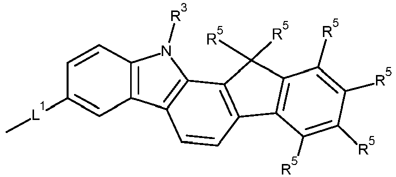 |
| Formel (A-7) | Formel (A-8) |
| 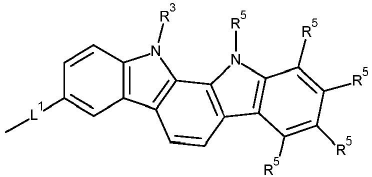 | 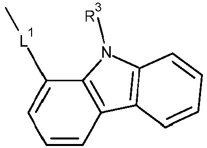 |
| Formel (A-9) | Formel (A-10) |

wobei die auftretenden Variablen definiert sind wie oben, und wobei die Carbazoleinheiten an den freien Positionen ihrer beiden Benzolringe jeweils mit einem oder mehreren Resten R³ substituiert sein können.

Bevorzugte Ausführungsformen der Formel (B) sind die Formeln (B-1) bis (B-7)

| | |
|---|---|
| 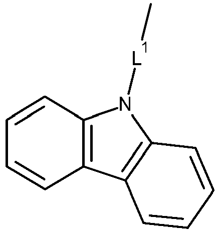 | 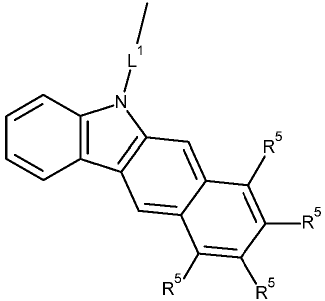 |
| Formel (B-1) | Formel (B-2) |
| 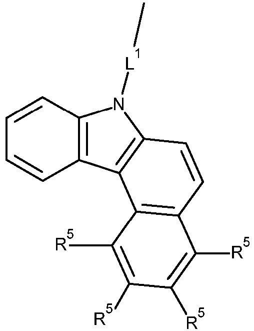 | 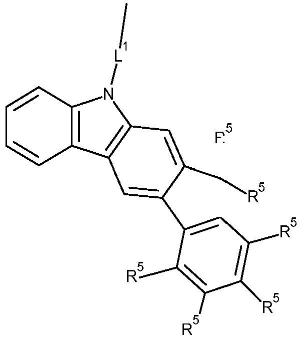 |
| Formel (B-3) | Formel (B-4) |
| 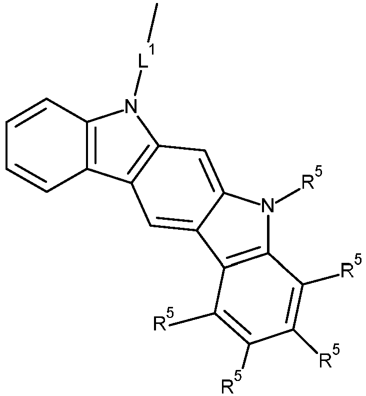 | 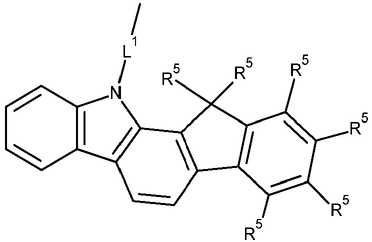 |
| Formel (B-5) | Formel (B-6) |
| 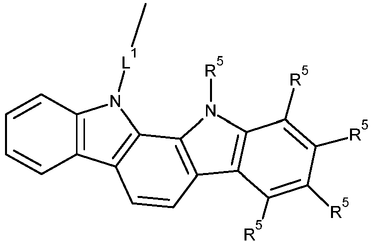 | |
| Formel (B-7) | |

wobei die auftretenden Variablen definiert sind wie oben, und wobei die Carbazoleinheiten an den freien Positionen ihrer beiden Benzolringe jeweils mit einem oder mehreren Resten R³ substituiert sein können.

Ar² entspricht bevorzugt der oben genannten Formel (A), besonders bevorzugt einer der Formeln (A-1) bis (A-3).

Bevorzugte Ausführungsformen der Gruppen Ar² sind in der folgenden Tabelle abgebildet:

| | | |
|---|---|---|
| 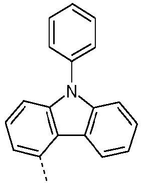 | 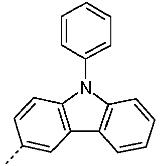 | 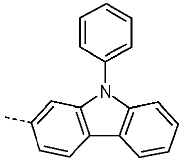 |
| Ar²-1 | Ar²-2 | Ar²-3 |
| 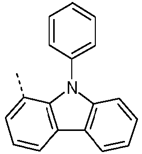 | 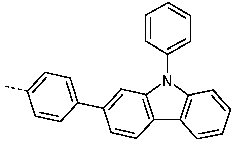 | 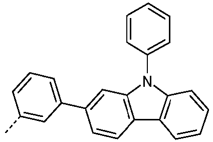 |
| Ar²-4 | Ar²-5 | Ar²-6 |
| 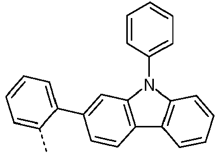 | 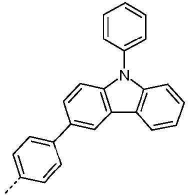 | 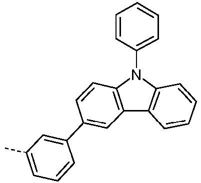 |
| Ar²-7 | Ar²-8 | Ar²-9 |
| 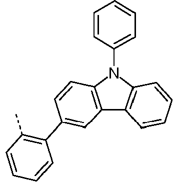 | 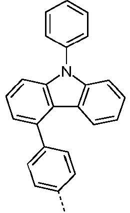 | 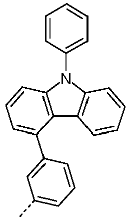 |
| Ar²-10 | Ar²-11 | Ar²-12 |
| 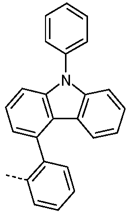 | 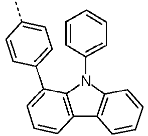 | 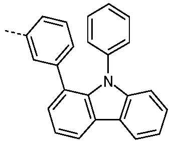 |
| Ar²-13 | Ar²-14 | Ar²-15 |
| 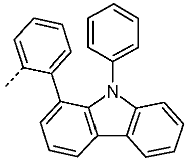 | 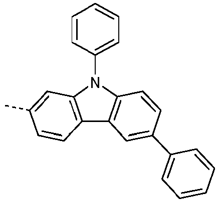 | 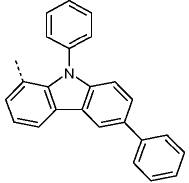 |
| Ar²-16 | Ar²-17 | Ar²-18 |
| 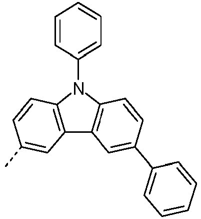 | 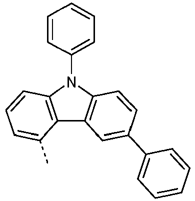 | 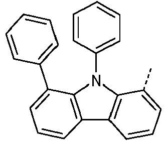 |
| Ar²-19 | Ar²-20 | Ar²-21 |
| 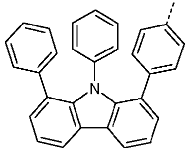 | 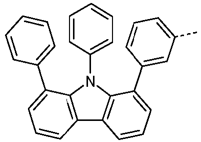 | 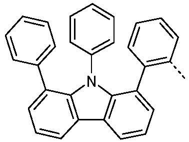 |
| Ar²-22 | Ar²-23 | Ar²-24 |
| 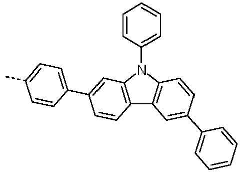 | 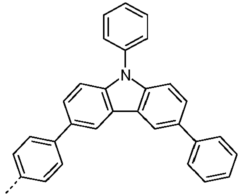 | 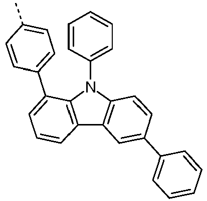 |
| Ar²-25 | Ar²-26 | Ar²-27 |
| 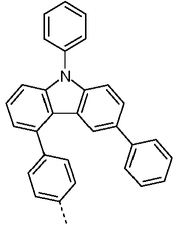 | 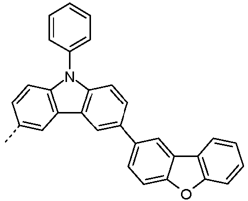 | 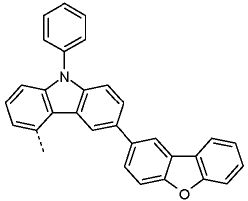 |
| Ar²-28 | Ar²-29 | Ar²-30 |
| 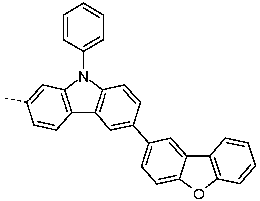 | 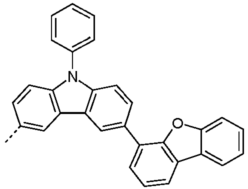 | 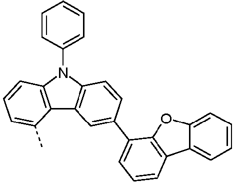 |
| Ar²-31 | Ar²-32 | Ar²-33 |
| 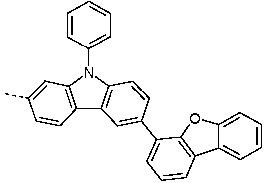 | 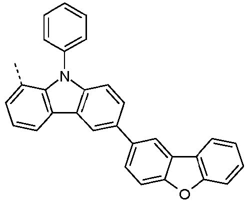 | 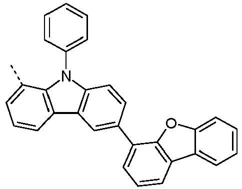 |
| Ar²-34 | Ar²-35 | Ar²-36 |
| 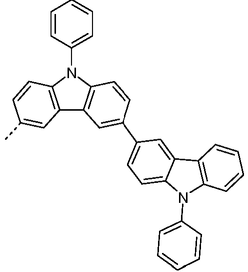 | 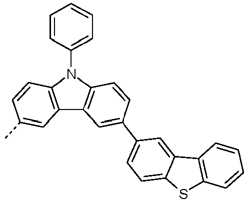 | 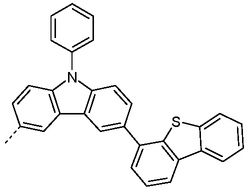 |
| Ar²-37 | Ar²-38 | Ar²-39 |
| 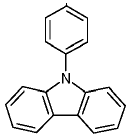 | 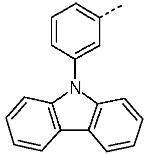 | 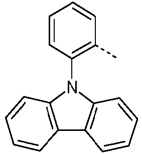 |
| Ar²-40 | Ar²-41 | Ar²-42 |
| 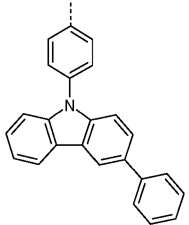 | 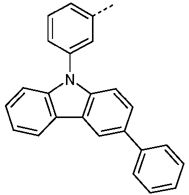 | 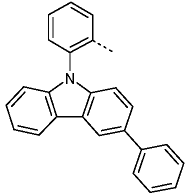 |
| Ar²-43 | Ar²-44 | Ar²-45 |
| 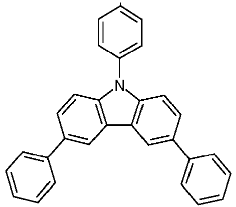 | 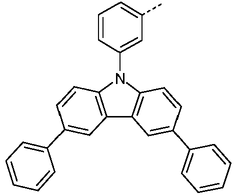 | 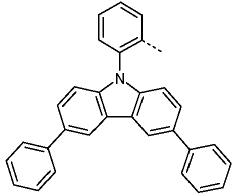 |
| Ar²-46 | Ar²-47 | Ar²-48 |
| 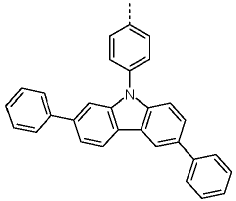 | 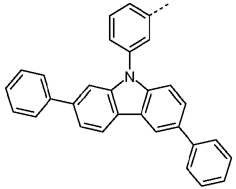 | 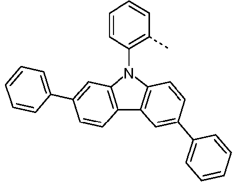 |
| Ar²-49 | Ar²-50 | Ar²-51 |
| 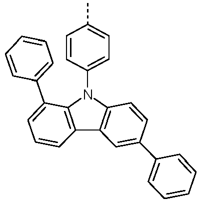 | 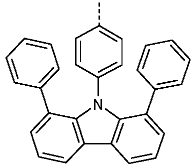 | 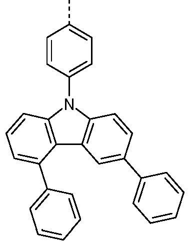 |
| Ar²-52 | Ar²-53 | Ar²-54 |
| 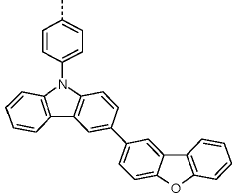 | 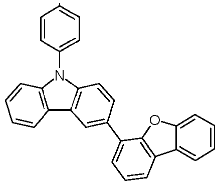 | 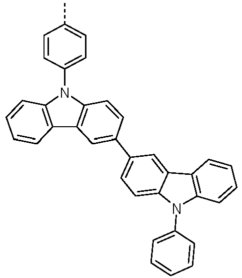 |
| Ar²-55 | Ar²-56 | Ar²-57 |
| 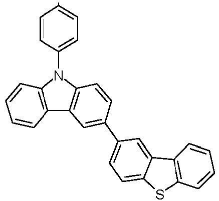 | 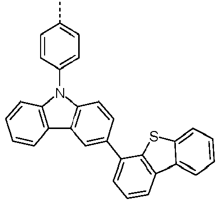 | 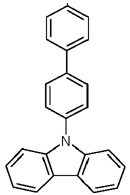 |
| Ar²-58 | Ar²-59 | Ar²-60 |
| 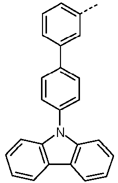 | 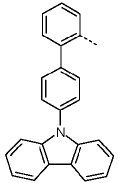 | 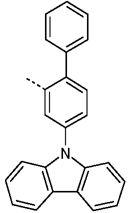 |
| Ar²-61 | Ar²-62 | Ar²-63 |
| 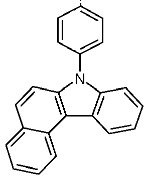 | 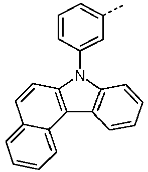 | 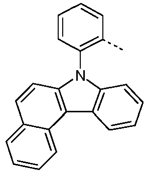 |
| Ar²-64 | Ar²-65 | Ar²-66 |
| 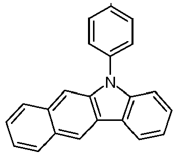 | 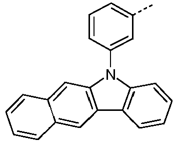 | 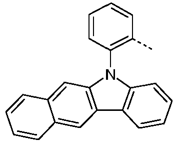 |
| Ar²-67 | Ar²-68 | Ar²-69 |
| 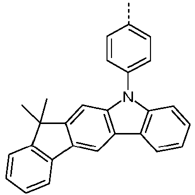 | 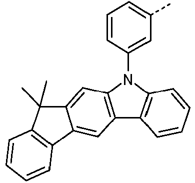 | 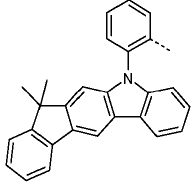 |
| Ar²-70 | Ar²-71 | Ar²-72 |
| 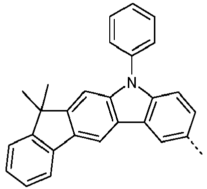 | 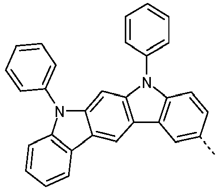 | 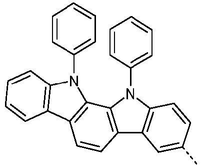 |
| Ar²-73 | Ar²-74 | Ar²-75 |
| 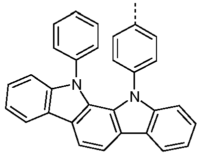 | | |
| Ar²-76 | | |

Z² ist bevorzugt bei jedem Auftreten gleich CR³, wobei Z² gleich C ist, wenn eine Gruppe L¹ daran gebunden ist.

L¹ ist bevorzugt gewählt aus aromatischen Ringsystemen mit 6 bis 30 aromatischen Ringatomen. L¹ ist besonders bevorzugt gewählt aus Einfachbindung, Benzol, Naphthalin, para-Biphenyl, meta-Biphenyl, ortho-Biphenyl, Terphenyl, Dibenzofuran, Carbazol, Dibenzothiophen, Pyridin, Pyrimidin, Pyrazin, Pyridazin, Triazin und Fluoren, ganz besonders bevorzugt gewählt aus Einfachbindung und Phenyl, wobei die genannten Gruppen jeweils mit einem oder mehreren Resten R³ substituiert sein können.

Bevorzugte Gruppen L¹ sind in der folgenden Tabelle abgebildet:

| | | |
|---|---|---|
| 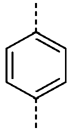 | 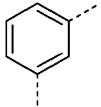 | 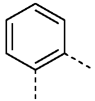 |
| (L¹-1) | (L¹-2) | (L¹-3) |
| 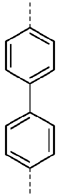 | 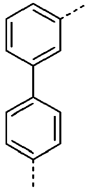 | 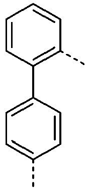 |
| (L¹-4) | (L¹-5) | (L¹-6) |
| 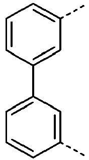 | 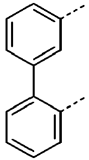 | 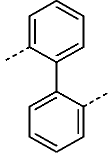 |
| (L¹-7) | (L¹-8) | (L¹-9) |
| 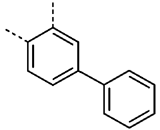 | 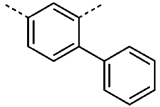 | 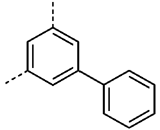 |
| (L¹-10) | (L¹-11) | (L¹-12) |
| 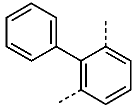 | 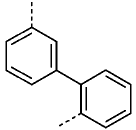 | 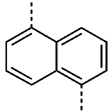 |
| (L¹-13) | (L¹-14) | (L¹-15) |
| 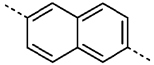 | 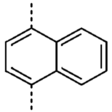 | 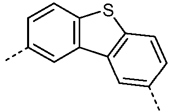 |
| (L¹-16) | (L¹-17) | (L¹-18) |
| 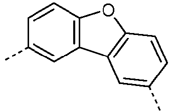 | 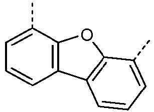 | 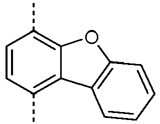 |
| (L¹-19) | (L¹-20) | (L¹-21) |
| 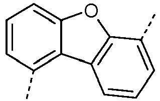 | 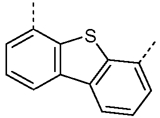 | 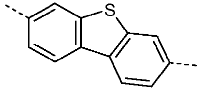 |
| (L¹-22) | (L¹-23) | (L¹-24) |
| 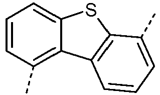 | 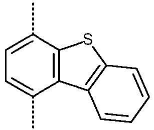 | 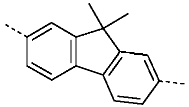 |
| (L¹-25) | (L¹-26) | (L¹-27) |
| 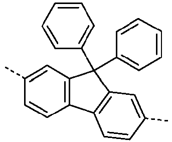 | 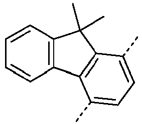 | 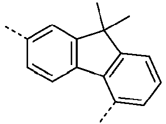 |
| (L¹-28) | (L¹-29) | (L¹-30) |
| 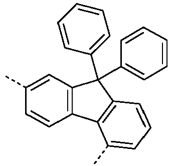 | 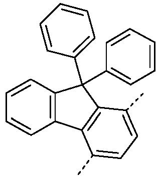 | 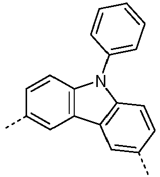 |
| (L¹-31) | (L¹-32) | (L¹-33) |
| 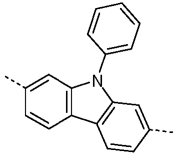 | 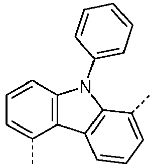 | 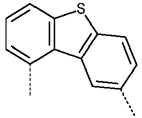 |
| (L¹-34) | (L¹-35) | (L¹-36) |
| 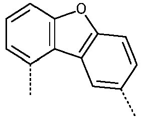 | | |
| (L¹-37) | | |

Ar³ ist bevorzugt ein aromatisches Ringsystem mit 6 bis 30 aromatischen Ringatomen, das mit einem oder mehreren Resten R⁴ substituiert sein kann. Ar³ ist besonders bevorzugt gewählt aus Phenyl, Biphenyl, Terphenyl, Fluorenyl, Fluorenyl-Phenyl, Naphthyl, Naphthyl-Phenyl, Spirobifluorenyl, Spirobifluorenyl-Phenyl, Pyridyl, Pyrimidyl, Triazinyl, Dibenzofuranyl, Dibenzofuranyl-Phenyl, benzo-kondensiertem Dibenzofuranyl, Dibenzothiophenyl, Dibenzothiophenyl-Phenyl, benzo-kondensiertem Dibenzothiophenyl, Carbazolyl, Carbazolyl-Phenyl und benzo-kondensiertem Carbazolyl, und Kombinationen aus zwei, drei oder vier dieser Gruppen, wobei die genannten Gruppen jeweils mit einem oder mehreren Resten R⁴ substituiert sein können.

Die Gruppe Ar³ entspricht bevorzugt nicht einer der Formeln (A) und (B).

Bevorzugte Ausführungsformen von Ar³ sind im Folgenden abgebildet:

| | | |
|---|---|---|
| 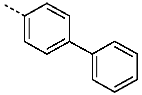 | 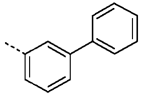 | 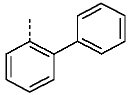 |
| Ar³-1 | Ar³-2 | Ar³-3 |
| 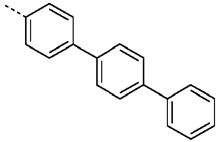 | 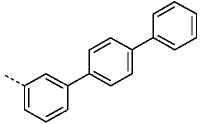 | 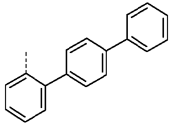 |
| Ar³-4 | Ar³-5 | Ar³-6 |
| 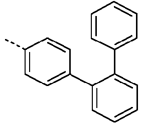 | 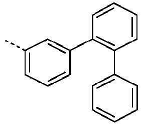 | 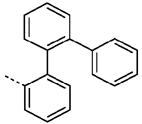 |
| Ar³-7 | Ar³-8 | Ar³-9 |
| 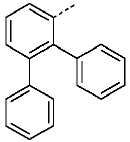 | 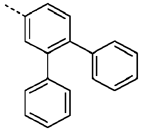 | 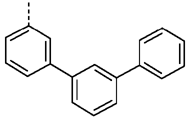 |
| Ar³-10 | Ar³-11 | Ar³-12 |
| 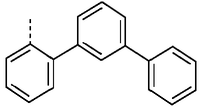 | 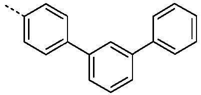 | 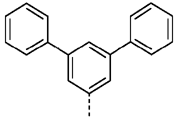 |
| Ar³-13 | Ar³-14 | Ar³-15 |
| 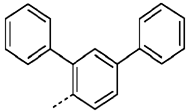 | 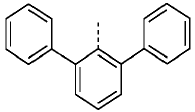 | 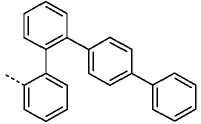 |
| Ar³-16 | Ar³-17 | Ar³-18 |
| 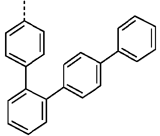 | 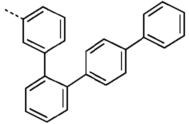 | 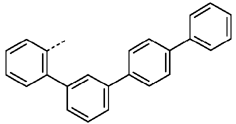 |
| Ar³-19 | Ar³-20 | Ar³-21 |
| 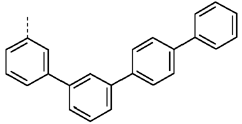 | 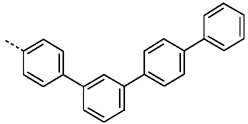 | 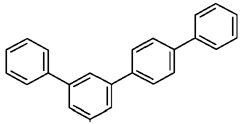 |
| Ar³-22 | Ar³-23 | Ar³-24 |
| 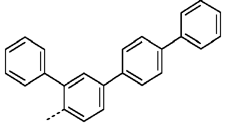 | 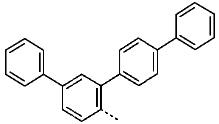 | 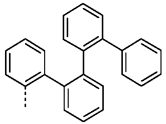 |
| Ar³-25 | Ar³-26 | Ar³-27 |
| 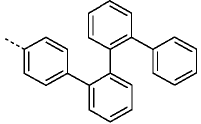 | 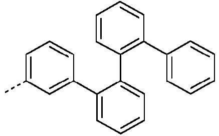 | 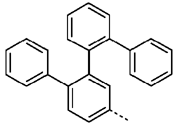 |
| Ar³-28 | Ar³-29 | Ar³-30 |
| 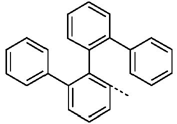 | 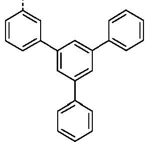 | 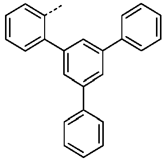 |
| Ar³-31 | Ar³-32 | Ar³-33 |
| 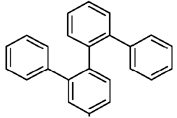 | 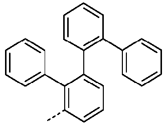 | 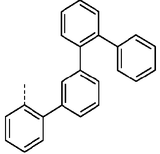 |
| Ar³-34 | Ar³-35 | Ar³-36 |
| 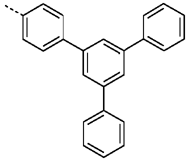 | 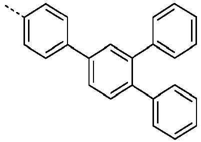 | 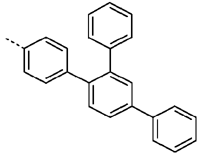 |
| Ar³-37 | Ar³-38 | Ar³-39 |
| 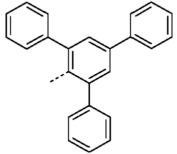 | 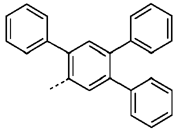 | 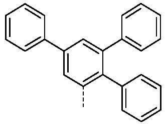 |
| Ar³-40 | Ar³-41 | Ar³-42 |
| 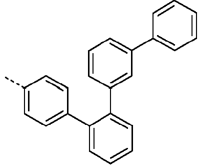 | 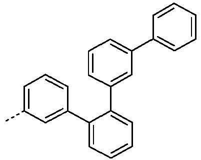 | 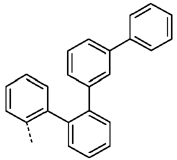 |
| Ar³-43 | Ar³-44 | Ar³-45 |
| 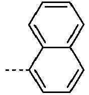 | 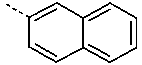 | 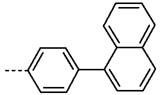 |
| Ar³-46 | Ar³-47 | Ar³-48 |
| 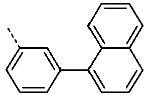 | 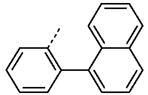 | 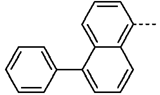 |
| Ar³-49 | Ar³-50 | Ar³-51 |
| 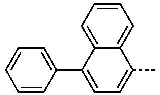 | 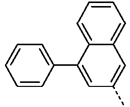 | 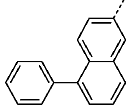 |
| Ar³-52 | Ar³-53 | Ar³-54 |
| 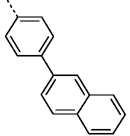 | 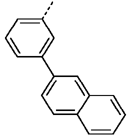 | 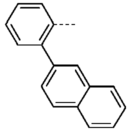 |
| Ar³-55 | Ar³-56 | Ar³-57 |
| 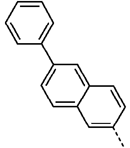 | 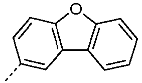 | 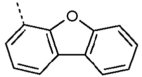 |
| Ar³-58 | Ar³-59 | Ar³-60 |
| 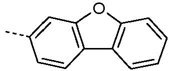 | 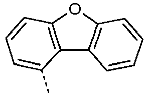 | 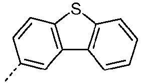 |
| Ar³-61 | Ar³-62 | Ar³-63 |
| 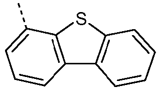 | 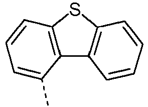 | 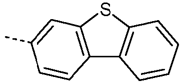 |
| Ar³-64 | Ar³-65 | Ar³-66 |
| 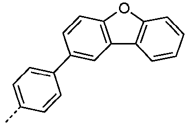 | 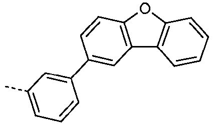 | 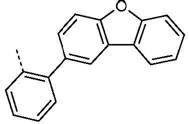 |
| Ar³-67 | Ar³-68 | Ar³-69 |
| 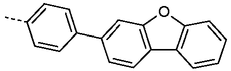 | 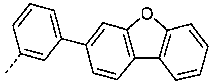 | 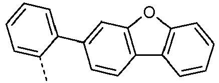 |
| Ar³-70 | Ar³-71 | Ar³-72 |
| 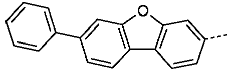 | 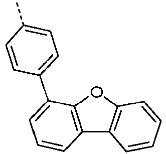 | 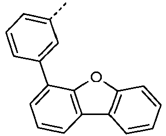 |
| Ar³-73 | Ar³-74 | Ar³-75 |
| 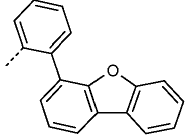 | 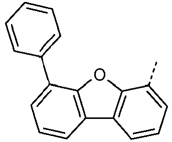 | 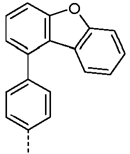 |
| Ar³-76 | Ar³-77 | Ar³-78 |
| 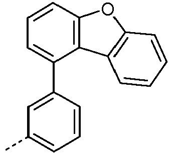 | 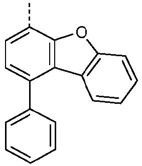 | 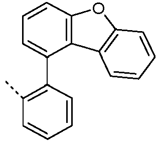 |
| Ar³-79 | Ar³-80 | Ar³-81 |
| 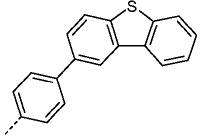 | 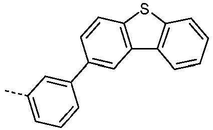 | 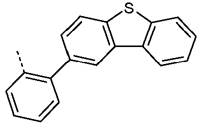 |
| Ar³-82 | Ar³-83 | Ar³-84 |
| 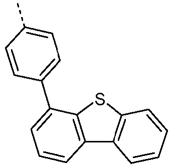 | 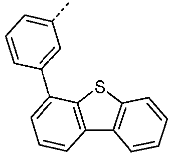 | 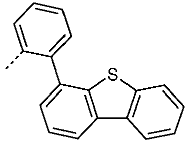 |
| Ar³-85 | Ar³-86 | Ar³-87 |
| 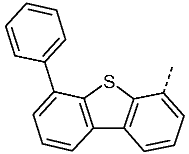 | 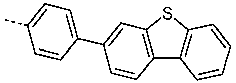 | 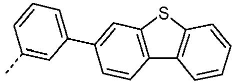 |
| Ar³-88 | Ar³-89 | Ar³-90 |
| 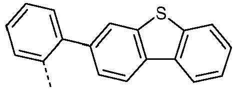 | 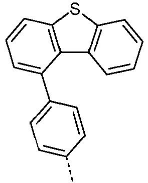 | 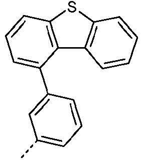 |
| Ar³-91 | Ar³-92 | Ar³-93 |
| 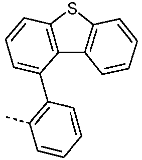 | 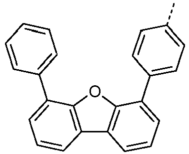 | 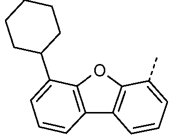 |
| Ar³-94 | Ar³-95 | Ar³-96 |
| 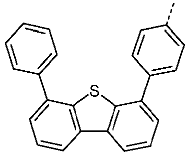 | 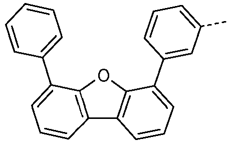 | 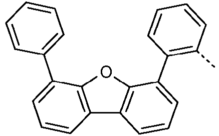 |
| Ar³-94 | Ar³-95 | Ar³-96 |
| 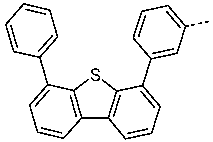 | 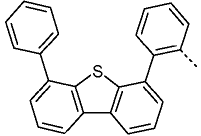 | 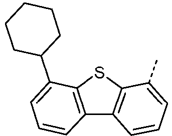 |
| Ar³-97 | Ar³-98 | Ar³-99 |
| 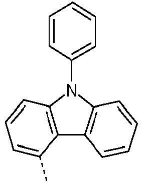 | 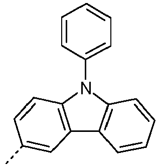 | 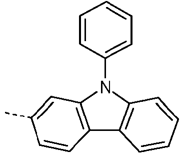 |
| Ar³-100 | Ar³-101 | Ar³-102 |
| 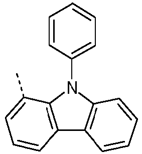 | 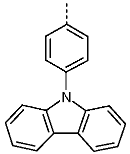 | 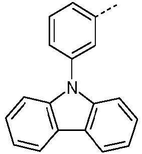 |
| Ar³-103 | Ar³-104 | Ar³-105 |
| 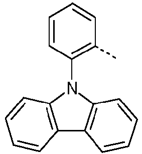 | 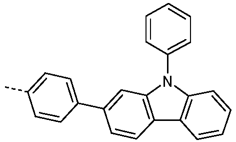 | 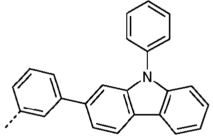 |
| Ar³-106 | Ar³-107 | Ar³-108 |
| 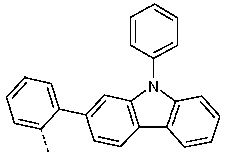 | 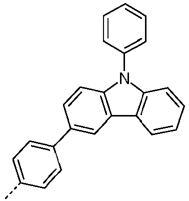 | 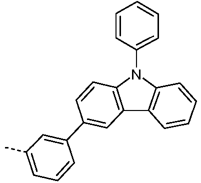 |
| Ar³-109 | Ar³-110 | Ar³-111 |
| 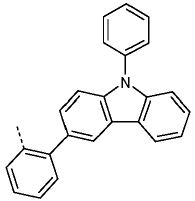 | 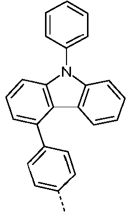 | 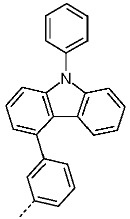 |
| Ar³-112 | Ar³-113 | Ar³-114 |
| 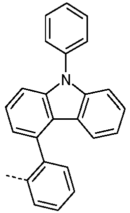 | 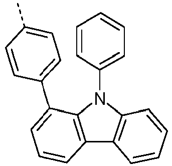 | 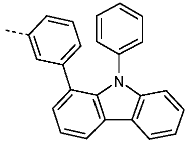 |
| Ar³-115 | Ar³-116 | Ar³-117 |
| 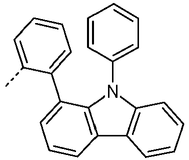 | 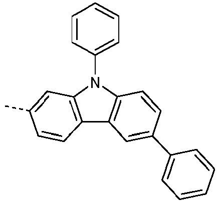 | 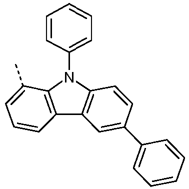 |
| Ar³-118 | Ar³-119 | Ar³-120 |
| 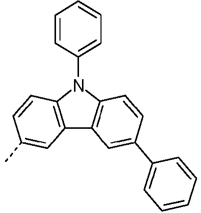 | 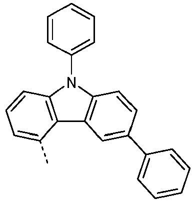 | 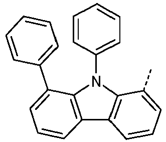 |
| Ar³-121 | Ar³-122 | Ar³-123 |
| 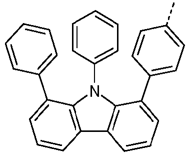 | 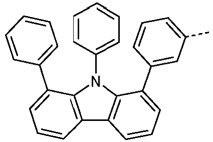 | 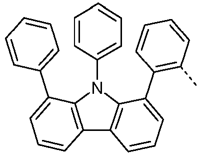 |
| Ar³-124 | Ar³-125 | Ar³-126 |
| 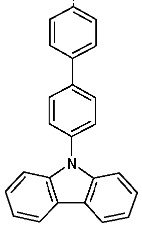 | 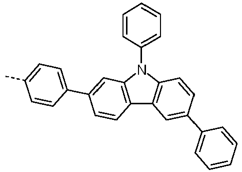 | 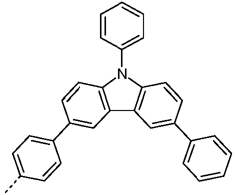 |
| Ar³-127 | Ar³-128 | Ar³-129 |
| 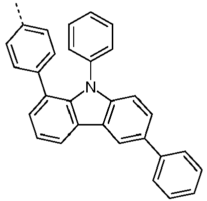 | 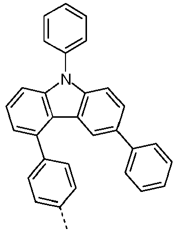 | 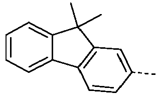 |
| Ar³-130 | Ar³-131 | Ar³-132 |
| 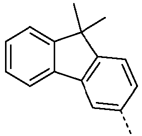 | 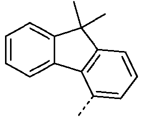 | 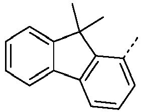 |
| Ar³-133 | Ar³-134 | Ar³-135 |
| 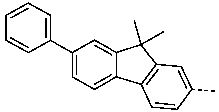 | 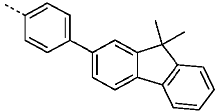 | 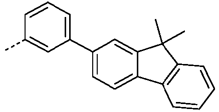 |
| Ar³-136 | Ar³-137 | Ar³-138 |
| 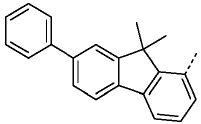 | 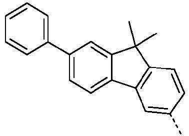 | 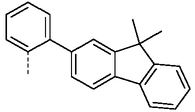 |
| Ar³-139 | Ar³-140 | Ar³-141 |
| 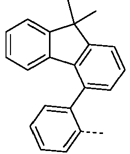 | 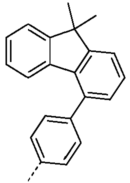 | 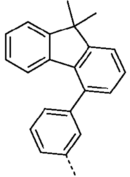 |
| Ar³-142 | Ar³-143 | Ar³-144 |
| 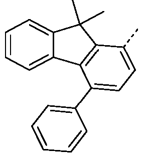 | 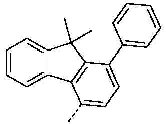 | 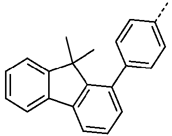 |
| Ar³-145 | Ar³-146 | Ar³-147 |
| 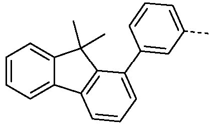 | 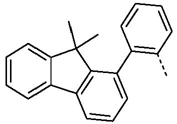 | 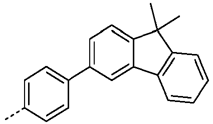 |
| Ar³-148 | Ar³-149 | Ar³-150 |
| 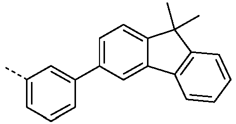 | 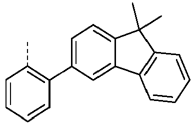 | 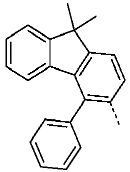 |
| Ar³-151 | Ar³-152 | Ar³-153 |
| 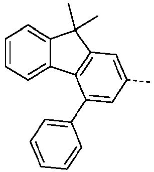 | 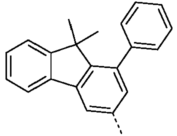 | 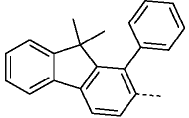 |
| Ar³-154 | Ar³-155 | Ar³-156 |
| 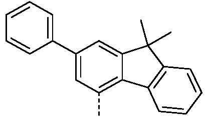 | 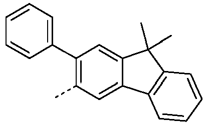 | 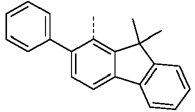 |
| Ar³-157 | Ar³-158 | Ar³-159 |
| 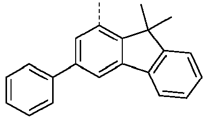 | 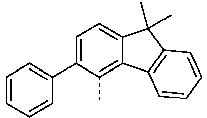 | 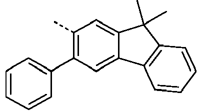 |
| Ar³-160 | Ar³-161 | Ar³-162 |
| 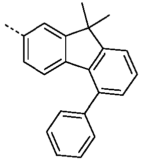 | 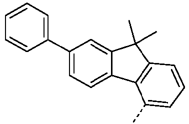 | 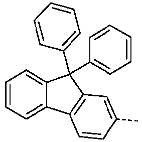 |
| Ar³-163 | Ar³-164 | Ar³-165 |
| 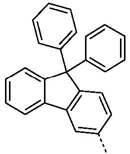 | 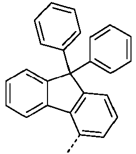 | 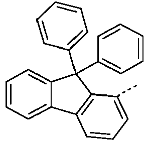 |
| Ar³-166 | Ar³-167 | Ar³-168 |
| 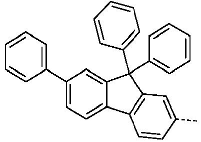 | 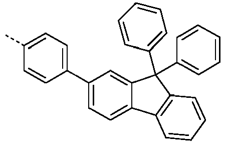 | 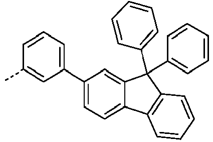 |
| Ar³-169 | Ar³-170 | Ar³-171 |
| 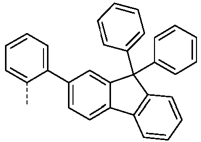 | 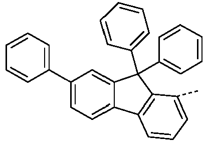 | 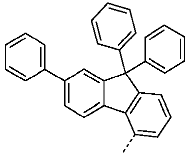 |
| Ar³-172 | Ar³-173 | Ar³-174 |
| 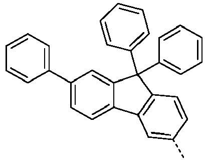 | 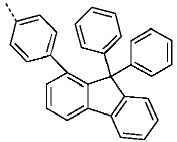 | 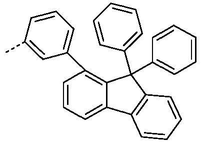 |
| Ar³-175 | Ar³-176 | Ar³-177 |
| 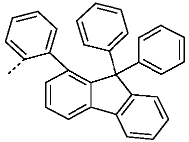 | 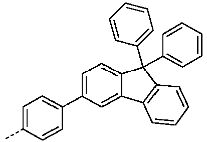 | 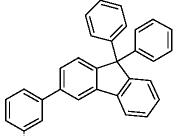 |
| Ar³-178 | Ar³-179 | Ar³-180 |
| 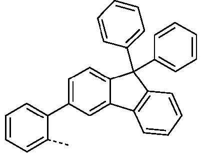 | 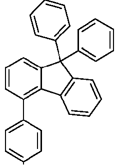 | 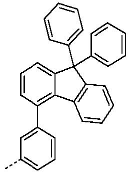 |
| Ar³-181 | Ar³-182 | Ar³-183 |
| 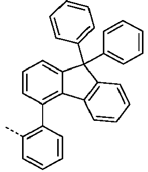 | 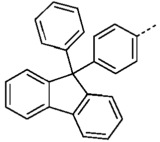 | 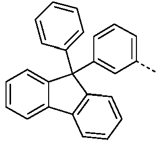 |
| Ar³-184 | Ar³-185 | Ar³-186 |
| 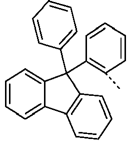 | 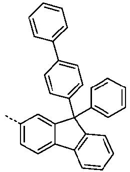 | 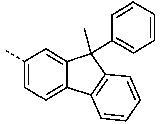 |
| Ar³-187 | Ar³-188 | Ar³-189 |
| 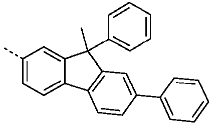 | 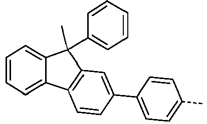 | 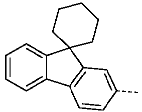 |
| Ar³-190 | Ar³-191 | Ar³-192 |
| 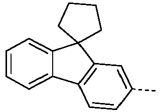 | 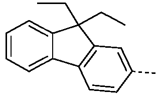 | 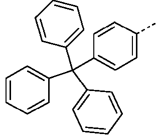 |
| Ar³-193 | Ar³-194 | Ar³-195 |
| 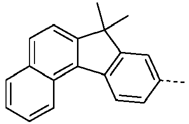 | 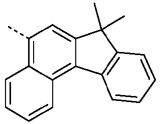 | 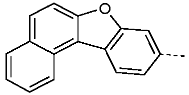 |
| Ar³-196 | Ar³-197 | Ar³-198 |
| 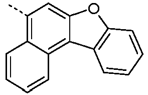 | 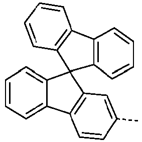 | 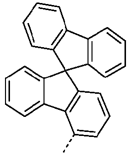 |
| Ar³-199 | Ar³-200 | Ar³-201 |
| 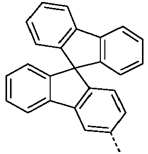 | 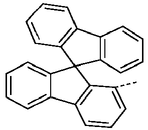 | 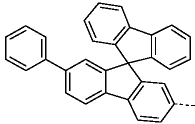 |
| Ar³-202 | Ar³-203 | Ar³-204 |
| 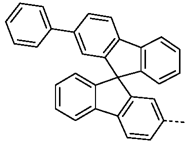 | 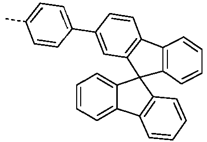 | 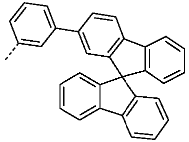 |
| Ar³-205 | Ar³-206 | Ar³-207 |
| 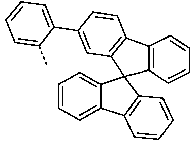 | 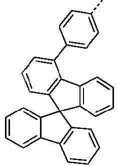 | 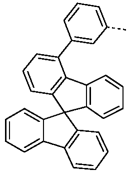 |
| Ar³-208 | Ar³-209 | Ar³-210 |
| 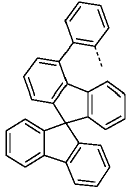 | 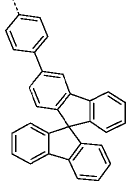 | 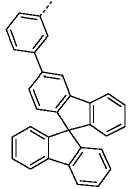 |
| Ar³-211 | Ar³-212 | Ar³-213 |
| 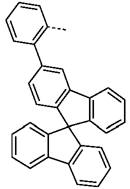 | 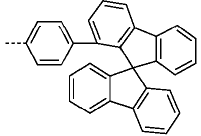 | 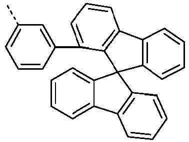 |
| Ar³-214 | Ar³-215 | Ar³-216 |
| 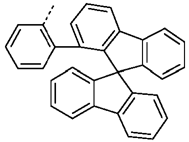 | 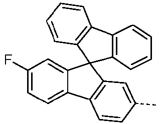 | 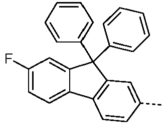 |
| Ar³-217 | Ar³-218 | Ar³-219 |
| 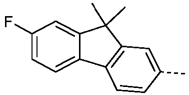 | 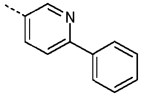 | 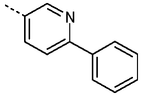 |
| Ar³-220 | Ar³-221 | Ar³-222 |
| 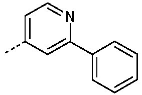 | 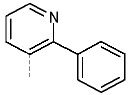 | 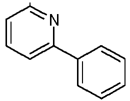 |
| Ar³-223 | Ar³-224 | Ar³-225 |
| 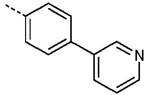 | 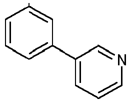 | 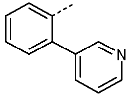 |
| Ar³-226 | Ar³-227 | Ar³-228 |
| 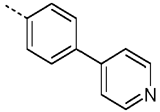 | 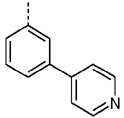 | 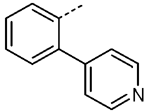 |
| Ar³-229 | Ar³-230 | Ar³-231 |
| 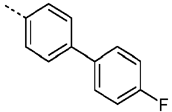 | 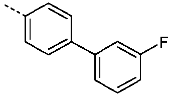 | 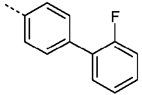 |
| Ar³-232 | Ar³-233 | Ar³-234 |
| 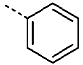 | 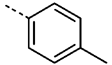 | 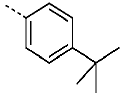 |
| Ar³-235 | Ar³-236 | Ar³-237 |
| 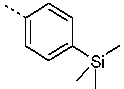 | 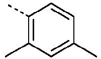 | 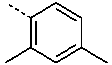 |
| Ar³-238 | Ar³-239 | Ar³-240 |
| 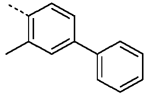 | 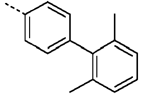 | 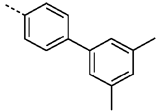 |
| Ar³-241 | Ar³-242 | Ar³-243 |
| 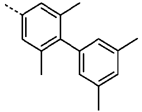 | 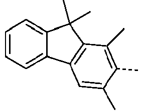 | 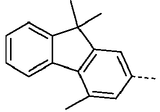 |
| Ar³-244 | Ar³-245 | Ar³-246 |
| 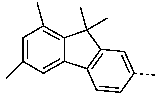 | | |
| Ar³-247 | | |

welche an den freien Positionen jeweils mit einem oder mehreren Resten R⁴ substituiert sein können.

Bevorzugt sind R³ und R⁴ bei jedem Auftreten gleich oder verschieden gewählt aus H, D, F, CN, Si(R⁵)₃, N(R⁵)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁵ substituiert sein können; und wobei in den genannten Alkylgruppen eine oder mehrere CH₂-Gruppen durch -C=C-, -R⁵C=CR⁵-, Si(R⁵)₂, C=O, C=NR⁵, -NR⁵-, -O-, -S-, -C(=O)O- oder -C(=O)NR⁵- ersetzt sein können.

Besonders bevorzugt ist R³ gleich H. Besonders bevorzugt ist R⁴ gleich H.

Bevorzugt ist R⁵ bei jedem Auftreten gleich oder verschieden gewählt aus H, D, F, CN, Si(R⁶)₃, N(R⁶)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können; und wobei in den genannten Alkylgruppen eine oder mehrere CH₂-Gruppen durch -C=C-, -R⁶C=CR⁶-, Si(R⁶)₂, C=O, C=NR⁶, -NR⁶-, -O-, -S-, -C(=O)O- oder -C(=O)NR⁶- ersetzt sein können. Besonders bevorzugt ist R⁵ gleich H.

Bevorzugt ist R⁶ bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkylgruppen, aromatischen Ringsysteme und heteroaromatischen Ringsysteme mit F oder CN substituiert sein können.

Erfindungsgemäß ist die Untereinheit der Formel (I) 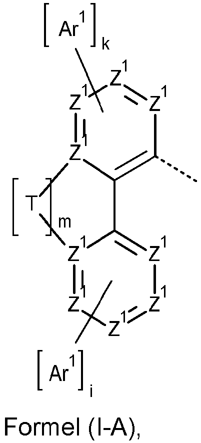
wobei die gestrichelte Linie die Bindung an den Rest der Formel darstellt, gewählt aus den folgenden Strukturen:

| | | |
|---|---|---|
| | | 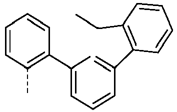 |
| | | Formel (I-A-45) |
| 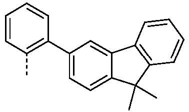 | 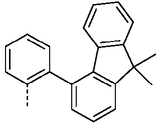 | |
| Formel (I-A-46) | Formel (I-A-47) | |
| 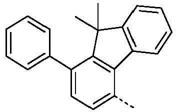 | 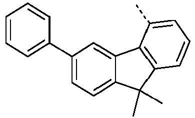 | 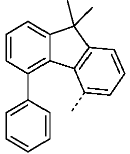 |
| Formel (I-A-49) | Formel (I-A-50) | Formel (I-A-51) |
| | 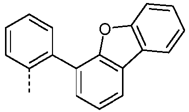 | 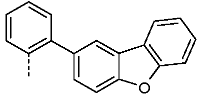 |
| | Formel (I-A-53) | Formel (I-A-54) |
| 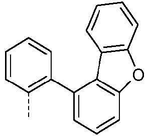 | | 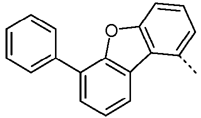 |
| Formel (I-A-55) | | Formel (I-A-57) |
| 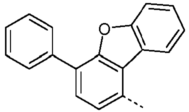 | 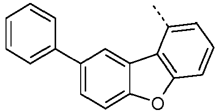 | 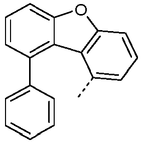 |
| Formel (I-A-58) | Formel (I-A-59) | Formel (I-A-60) |
| | 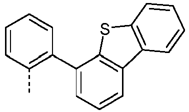 | 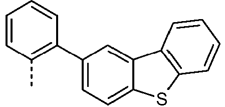 |
| | Formel (I-A-62) | Formel (I-A-63) |
| 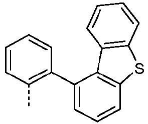 | | 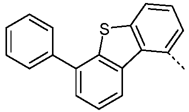 |
| Formel (I-A-64) | | Formel (I-A-66) |
| 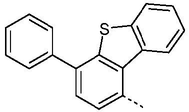 | 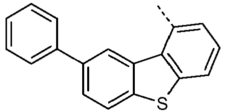 | 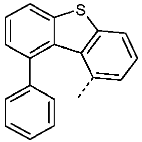 |
| Formel (I-A-67) | Formel (I-A-68) | Formel (I-A-69) |
| | 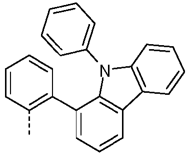 | 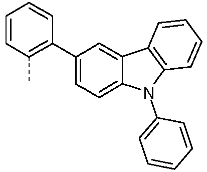 |
| | Formel (I-A-71) | Formel (I-A-72) |
| 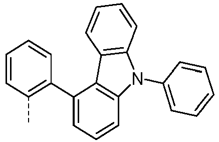 | | 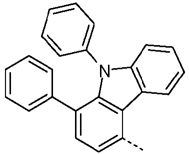 |
| Formel (I-A-73) | | Formel (I-A-75) |
| 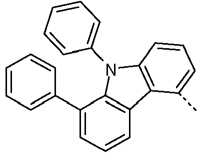 | 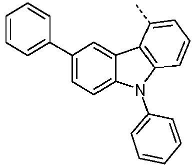 | 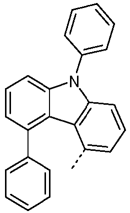 |
| Formel (I-A-76) | Formel (I-A-77) | Formel (I-A-78) |
| | 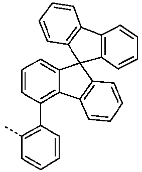 | 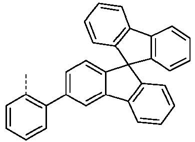 |
| | Formel (I-A-80) | Formel (I-A-81) |
| 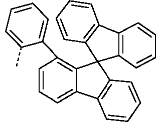 | | 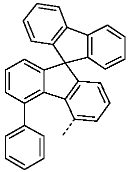 |
| Formel (I-A-82) | | Formel (I-A-84) |
| 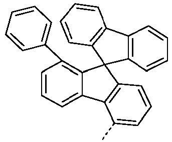 | 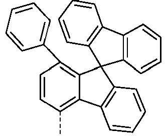 | 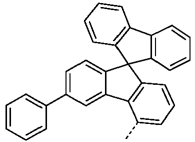 |
| Formel (I-A-85) | Formel (I-A-86) | Formel (I-A-87) |
| 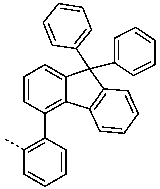 | | 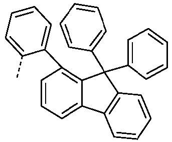 |
| Formel (I-A-88) | | Formel (I-A-90) |
| 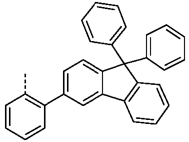 | | 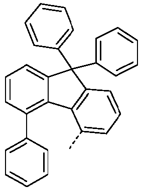 |
| Formel (I-A-91) | | Formel (I-A-93) |
| 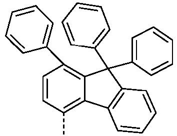 | 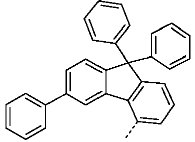 | 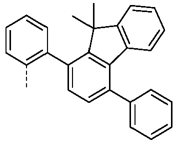 |
| Formel (I-A-94) | Formel (I-A-95) | Formel (I-A-96) |
| 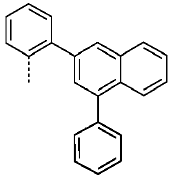 | | 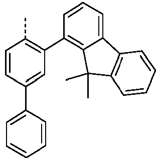 |
| Formel (I-A-109) | | Formel (I-A-111) |
| 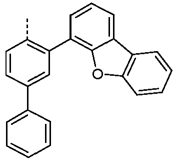 | 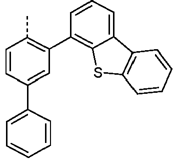 | 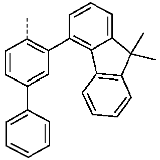 |
| Formel (I-A-112) | Formel (I-A-113) | Formel (I-A-114) |
| 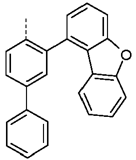 | 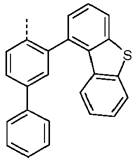 | |
| Formel (I-A-115) | Formel (I-A-116) | |
| 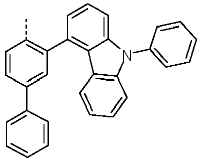 | 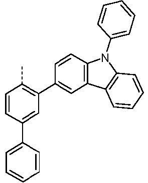 | |
| Formel (I-A-118) | Formel (I-A-119) | |
| 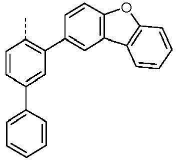 | 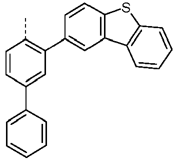 | |
| Formel (I-A-121) | Formel (I-A-122) | |

wobei die gestrichelte Linie die Bindung an das N-Atom in Formel (I) darstellt.

Bevorzugte Ausführungsformen der Formel (I) entsprechen der folgenden Formel (1-2)

| |
|---|
| 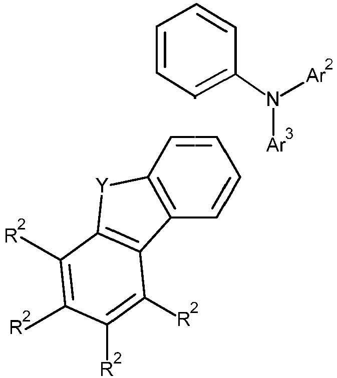 |
| Formel (I-2) |

wobei für die auftretenden Variablen gilt:
Y ist gewählt aus C(R¹)₂, Si(R¹)₂, NR¹, O, und S, bevorzugt aus C(R¹)₂, NR¹, O, und S;
die freien Positionen an den Benzolringen können jeweils mit einem Rest R¹ substituiert sein, wobei
R¹ und R² sind bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ bzw. R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch-R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
und die sonstigen auftretenden Variablen sind definiert wie oben und entsprechen bevorzugt ihren oben angegebenen bevorzugten Ausführungsformen.

Eine bevorzugte Ausführungsform der Formel (1-2) entspricht der folgenden Formel (I-2A)

| |
|---|
| 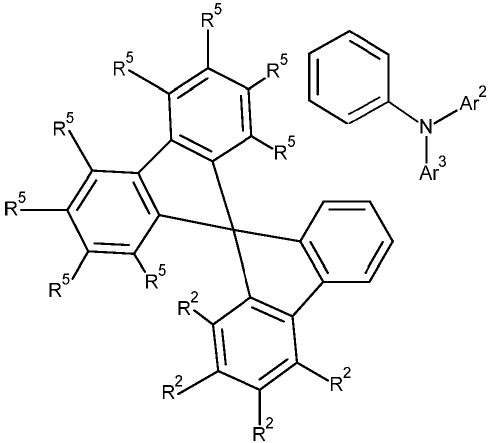 |
| Formel (I-2A) |

wobei für die auftretenden Variablen gilt:
die freien Positionen an den Benzolringen können jeweils mit einem Rest R¹ substituiert sein,
und die sonstigen auftretenden Variablen sind definiert wie oben und entsprechen bevorzugt ihren oben angegebenen bevorzugten Ausführungsformen.

Bevorzugte Ausführungsformen der Formel (1-2) entsprechen den folgenden Formeln (I-2-1) bis (I-2-3)

| |
|---|
| 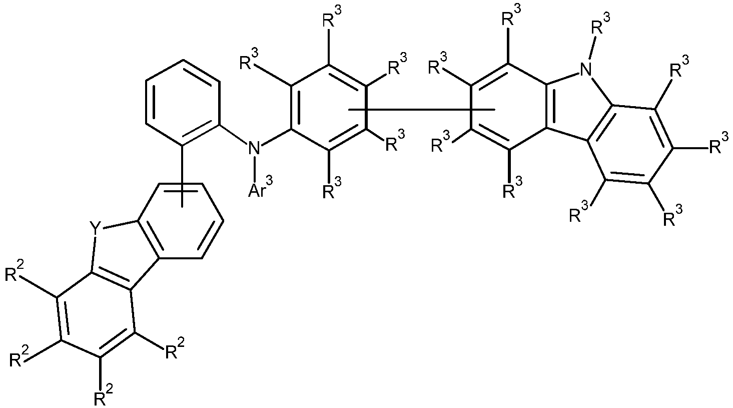 |
| Formel (I-2-1) |
| 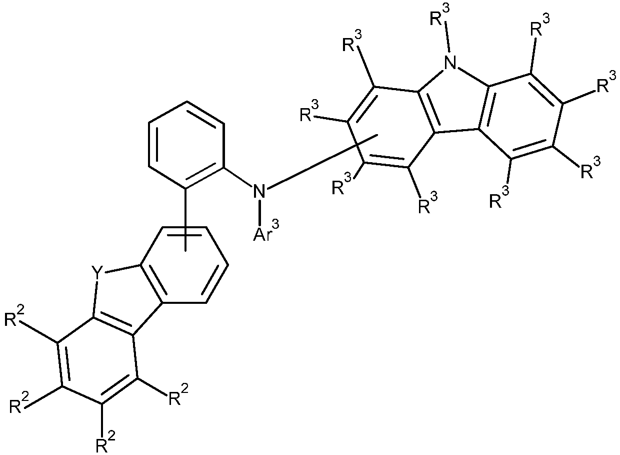 |
| Formel (I-2-2) |
| 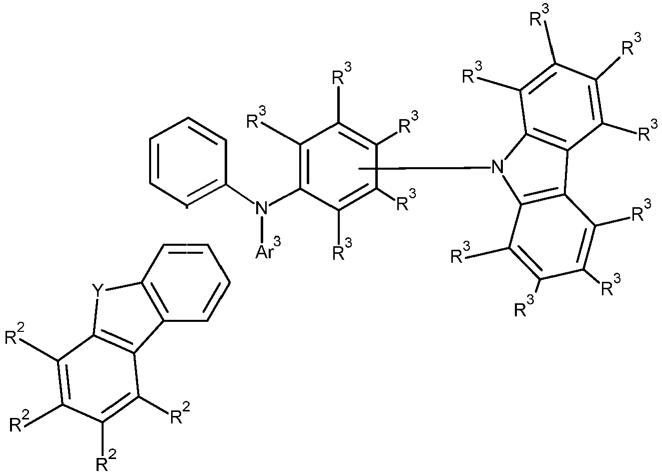 |
| Formel (I-2-3) |

wobei für die auftretenden Variablen gilt:
Y ist gewählt aus C(R¹)₂, Si(R¹)₂, NR¹, O, und S, bevorzugt aus C(R¹)₂, NR¹, O, und S;
die freien Positionen an den Benzolringen können jeweils mit einem Rest R¹ substituiert sein,
und die sonstigen auftretenden Variablen sind definiert wie oben und entsprechen bevorzugt ihren oben angegebenen bevorzugten Ausführungsformen.

Bevorzugte Ausführungsformen der Formel (I-2A) entsprechen den folgenden Formeln (I-2A-1) bis (I-2A-3)

| |
|---|
| 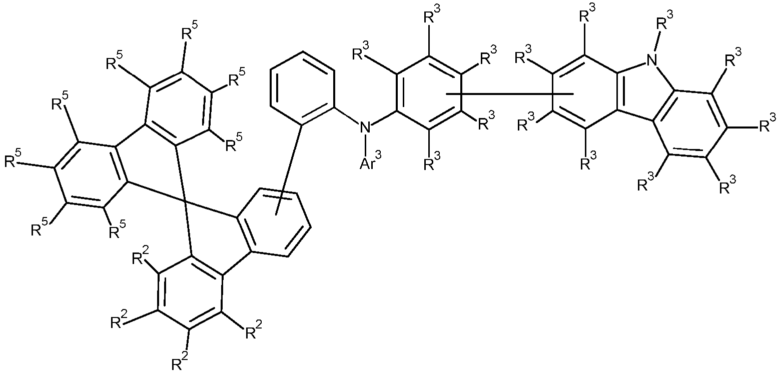 |
| Formel (I-2A-1) |
| 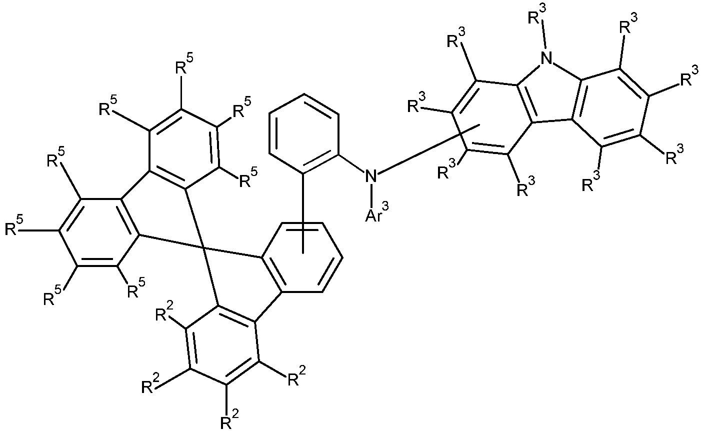 |
| Formel (I-2A-2) |
| 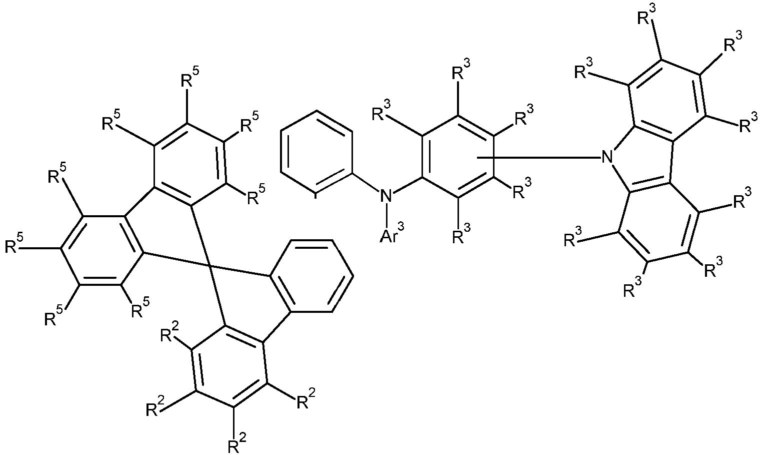 |
| Formel (I-2A-3) |

wobei für die auftretenden Variablen gilt:
die freien Positionen an den Benzolringen können jeweils mit einem Rest R¹ substituiert sein,
und die sonstigen auftretenden Variablen sind definiert wie oben und entsprechen bevorzugt ihren oben angegebenen bevorzugten Ausführungsformen.

Bevorzugte spezifische Verbindungen gemäß Formel (I) sind in der folgenden Tabelle abgebildet, wobei Verbindungen, die nicht anspruchsgemäß sind, mit # gekennzeichnet sind:

| | | |
|---|---|---|
| 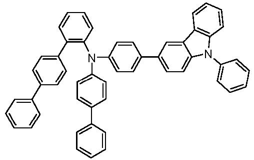 | 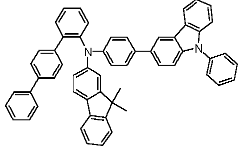 | 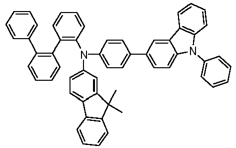 |
| 1# | 2# | 3# |
| 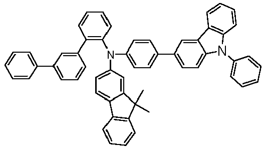 | 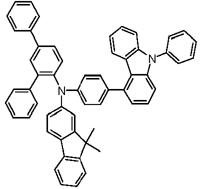 | 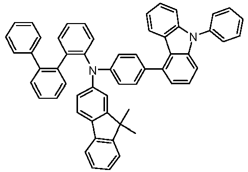 |
| 4# | 5# | 6# |
| 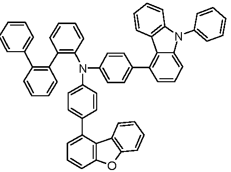 | 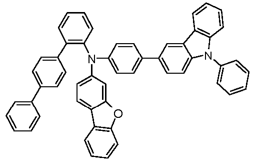 | 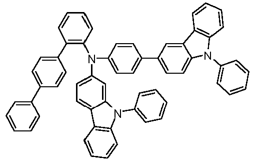 |
| 7# | 8# | 9# |
| 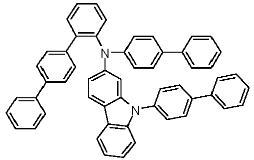 | 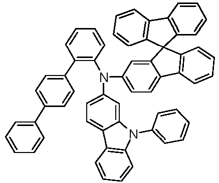 | 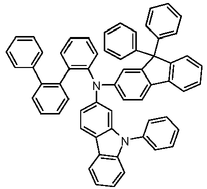 |
| 10# | 11# | 12# |
| 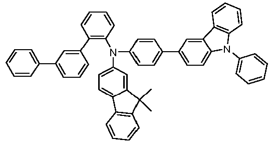 | 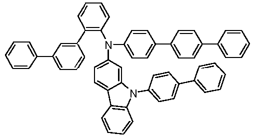 | 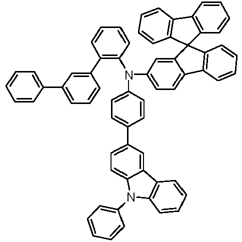 |
| 13# | 14# | 15# |
| 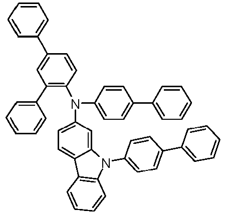 | 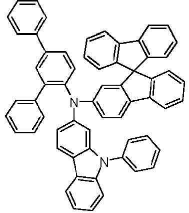 | 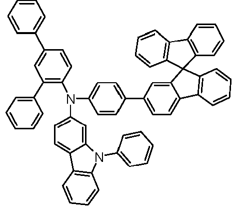 |
| 16# | 17# | 18# |
| 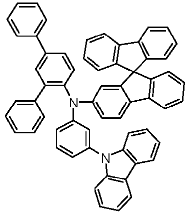 | 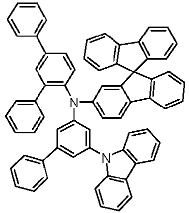 | 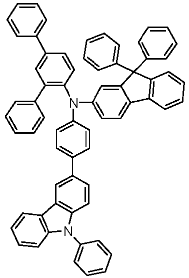 |
| 19# | 20# | 21# |
| 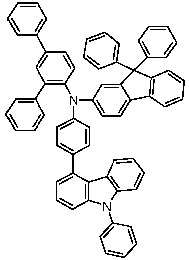 | 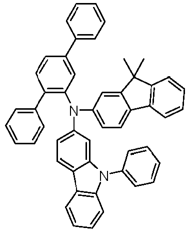 | 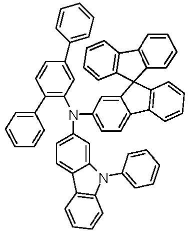 |
| 22# | 23# | 24# |
| 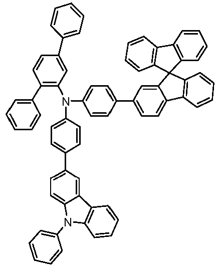 | 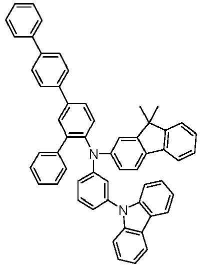 | 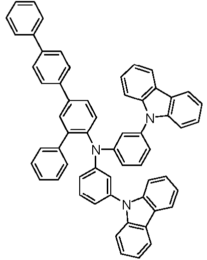 |
| 25# | 26# | 27# |
| 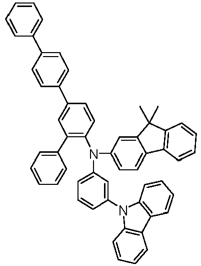 | 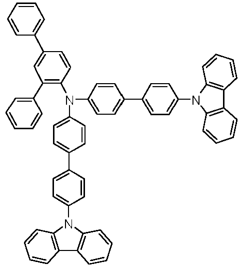 | 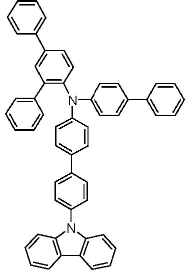 |
| 28# | 29# | 30# |
| 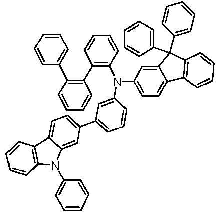 | 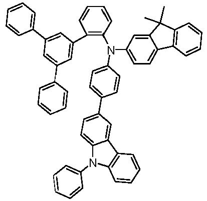 | 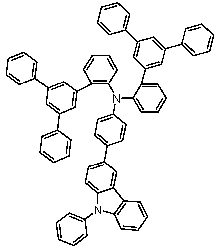 |
| 31# | 32# | 33# |
| 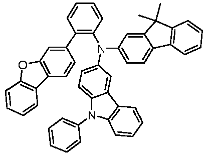 | 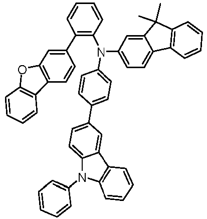 | 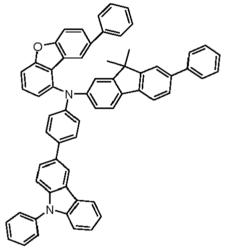 |
| 34# | 35# | 36 |
| 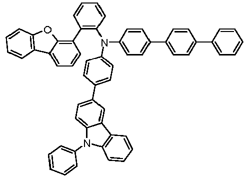 | 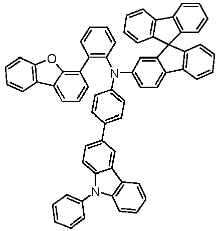 | 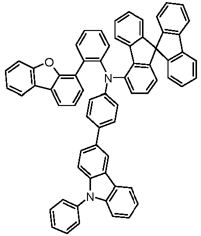 |
| 37 | 38 | 39 |
| 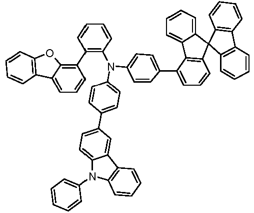 | 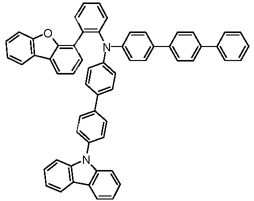 | 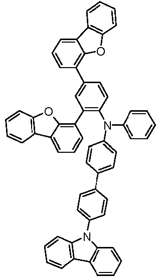 |
| 40 | 41 | 42# |
| 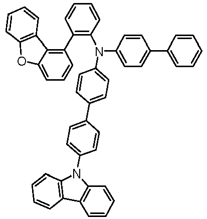 | 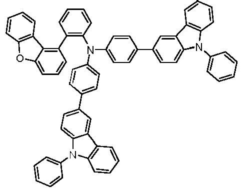 | 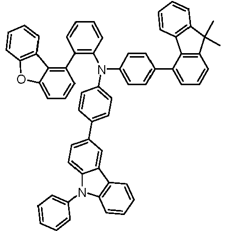 |
| 43 | 44 | 45 |
| 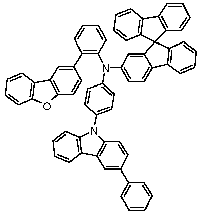 | 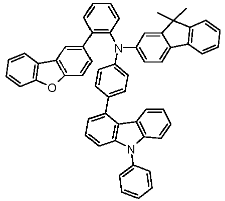 | 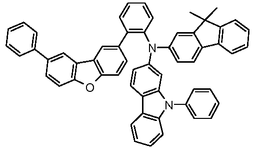 |
| 46 | 47 | 48# |
| 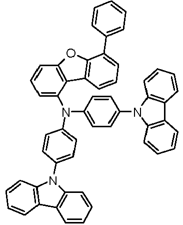 | 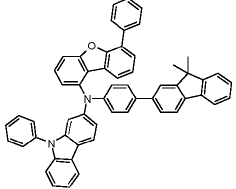 | 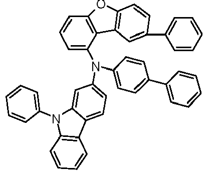 |
| 49 | 50 | 51 |
| 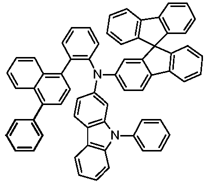 | 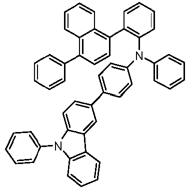 | 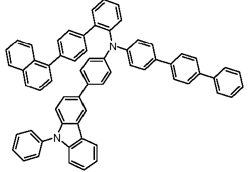 |
| 52# | 53# | 54# |
| 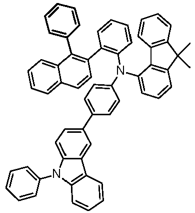 | 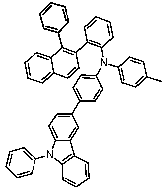 | 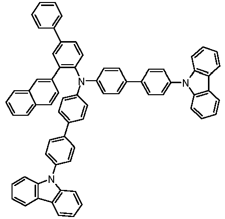 |
| 55# | 56# | 57# |
| 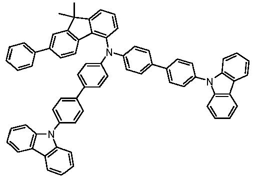 | 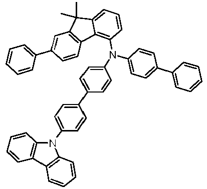 | 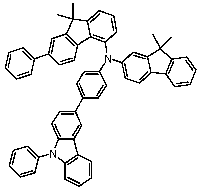 |
| 58# | 59# | 60# |
| 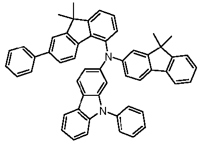 | 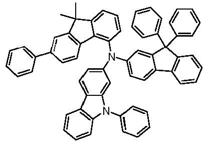 | 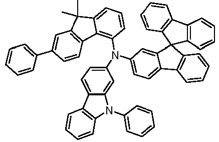 |
| 61# | 62# | 63# |
| 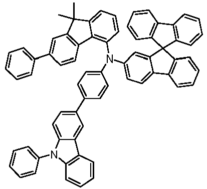 | 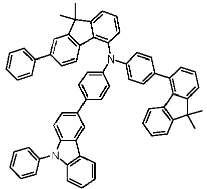 | 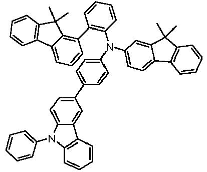 |
| 64# | 65# | 66 |
| 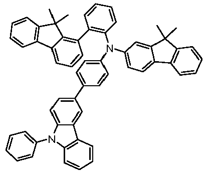 | 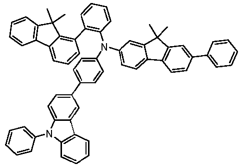 | 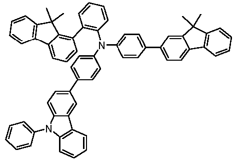 |
| 67 | 68 | 69 |
| 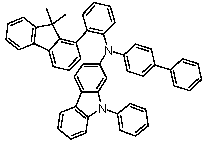 | 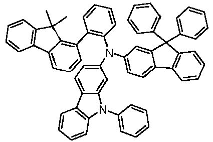 | 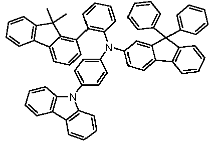 |
| 70 | 71 | 72 |
| 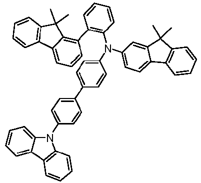 | 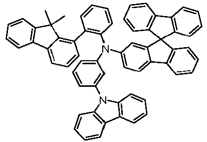 | 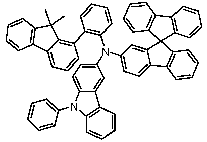 |
| 73 | 74 | 75 |
| 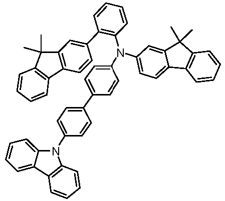 | 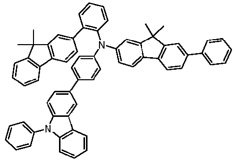 | 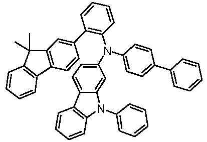 |
| 76# | 77# | 78# |
| 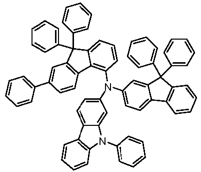 | 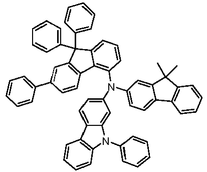 | 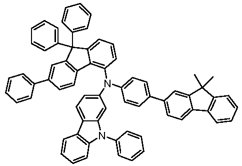 |
| 79# | 80# | 81# |
| 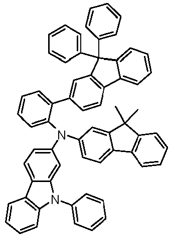 | 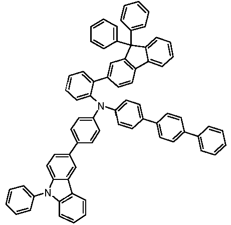 | 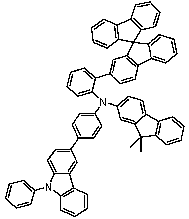 |
| 82 | 83 | 84 |
| 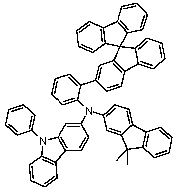 | 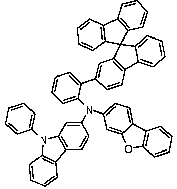 | 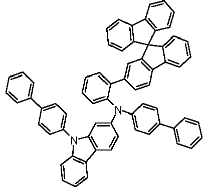 |
| 85 | 86 | 87 |
| 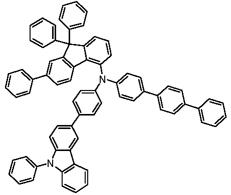 | 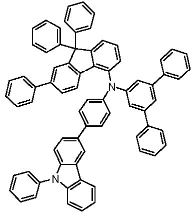 | 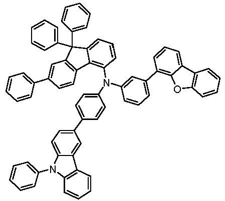 |
| 88# | 89# | 90# |
| 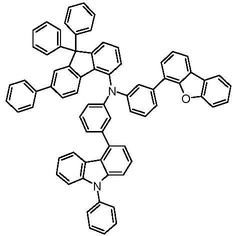 | 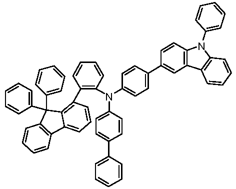 | 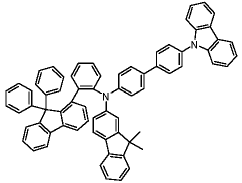 |
| 91# | 92# | 93# |
| 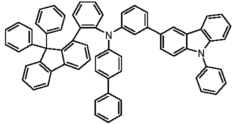 | 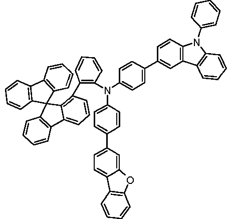 | 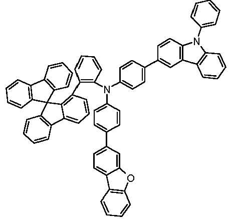 |
| 94# | 95 | 96 |
| 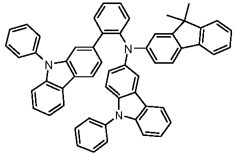 | 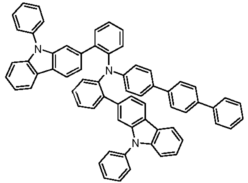 | 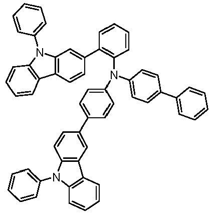 |
| 97# | 98# | 99# |
| 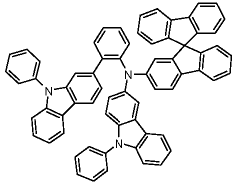 | 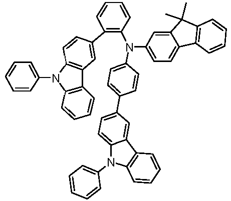 | 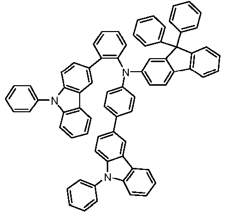 |
| 100# | 101 | 102 |
| 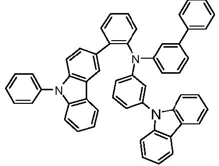 | 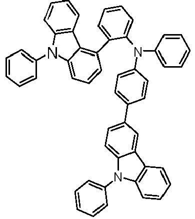 | 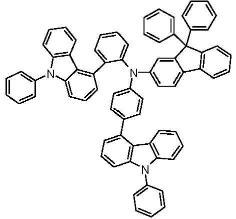 |
| 103 | 104 | 105 |
| 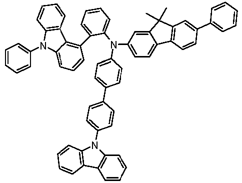 | 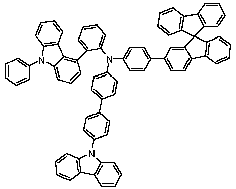 | 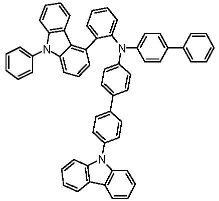 |
| 106 | 107 | 108 |
| 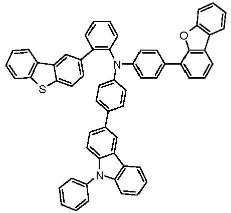 | 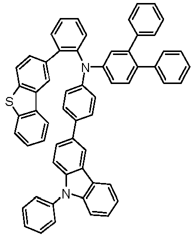 | 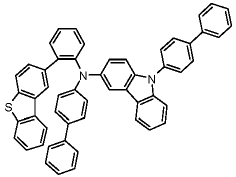 |
| 109 | 110 | 111 |
| 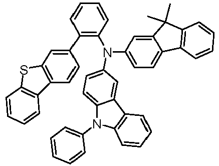 | 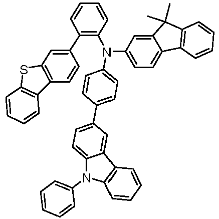 | 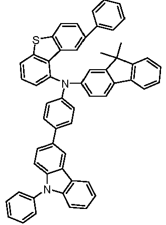 |
| 112# | 113# | 114 |
| 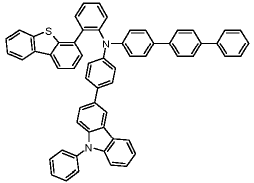 | 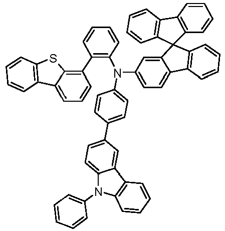 | 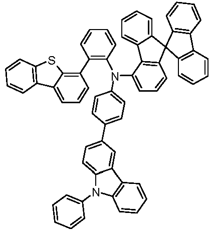 |
| 115 | 116 | 117 |
| 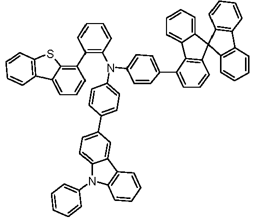 | 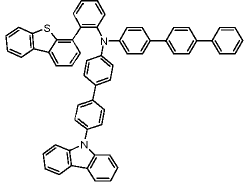 | 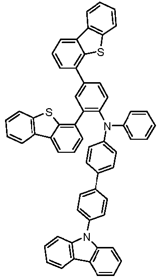 |
| 118 | 119 | 120# |
| 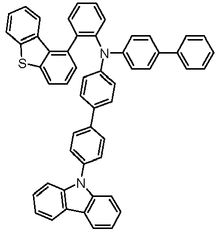 | 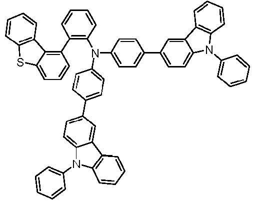 | 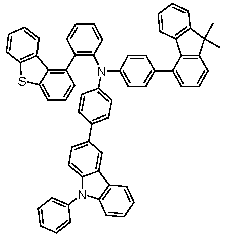 |
| 121 | 122 | 123 |
| 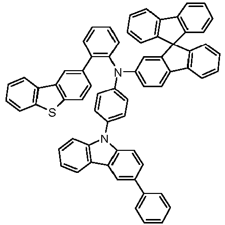 | 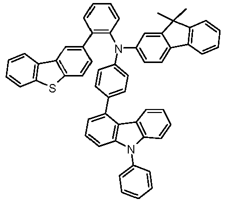 | 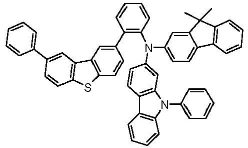 |
| 124 | 125 | 126# |
| 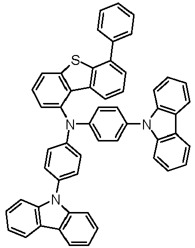 | 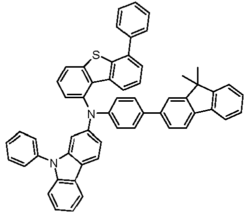 | 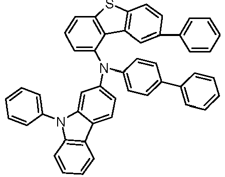 |
| 127 | 128 | 129 |

Die Verbindungen gemäß Formel (I) können unter Einsatz bekannter Reaktionen der organischen Chemie hergestellt werden, insbesondere unter Einsatz von metallkatalysierten Kupplungsreaktionen wie Suzuki-Kupplung und Buchwald-Kupplung.

Ein bevorzugtes Verfahren zur Herstellung der Verbindungen gemäß Formel (I) ist im Folgenden näher erläutert (Schema 1). Der Fachmann kann dieses Verfahren im Rahmen seiner allgemeinen Kenntnisse der organischen Synthesechemie abwandeln und anpassen, soweit erforderlich.

Gemäß Schema 1 wird in einem ersten Schritt ein Biphenyl-Derivat, das mit zwei reaktiven Gruppen X und Y substituiert ist, wobei die Gruppe X in ortho-Position zur Bindung zwischen den beiden Phenylgruppen vorliegt, in einer Suzuki-Reaktion mit einem aromatischen oder heteroaromatischen Ringsystem Ar, das mit einer Boronsäuregruppe substituiert ist, umgesetzt. Dabei wird das Ringsystem Ar an der Position der reaktiven Gruppe Y eingeführt. In einem zweiten Schritt wird das erhaltene Intermediat mit einer Aminverbindung der Formel HNAr₂ in einer Buchwald-Kupplungsreaktion umgesetzt. Dabei wird die Gruppe -NAr₂ in der Position der reaktiven Gruppe X eingeführt, so dass sie in ortho-Position zur Bindung zwischen den beiden Phenylgruppen vorliegt. 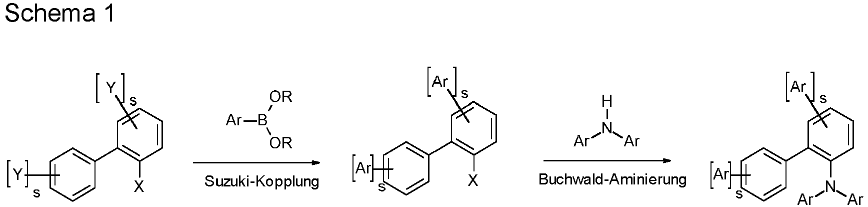
X, Y= reaktive Gruppen, bevorzugt Cl, Br, I oder andere reaktive Gruppe wie bspw. Triflat, Tosylat
Ar = aromatisches oder heteroaromatisches Ringsystem
R = organischer Rest

Die erhaltene Verbindung kann gegebenenfalls weiter modifiziert werden.

Gegenstand der vorliegenden Anmeldung ist damit ein Verfahren zur Herstellung einer Verbindung der Formel (I), dadurch gekennzeichnet, dass in einem ersten Schritt i) ein Biphenyl-Derivat, das mit reaktiven Gruppen X und Y substituiert ist, wobei Gruppe X in ortho-Position zur Bindung zwischen den beiden Phenylgruppen vorliegt, mit einem aromatischen oder heteroaromatischen Ringsystem, das mit einer Boronsäuregruppe substituiert ist, umgesetzt wird, so dass das aromatische oder heteroaromatische Ringsystem in der Position der Gruppe Y eingeführt wird, und dass in einem zweiten Schritt ii) das in Schritt i) erhaltene Intermediat mit einer Verbindung der Formel HNAr₂ umgesetzt wird, wobei Ar gewählt ist aus aromatischen Ringsystemen und heteroaromatischen Ringsystemen, wobei in dieser Umsetzung die Gruppe -NAr₂ in der Position der Gruppe X eingeführt wird.

Bevorzugt ist die Reaktion des Schritts i) eine Suzuki-Kupplungsreaktion. Bevorzugt ist die Reaktion des Schritts ii) eine Buchwald-Kupplungsreaktion.

Das im Schritt i) gebildete Intermediat entspricht dabei bevorzugt einer Formel (I-Int-1) 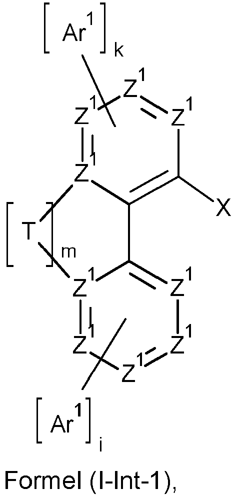 wobei
- Z¹: ist bei jedem Auftreten gleich oder verschieden gewählt aus CR¹ und N, wobei Z¹ gleich C ist, wenn eine Gruppe Ar¹ oder T daran gebunden ist;
- Ar¹: ist bei jedem Auftreten gleich oder verschieden ein aromatisches Ringsystem mit 6 bis 30 aromatischen Ringatomen, das mit einem oder mehreren Resten R² substituiert sein kann;
- T: ist gewählt aus C(R¹)₂, Si(R¹)₂, NR¹, O, und S;
- m: ist gleich 0 oder 1;
- i: ist gleich 0, 1, 2, 3, 4, oder 5;
- k: ist gleich 0, 1, 2, 3, oder 4;

wobei die Summe von k und i mindestens gleich 1 ist und
die übrigen auftretenden Variablen wie oben definiert sind, und wobei X eine reaktive Gruppe ist, bevorzugt Cl, Br, I oder eine Triflat- oder Tosylatgruppe, besonders bevorzugt Cl oder Br.

Die Verbindung der Formel HNAr₂, die in Schritt ii) eingesetzt wird, entspricht dabei bevorzugt einer Formel (I-Int-2) 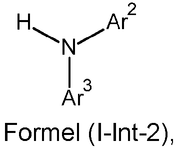 wobei die auftretenden Variablen wie oben definiert sind.

Die oben beschriebenen Verbindungen der Formel (I), insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I), wobei die Bindung(en) zum Polymer, Oligomer oder Dendrimer an beliebigen, in Formel (I) mit R³ oder R⁴ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine lochtransportierende Schicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht(en)-wahlweise Elektronenblockierschicht-emittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen (für den prinzipiellen Aufbau siehe z. B. WO 2005/011013). Die erfindungsgemäßen Verbindungen sind dabei bevorzugt in einer Lochtransportschicht, Lochinjektionsschicht, Elektronenblockierschicht, emittierenden Schicht, lochblockierenden Schicht und/oder elektronentransportierenden Schicht vorhanden, besonders bevorzugt in einer emittierenden Schicht als Matrixmaterial, in einer Lochblockierschicht und/oder in einer Elektronentransportschicht.

Es ist erfindungsgemäß bevorzugt, wenn die Verbindung gemäß Formel (I) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht, einer emittierenden Schicht, einer Lochblockierschicht, und/oder einer Elektronentransportschicht enthalten sein. Besonders bevorzugt ist sie in diesem Fall in einer Elektronenblockierschicht oder in einer emittierenden Schicht in Kombination mit einer phosphoreszierenden emittierenden Verbindung enthalten.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen. Weitere Beispiele sind in der folgenden Tabelle aufgeführt:

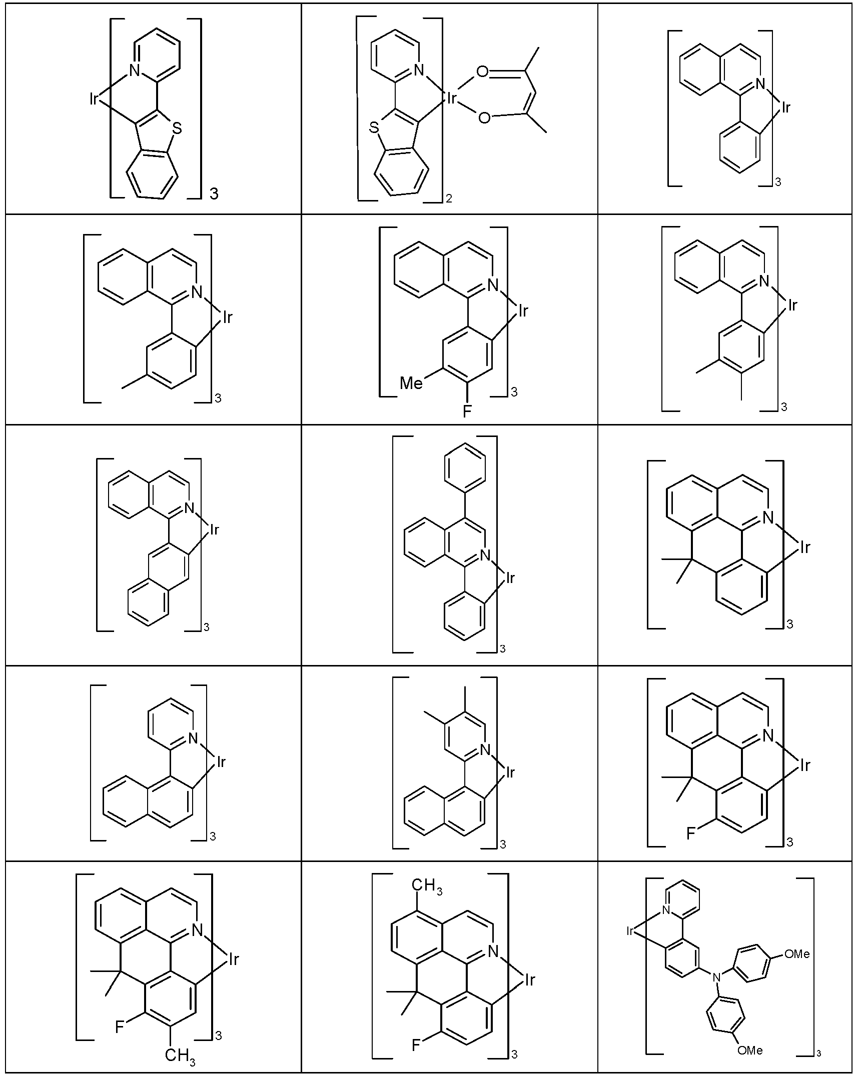

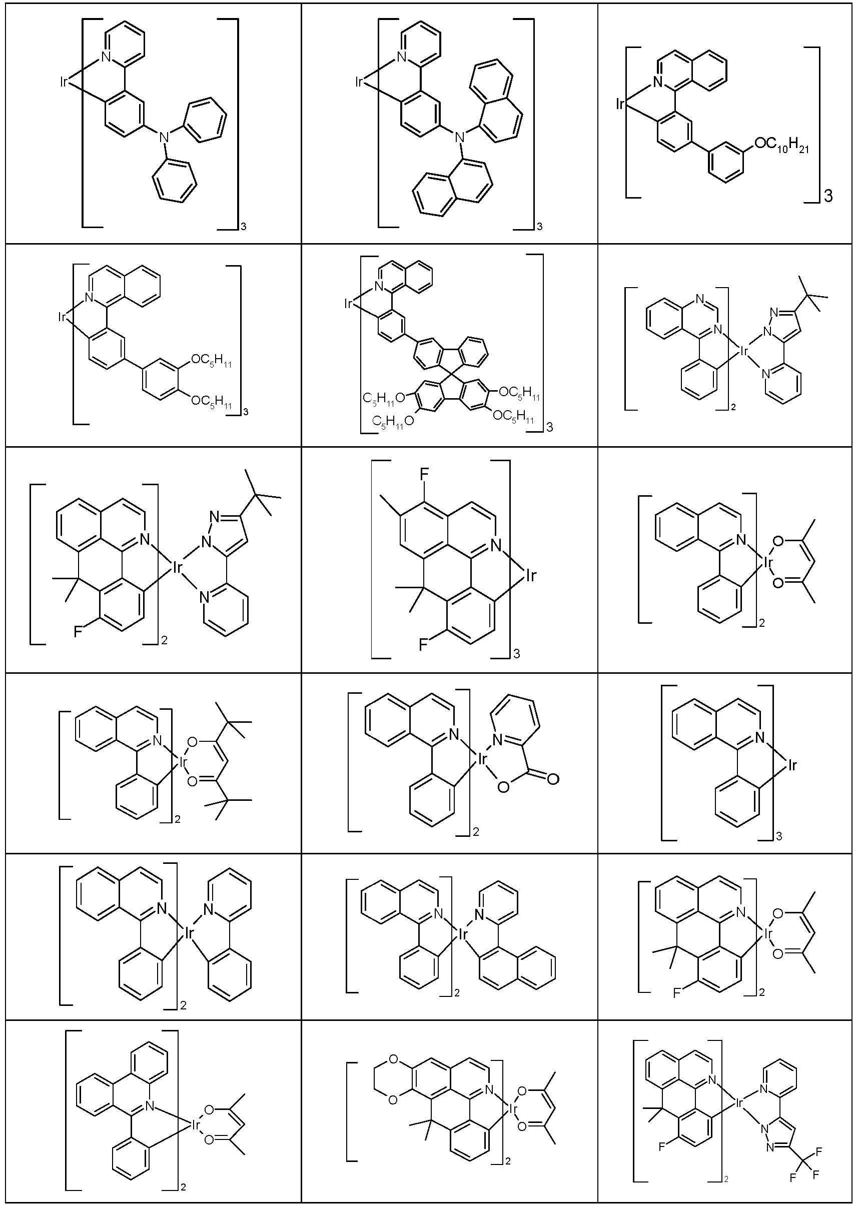

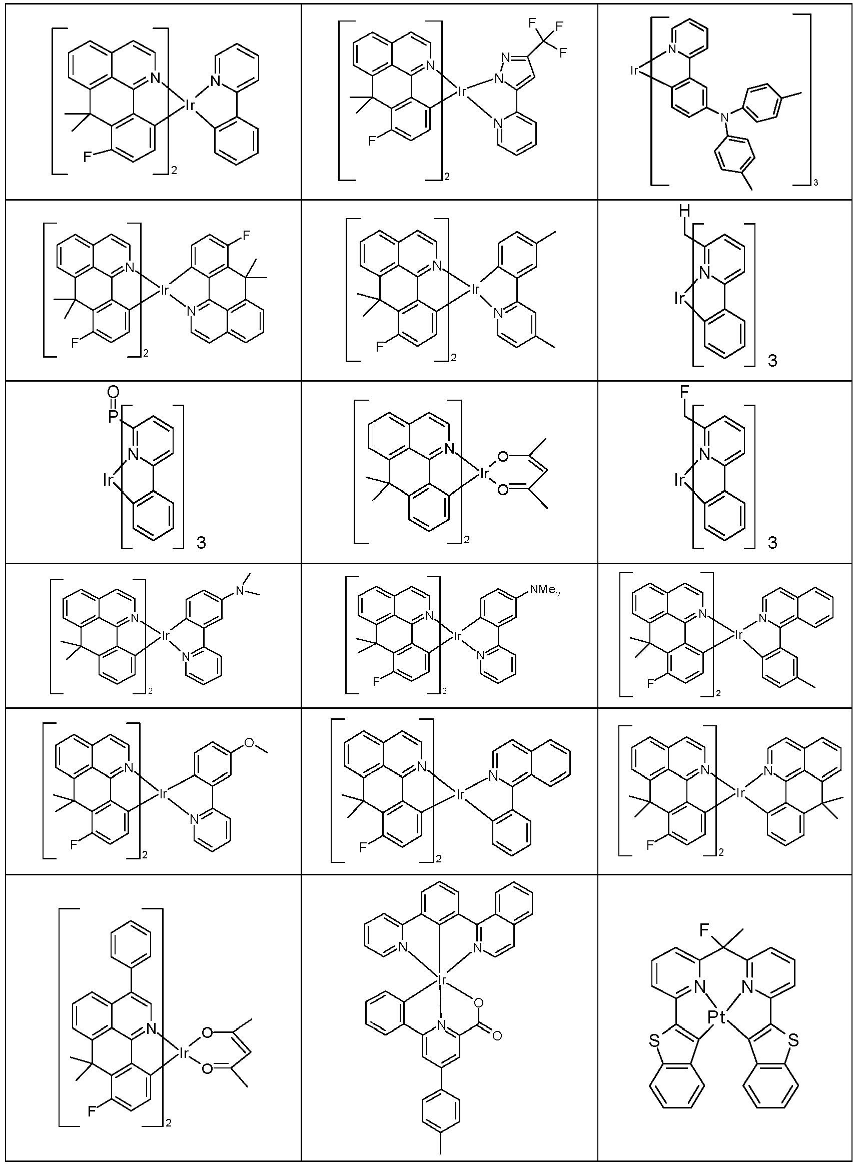

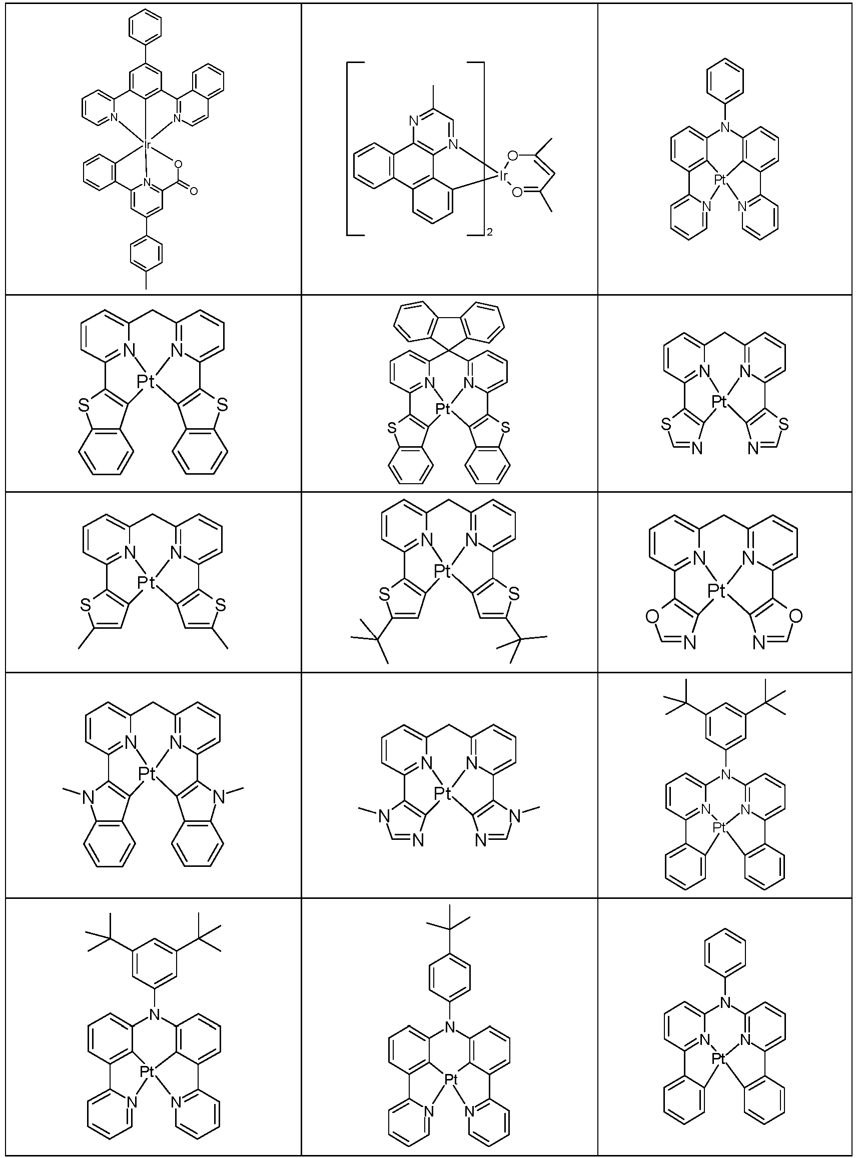

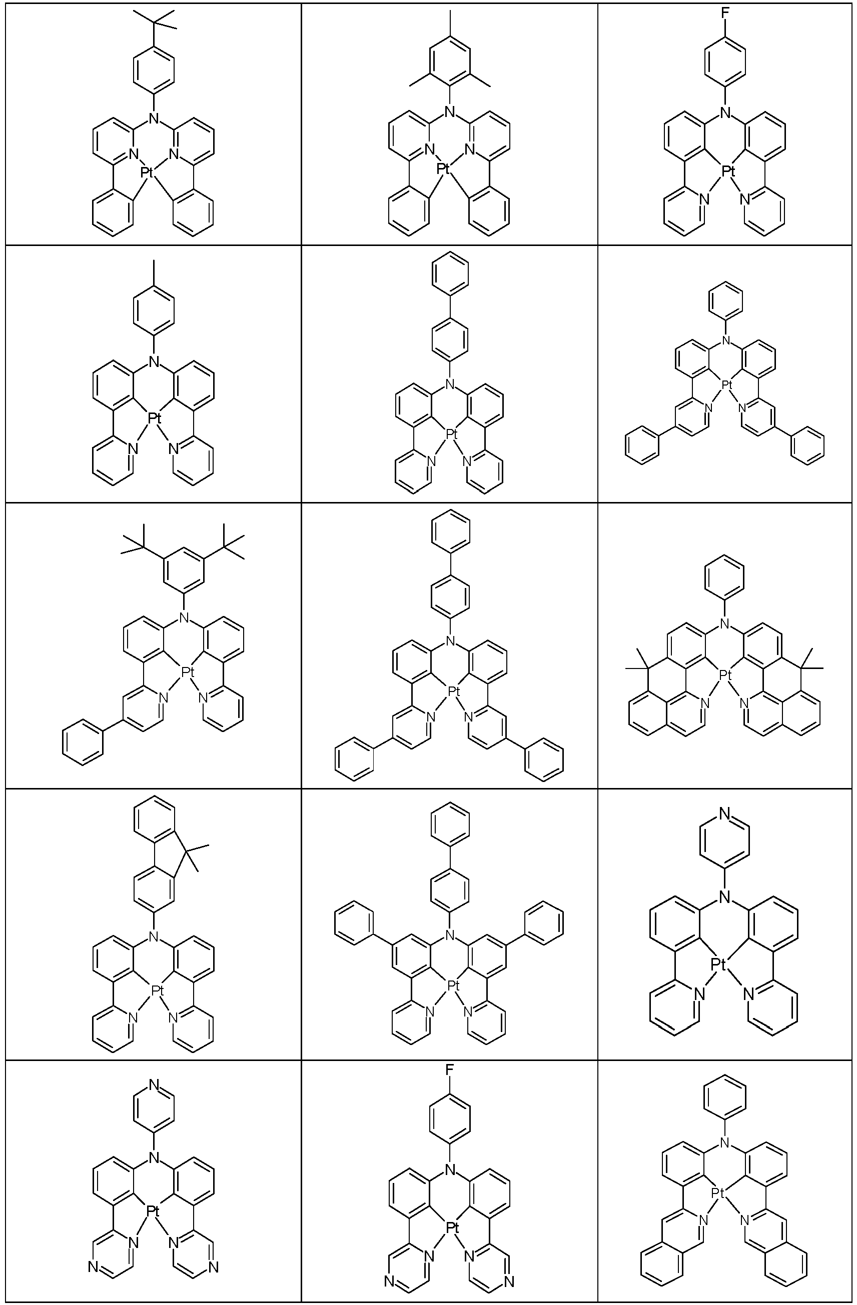

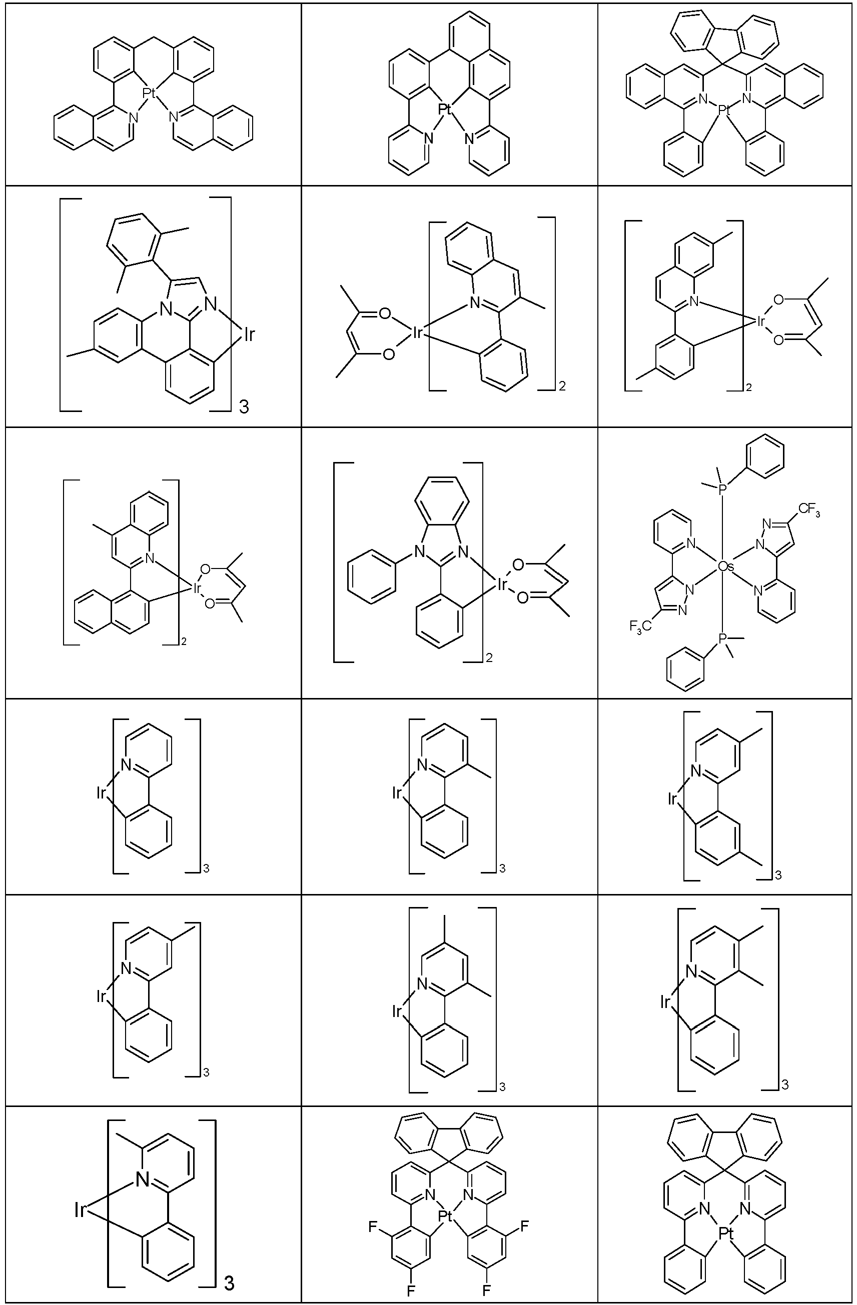

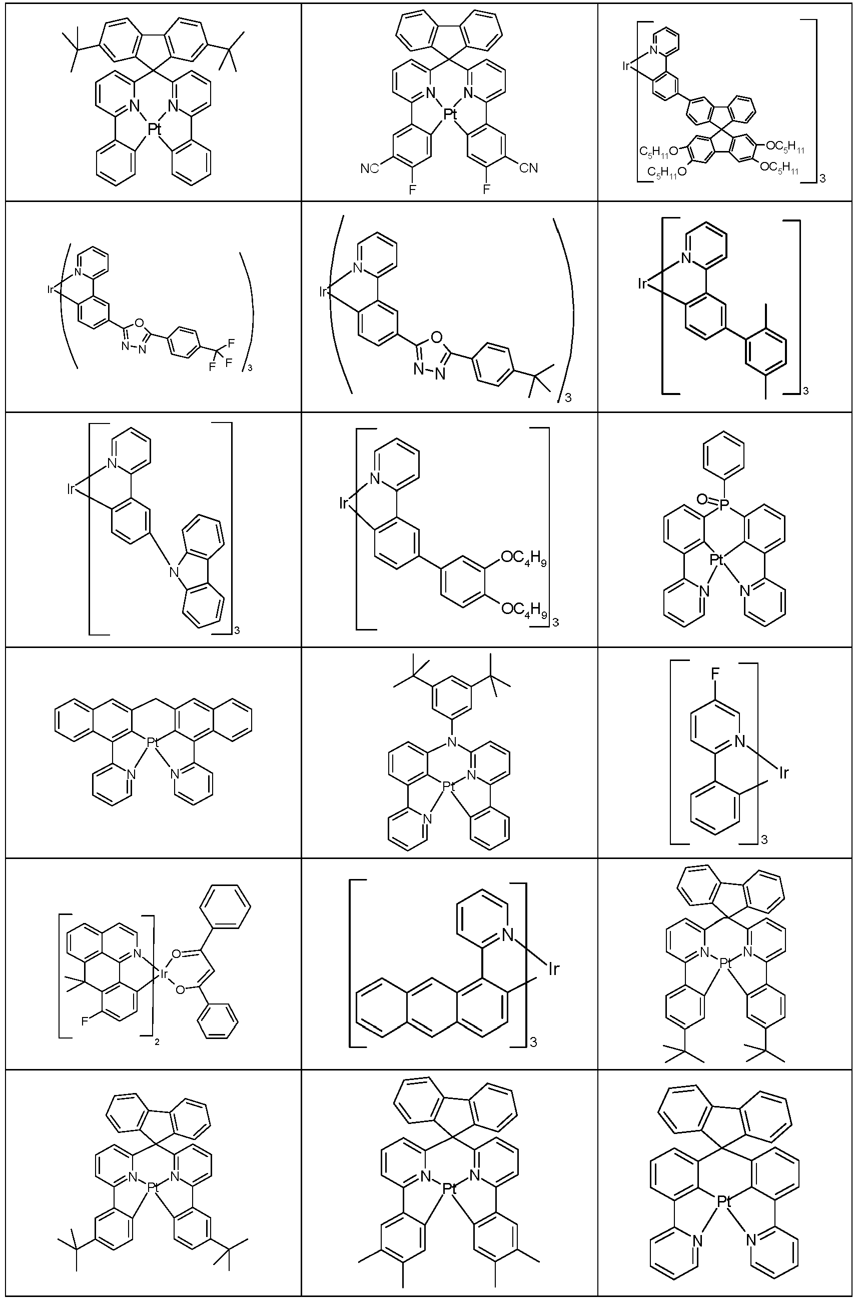

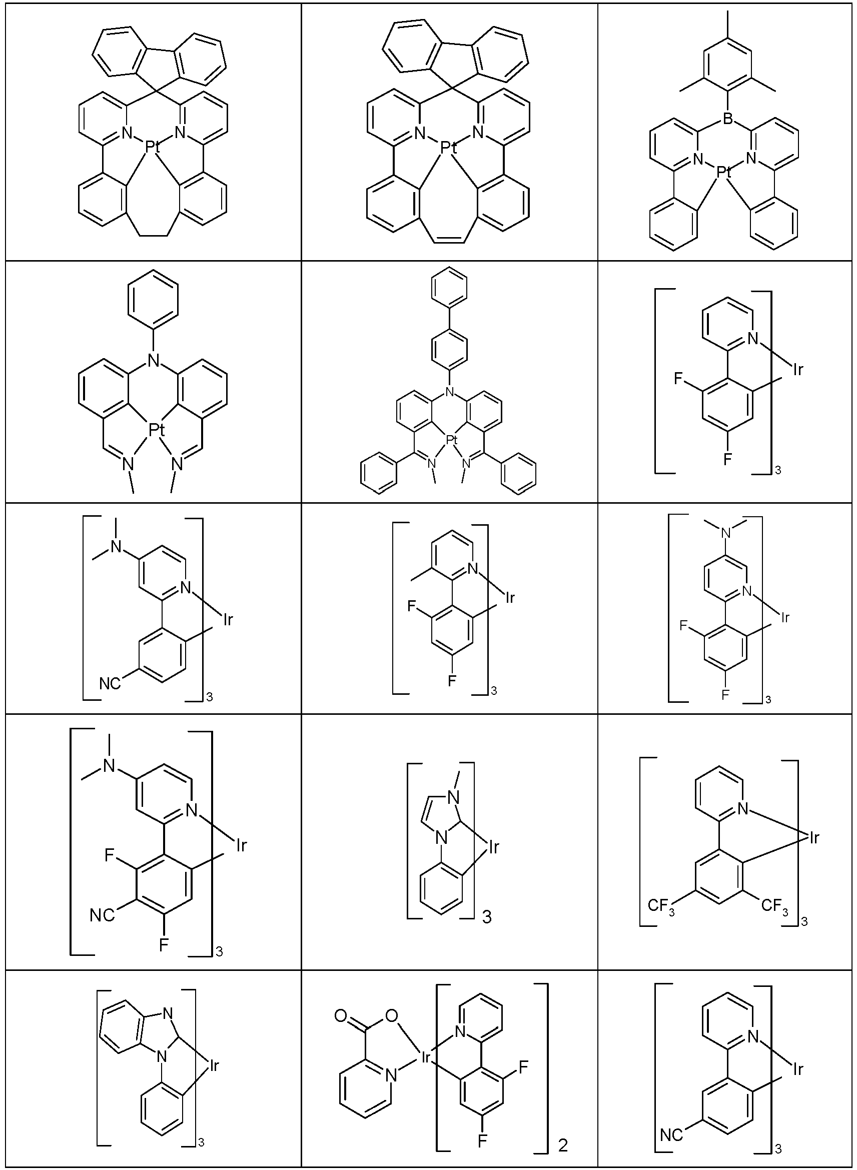

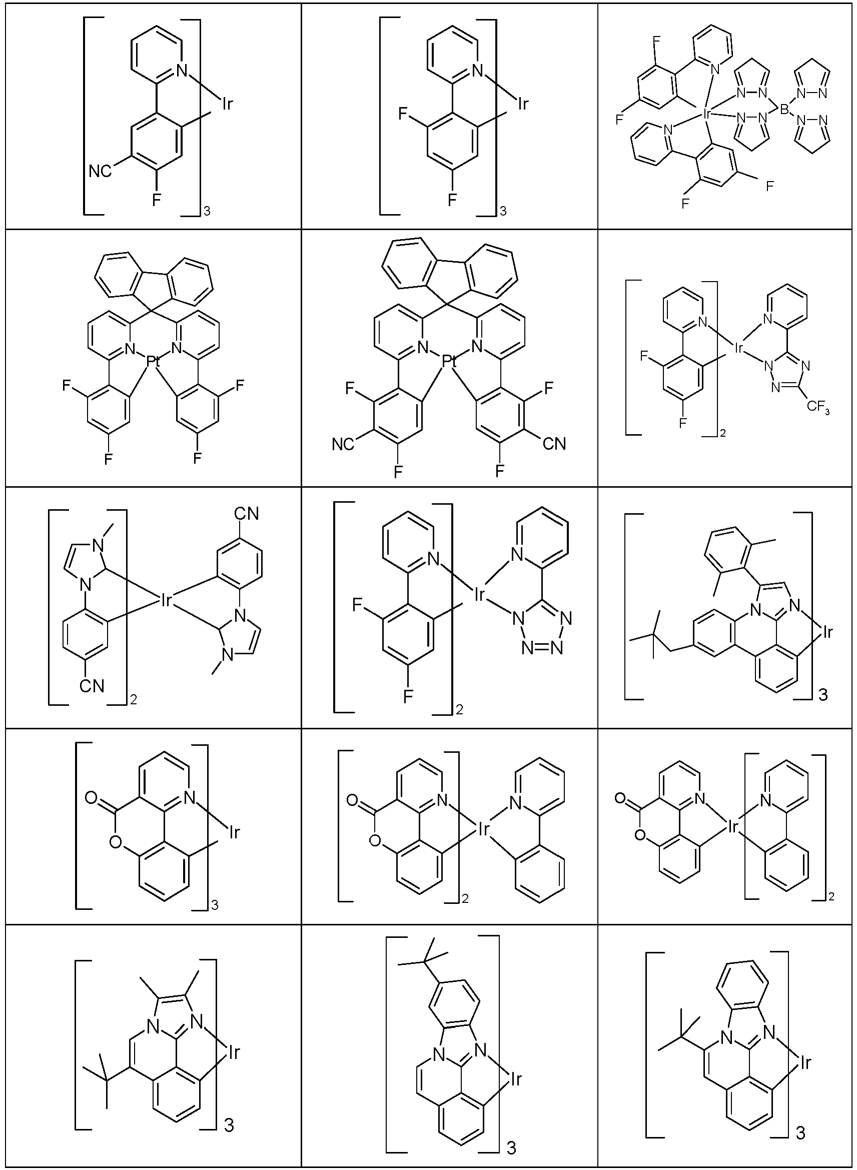

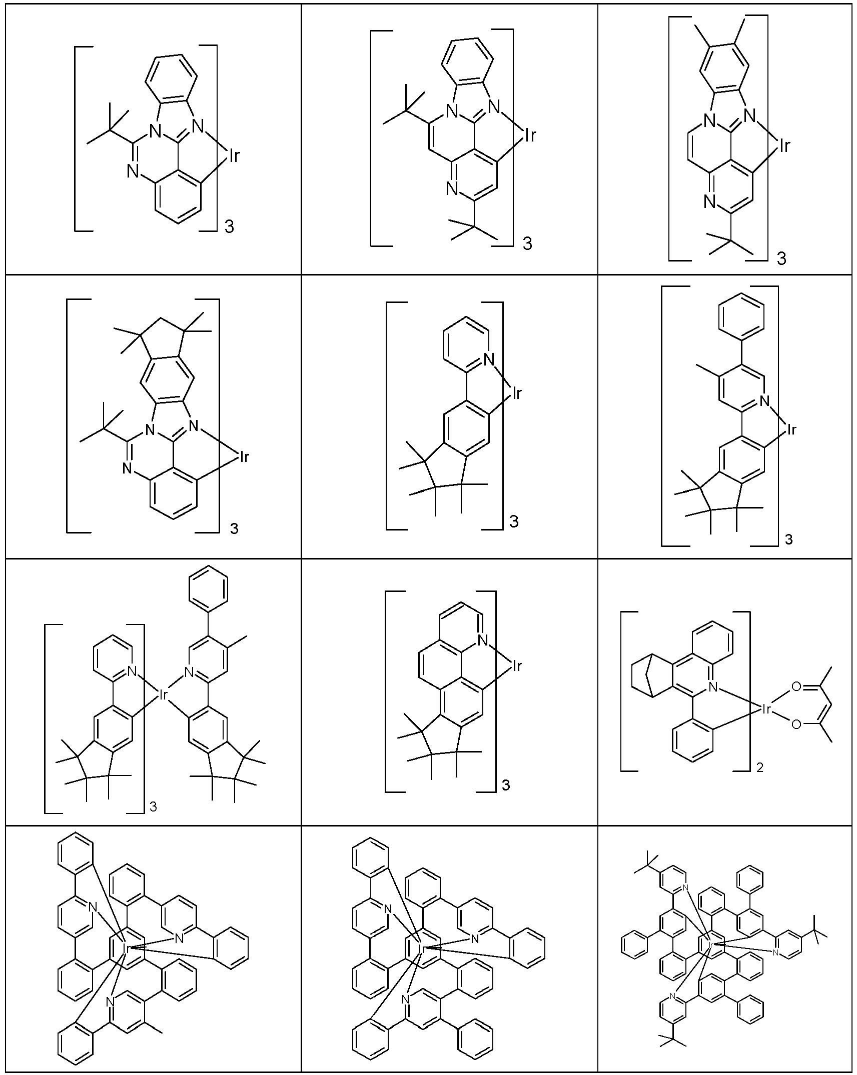

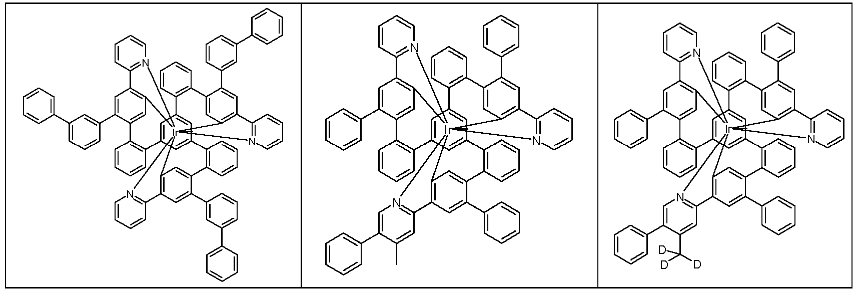

In einer bevorzugten Ausführungsform der Erfindung werden die Verbindungen gemäß Formel (I) als lochtransportierendes Material eingesetzt. Die Verbindungen liegen dann bevorzugt in einer lochtransportierenden Schicht vor. Bevorzugte Ausführungsformen von lochtransportierenden Schichten sind Lochtransportschichten, Elektronenblockierschichten und Lochinjektionsschichten. Besonders bevorzugt ist in der Elektronenblockierschicht der Vorrichtung mindestens eine Verbindung der Formel (I) vorhanden.

Eine Lochtransportschicht gemäß der vorliegenden Anmeldung ist eine Schicht mit lochtransportierender Funktion, welche sich zwischen Anode und emittierender Schicht befindet. Insbesondere ist es eine lochtransportierende Schicht, die keine Lochinjektionsschicht und keine Elektronenblockierschicht ist.

Lochinjektionsschichten und Elektronenblockierschichten werden im Sinne der vorliegenden Anmeldung als spezielle Ausführungsformen von lochtransportierenden Schichten verstanden. Eine Lochinjektionsschicht ist dabei im Fall von mehreren lochtransportierenden Schichten zwischen Anode und emittierender Schicht eine lochtransportierende Schicht, welche sich direkt an die Anode anschließt oder nur durch eine einzelne Beschichtung der Anode von ihr getrennt ist. Eine Elektronenblockierschicht ist im Fall von mehreren lochtransportierenden Schichten zwischen Anode und emittierender Schicht diejenige lochtransportierende Schicht, welche sich direkt anodenseitig an die emittierende Schicht anschließt. Bevorzugt enthält die erfindungsgemäße OLED zwei, drei oder vier lochtransportierende Schichten zwischen Anode und emittierender Schicht, von denen bevorzugt mindestens eine eine Verbindung gemäß Formel (I) enthält, besonders bevorzugt genau eine oder zwei eine Verbindung gemäß Formel (I) enthalten.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden.

Gemäß einer bevorzugten Ausführungsform enthält eine lochtransportierende Schicht enthaltend die Verbindung der Formel (I) zusätzlich eine oder mehrere weitere lochtransportierende Verbindungen. Diese weiteren lochtransportierenden Verbindungen sind bevorzugt gewählt aus Triarylamin-Verbindungen, besonders bevorzugt aus Mono-Triarylaminverbindungen. Ganz besonders bevorzugt sind sie gewählt aus den weiter unten angegebenen bevorzugten Ausführungsformen von Lochtransportmaterialien. In der beschriebenen bevorzugten Ausführungsform sind die Verbindung der Formel (I) und die eine oder mehrere weiteren lochtransportierenden Verbindungen bevorzugt jeweils in einem Anteil von mindestens 20% vorhanden, besonders bevorzugt jeweils in einem Anteil von mindestens 30% vorhanden.

Gemäß einer bevorzugten Ausführungsform enthält eine lochtransportierende Schicht enthaltend die Verbindung der Formel (I) zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoOs, WO₃ und ReOs. Nochmals weiterhin bevorzugt sind Komplexe von Bismut in der Oxidationsstufe (III), insbesondere Bismut(III)-Komplexe mit elektronenarmen Liganden, insbesondere Carboxylat-Liganden.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden insbesondere die folgenden Verbindungen:

| | | |
|---|---|---|
| 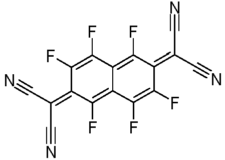 | 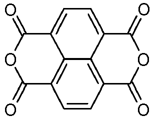 | 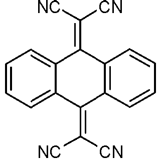 |
| (D-1) | (D-2) | (D-3) |
| 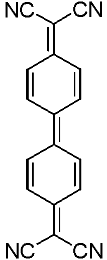 | 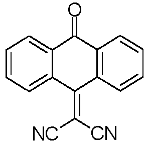 | 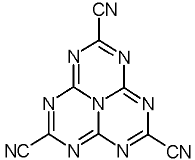 |
| (D-4) | (D-5) | (D-6) |
| 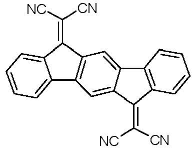 | 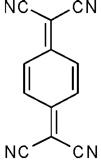 | 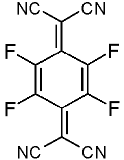 |
| (D-7) | (D-8) | (D-9) |
| 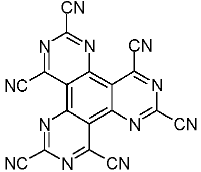 | 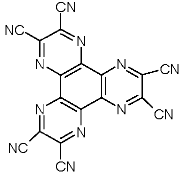 | 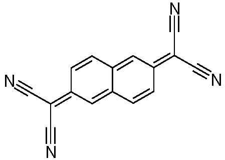 |
| (D-10) | (D-11) | (D-12) |
| 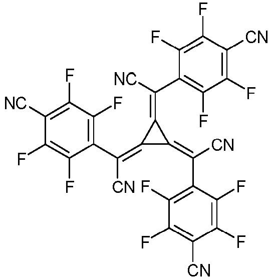 | | |
| (D-13) | | |

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, in einer OLED verwendet. Besonders bevorzugt wird das Hexaazatriphenylenderivat dabei in einer separaten Schicht eingesetzt.

In einer bevorzugten Ausführungsform der vorliegenden Erfindung wird die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen, bevorzugt für phosphoreszierende Emitter, verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Entsprechend ist, wenn die Verbindung der Formel (I) als Matrixmaterial für einen phosphoreszierenden Emitter in der emittierenden Schicht einer OLED eingesetzt wird, eine zweite Matrixverbindung in der emittierenden Schicht vorhanden, die elektronentransportierende Eigenschaften aufweist. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Genauere Angaben zu Mixed-Matrix-Systemen sind unter anderem in der Anmeldung WO 2010/108579 enthalten, deren entsprechende technische Lehre an in diesem Zusammenhang mit einbezogen ist.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen, darunter insbesondere aus denjenigen, die elektronentransportierende Eigenschaften aufweisen.

Im Folgenden werden bevorzugte Ausführungsformen für die verschiedenen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in der noch nicht offengelegten Anmeldung EP 15182993.4 offenbarten erweiterten Benzoindenofluorene, die in den noch nicht offengelegten Anmeldungen EP 15181178.3 und EP 15181177.5 offenbarten Phenoxazine, und die in WO 2016/150544 offenbarten Fluoren-Derivate, die mit Furan-Einheiten oder mit Thiophen-Einheiten verbunden sind.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in der noch nicht offengelegten Anmeldung EP 15180777.3 offenbarten Indeno-Benzofurane, und die in der noch nicht offengelegten Anmeldung EP 15182962.9 offenbarten Phenanthryl-Anthracene.

Bevorzugte Matrixmaterialien für phosphoreszierende emittierende Verbindungen sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, DiazaphospholDerivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Bevorzugt umfasst die erfindungsgemäße OLED zwei oder mehr unterschiedliche lochtransportierende Schichten. Die Verbindung der Formel (I) kann dabei in einer oder in mehreren oder in allen lochtransportierenden Schichten eingesetzt werden. Gemäß einer bevorzugten Ausführungsform wird die Verbindung der Formel (I) in genau einer oder genau zwei lochtransportierenden Schichten eingesetzt, und in den weiteren vorhandenen lochtransportierenden Schichten werden andere Verbindungen eingesetzt, bevorzugt aromatische Aminverbindungen. Weitere Verbindungen, die neben den Verbindungen der Formel (I) bevorzugt in lochtransportierenden Schichten der erfindungsgemäßen OLEDs eingesetzt werden, sind insbesondere Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938, WO 2014/015935 und WO 2015/082056), Spiro-Dibenzopyran-Amine (z. B. gemäß WO 2013/083216), Dihydroacridin-Derivate (z. B. gemäß WO 2012/150001), Spirodibenzofurane und Spirodibenzothiophene, z.B. gemäß WO 2015/022051 und den noch nicht offen gelegten Anmeldungen PCT/EP2015/002475 und PCT/EP2016/000084, Phenanthren-Diarylamine, z.B. gemäß WO 2015/131976, Spiro-Tribenzotropolone, z.B. gemäß der noch nicht offen gelegten Anmeldung PCT/EP2015/002225, Spirobifluorene mit meta-Phenyldiamingruppen, z.B. gemäß der noch nicht offen gelegten Anmeldung PCT/EP2015/002112, Spiro-Bisacridine, zB. gemäß WO 2015/158411, Xanthen-Diarylamine, z.B. gemäß WO 2014/072017, und 9,10-Dihydroanthracen-Spiroverbindungen mit Diarylaminogruppen gemäß WO 2015/086108.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Beispiele

### A) Synthesebeispiele

### Synthese der Verbindung Biphenyl-4-yl-(9-phenyl-9H-carbazol-2-yl)-[1,1';3',1"]terphenyl-2-yl-amin* (1-1) sowie der Verbindungen (1-2) bis (1-22)

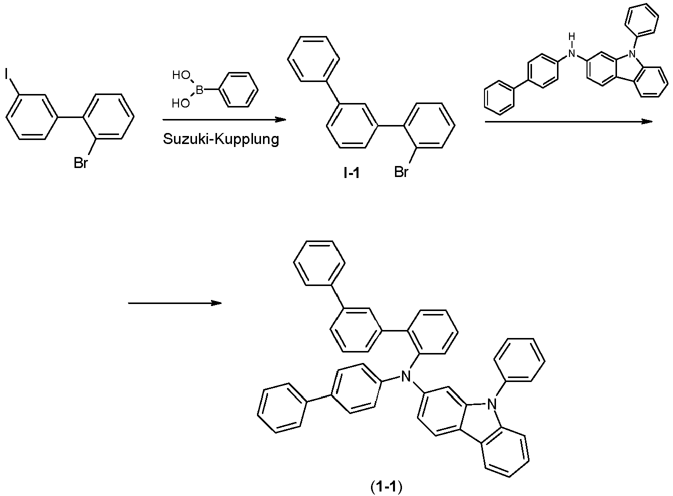

* Referenzverbindung

### Synthese der Zwischenstufe I-1: 2-Bromo-[1,1';3',1"]-terphenyl

14,3 g (117 mmol) Phenyl-boronsäure, 40 g (111,4 mmol) 2-Bromo-3'-iodo-biphenyl und 84 mL einer wässrigen 2 M K₂CO₃-Lösung (168 mmol) werden in 400 mL Toluol suspendiert. Zu dieser Suspension werden 1,2 g (1,2mmol) Tetrakis-(triphenyl)phosphin-palladium(0) gegeben. Die Reaktionsmischung wird 16 h unter Rückfluss erhitzt. Nach Erkalten wird die organische Phase abgetrennt, über Kieselgel filtriert, dreimal mit 150 mL Wasser gewaschen und anschließend zur Trockene eingeengt. Nach Filtration des Rohproduktes über Kieselgel mit Heptan/Essigsäureester erhält man 29 g (85%) 2-Bromo-[1,1';3',1"]-terphenyl.

Analog dazu werden folgende Verbindungen hergestellt:

| | **Edukt 1** | **Edukt 2** | **Produkt** |
|---|---|---|---|
| **I-2** | 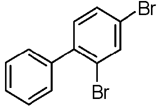 | 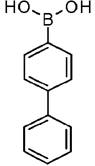 | 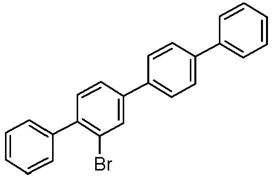 |
| **I-3** | 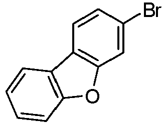 | 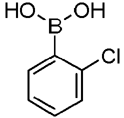 | 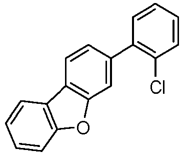 |
| **I-4** | 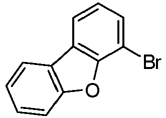 | 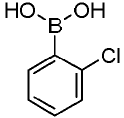 | 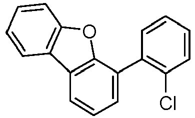 |
| **I-5** | 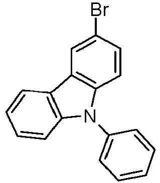 | 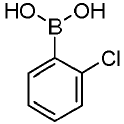 | 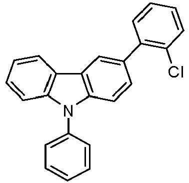 |
| **I-6** | 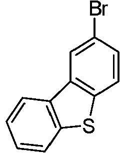 | 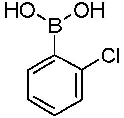 | 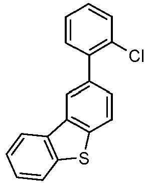 |
| **I-7** | 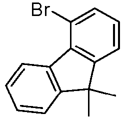 | 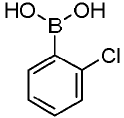 | 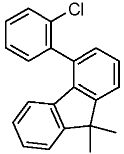 |
| **I-8** | 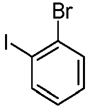 | 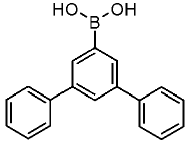 | 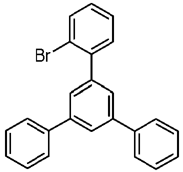 |
| **I-9** | 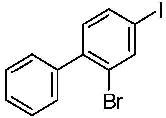 | 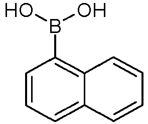 | 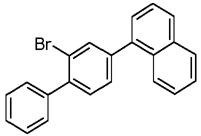 |
| **I-10** | 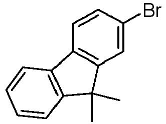 | 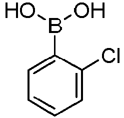 | 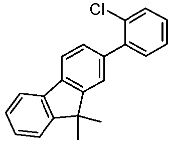 |
| **I-11** | 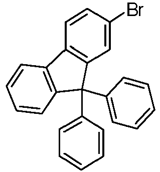 | 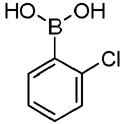 | 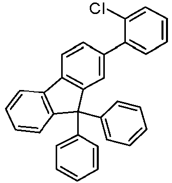 |
| **I-12** | 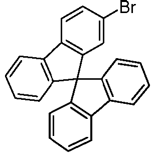 | 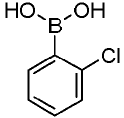 | 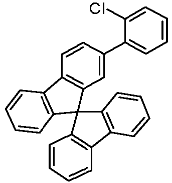 |

### Synthese der Zwischenstufe I-13: 5-chloro-9,9-dimethyl-2-phenylfluoren

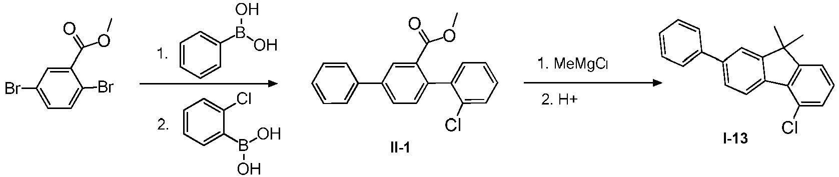

### Zwischenstufe II-1

8.3 g Phenylboronsäure (68 mmol) und 20 g di-Brom-carbonsäureester-Derivat (68 mmol) werden in 400mL Toluol, 160 mL Ethanol und 80 mL Wasser suspendiert. 14.4 g Natriumcarbonat werden dazugegeben. Die Lösung wird entgast und mit N₂ gesättigt. Danach wird sie mit 0,79 g (0,68 mmol) Pd(Ph₃P)₄ versetzt. Die Reaktionsmischung wird 4 h unter Schutzatmosphäre erhitzt (80°C). Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Heptan/Essigester wird der verbleibende Rückstand aus EtOH umkristallisiert. Die Ausbeute beträgt 11.0 g (55% d. Th).

5,9 g 2-Chlor-phenylboronsäure (38 mmol) und 11 g des Brom-Derivats (38 mmol) werden in 200 mL Toluol und 70 mL Wasser suspendiert. 7,2 g Natriumcarbonat (67,6 mmol) werden dazugegeben. Die Lösung wird entgast und mit N₂ gesättigt. Danach wird sie mit 140 mg (0,15 mmol) Pd₂(dba)₃ und 250 mg SPhos (0,3 mmol) versetzt. Die Reaktionsmischung wird 12 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert.

Nach Filtration des Rohproduktes über Kieselgel mit Toluol wird der verbleibende Rückstand aus EtOH umkristallisiert. Die Ausbeute beträgt 10,4 g (85% d. Th).

Analog dazu werden folgende Verbindungen hergestellt:

| | **Edukt 1** | **Boronsäure 1** | **Boronsäure 2** | **Produkt** |
|---|---|---|---|---|
| **II-2** | 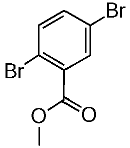 | 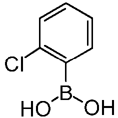 | 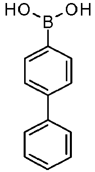 | 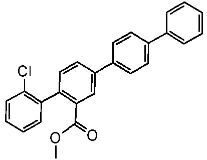 |
| **II-3** | 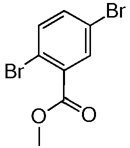 | 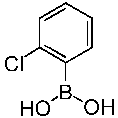 | 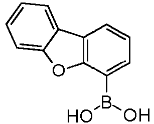 | 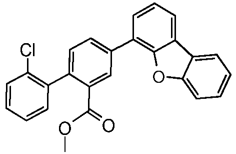 |
| **ii-4** | 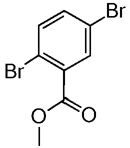 | 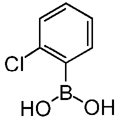 | 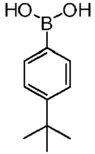 | 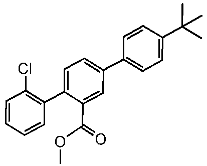 |

### Zwischenstufe I-13

10,4 g (32,2 mmol) Zwischenstufe II-1 werden in einem ausgeheizten Kolben in 100 mL getrocknetem THF gelöst. Die Lösung wird mit N₂ gesättigt. Die klare Lösung wird auf -5°C abgekühlt und dann werden 32,2 mL (96,7 mmol) einer 3M-Methylmagnesiumchlorid-Lösung zugegeben. Die Reaktionsmischung wird langsam auf Raumtemperatur erwärmt und dann mit Ammoniumchlorid gequencht. Das Gemisch wird im Anschluss zwischen Essigester und Wasser verteilt, die organische Phase dreimal mit Wasser gewaschen, über Na₂SO₄ getrocknet und einrotiert. Die einrotierte Lösung wird in Toluol gelöst und mit 8 g Amberlyst 15 versetzt. Der Ansatz wird auf 110°C erhitzt und 4 h auf dieser Temperatur gehalten.

Dabei fällt ein weißer Feststoff aus. Der Ansatz wird dann auf Raumtemperatur abgekühlt, der ausgefallene Feststoff wird abgesaugt und mit Heptan nachgewaschen. Der Rückstand wird im Vakuum bei 40°C getrocknet. Nach Filtration des Rohproduktes über Kieselgel mit Heptan:Essigsester, 1:1 erhält man 9,3 g (90% der Theorie) des Produkts.

Analog dazu werden folgende Verbindungen hergestellt:

| | **Edukt 1** | **Edukt 2** | **Produkt** |
|---|---|---|---|
| **I-13** | 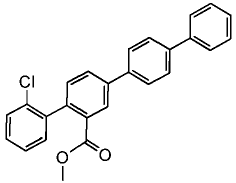 | | 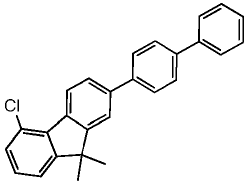 |
| | | MeMgCl | |
| **I-14** | 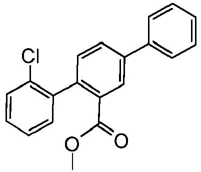 | PhLi | 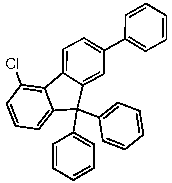 |
| **I-15** | 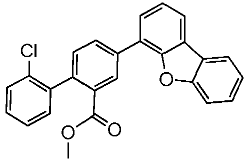 | MeMgBr | 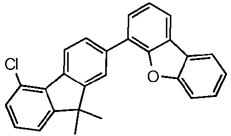 |
| **I-16** | 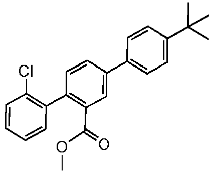 | MeMgCl | 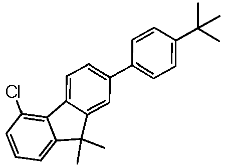 |
| **1-17** | 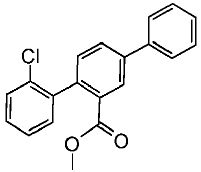 | 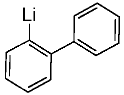 | 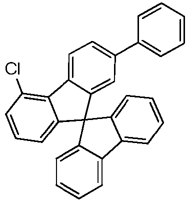 |

### Synthese von Biphenyl-4-yl-(9-phenyl-9H-carbazol-2-yl)-[1,1';3',1"]terphenyl-2-yl-amin) (Verbindung 1-1) sowie den Verbindungen (1-2) bis (1-14)

16,2 g Biphenyl-4-yl-(9-phenyl-9H-carbazol-2-yl)-amin (48,5 mmol) und 15 g 2-Bromo-[1,1';3',1"]-terphenyl (48,5 mmol) werden in 300 mL Toluol gelöst. Die Lösung wird entgast und mit N₂ gesättigt. Danach wird sie mit 1,94 mL (1,94 mmol) einer 1 M Tri-tert-Butylphosphin-Lösung und 0,89 g (0,97 mmol) Pd₂(dba)₃ versetzt. Anschließend werden 7,0 g Natrium-tert-butylat (72,8 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert. Der Rückstand 22,3 g (72% der Theorie) wird abschließend im Hochvakuum sublimiert.

Analog dazu werden folgende Verbindungen hergestellt:

| | **Edukt 1** | **Edukt 2** | **Produkt** |
|---|---|---|---|
| **1-2*** | 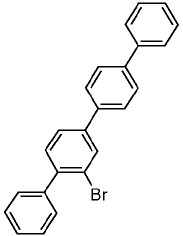 | 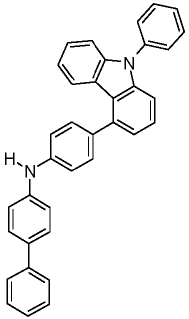 | 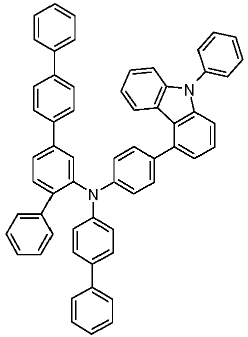 |
| **1-3*** | 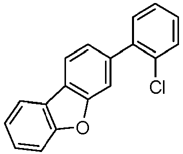 | 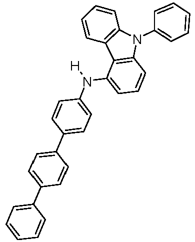 | 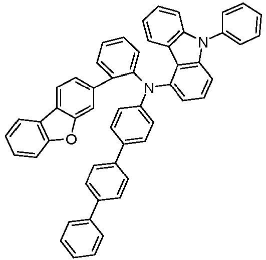 |
| **1-4** | 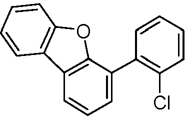 | 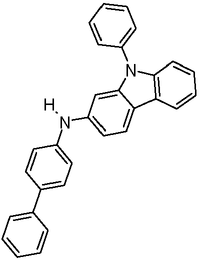 | 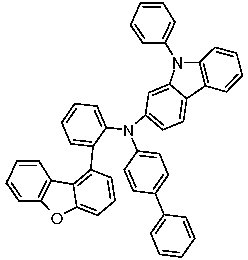 |
| **1-5** | 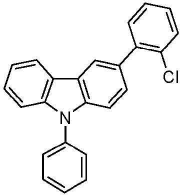 | 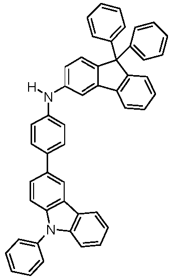 | 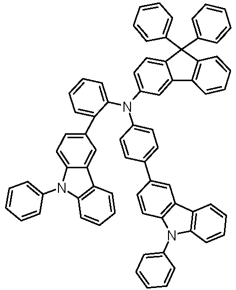 |
| **1-6** | 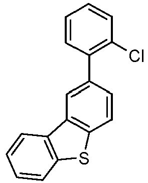 | 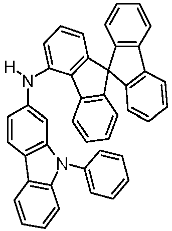 | 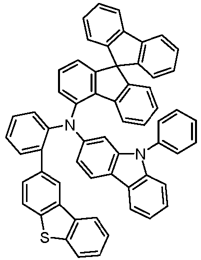 |
| **1-7** | 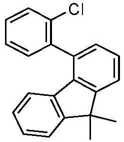 | 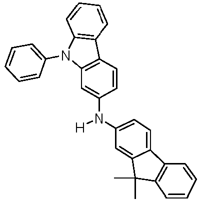 | 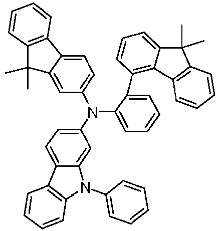 |
| **1-8*** | 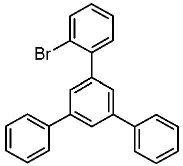 | 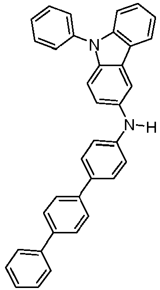 | 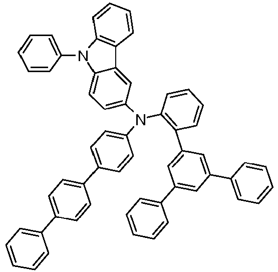 |
| **1-9*** | 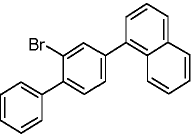 | 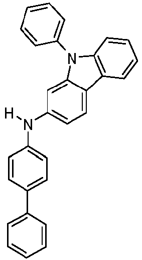 | 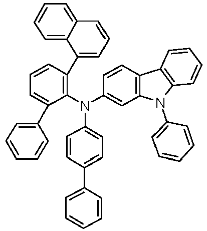 |
| **1-10*** | 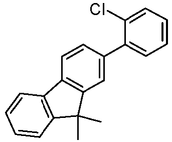 | 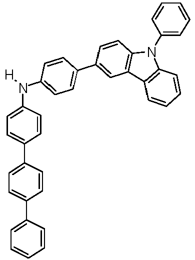 | 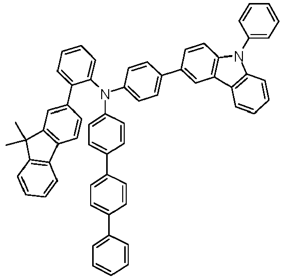 |
| **1-11*** | 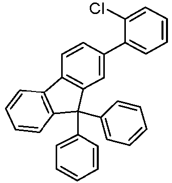 | 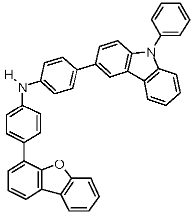 | 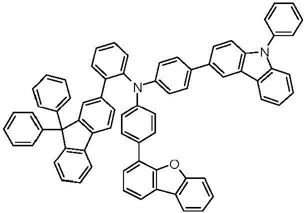 |
| **1-12*** | 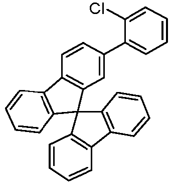 | 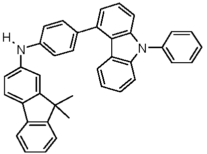 | 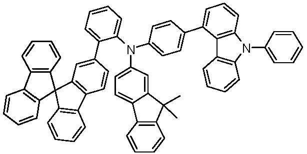 |
| **1-13*** | 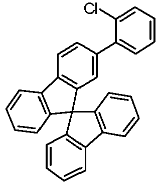 | 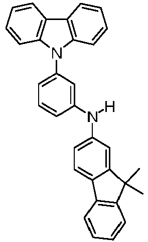 | 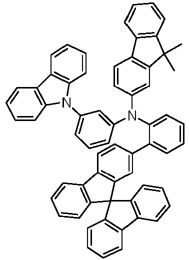 |
| **1-14** | 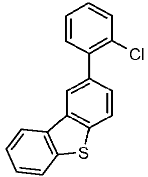 | 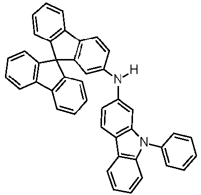 | 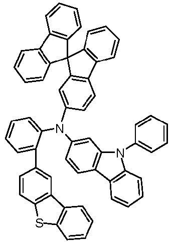 |
| **1-15*** | 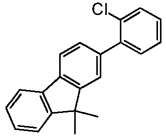 | 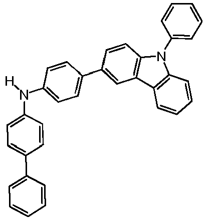 | 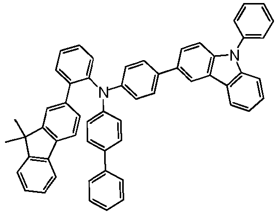 |
| **1-16*** | 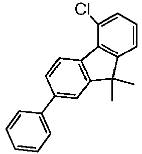 | 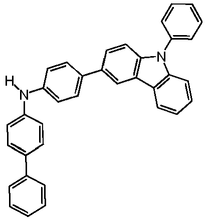 | 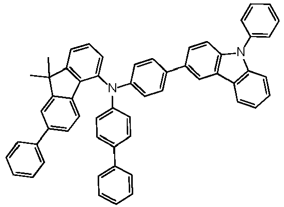 |
| **1-17** | 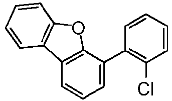 | 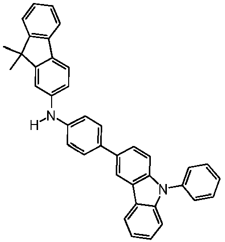 | 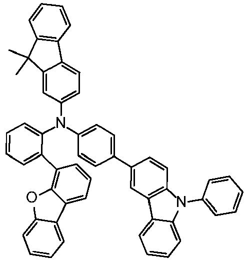 |
| **1-18** | 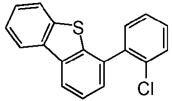 | 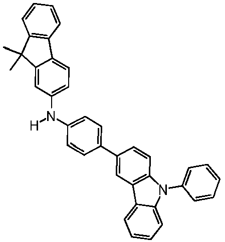 | 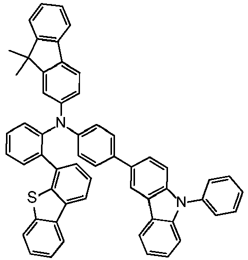 |
| **1-19*** | 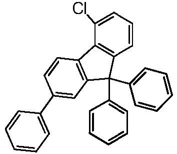 | 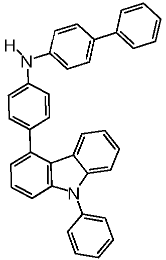 | 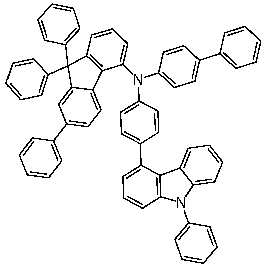 |
| **1-20*** | 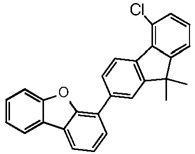 | 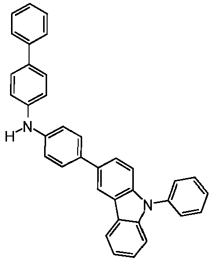 | 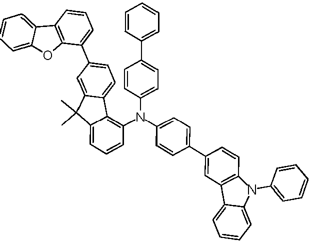 |
| **1-21*** | 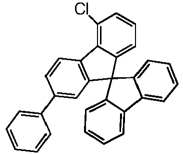 | 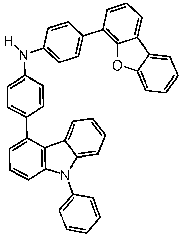 | 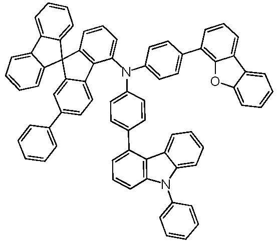 |
| **1-22*** | 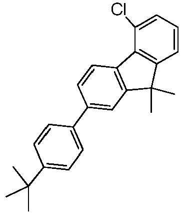 | 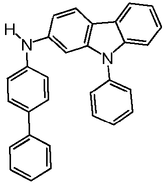 | 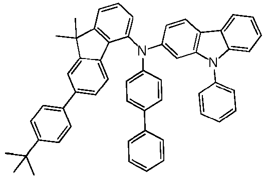 |

| | | | |
|---|---|---|---|
| * Referenzverbindung | | | |

### B) Device-Beispiele

Es werden Beispiel-OLEDs gemäß der folgenden allgemeinen Vorschrift hergestellt:

Als Substrate werden Glasplättchen, die mit einer 50 nm dicken Schicht von strukturiertem ITO (Indium-Zinn-Oxid) beschichtet sind, verwendet. Darauf wird die folgende Schichtstruktur aufgebracht: Lochinjektionsschicht (HIL) / Lochtransportschicht (HTL) / Elektronenblockierschicht (EBL) / Emissionsschicht (EML) / Elektronentransportschicht (ETL) / Elektroneninjektionsschicht (EIL) / Kathode. Die Kathode besteht aus einer Aluminiumschicht einer Dicke von 100 nm. Die Materialien, die in den entsprechenden Schichten der Beispiel-OLEDs verwendet werden, sind in Tabelle 1 genannt, und die chemischen Strukturen dieser Materialien sind in Tabelle 3 aufgeführt.

Die Materialien werden mittels thermischer Gasphasenabscheidung in einer Vakuumkammer aufgetragen. Dabei besteht die Emissionsschicht immer aus zwei Matrixmaterialien (Hosts) und einem emittierenden Dotanden (Emitter), der den Matrixmaterialien in einem bestimmten Volumenanteil durch Co-Evaporation beigemischt wird. Die Prozentangaben hinter den Materialien sind daher als Volumen-Prozent zu verstehen. Entsprechendes gilt für andere Schichten als die emittierende Schicht. Diese können ebenfalls entsprechend zwei oder mehr Materialien enthalten.

Die OLEDs werden standardmäßig charakterisiert. Hierfür werden die Elektrolumineszenzspektren und die externe Quanteneffizienz (EQE, gemessen in %), als Funktion der Leuchtdichte, berechnet aus Stromfluss-/Spannung-/Leuchtdichte-Kennlinien (IUL-Kennlinien), bestimmt. Dabei werden Lambert'sche Emissionscharakteristika angenommen. Weiterhin wird die Betriebsspannung bestimmt (U, in V).

EQE @ 1000 cd/m² stellt die externe Quanteneffizienz bei einer Betriebs-Leuchtdichte von 1000 cd/m² dar. EQE @ 10 mA/cm² stellt die externe Quanteneffizienz bei einer Stromdichte von 10 mA/cm² dar.

### Verwendung der Verbindungen in der EBL von grün phosphoreszierenden OLEDs

OLED-Beispiele V1 bis E12 weisen den in Tabelle 1a gezeigten Schichtaufbau auf, wobei in der EBL jeweils eine der Verbindungen 1-1*, 1-2*, 1-3*, 1-4, 1-6, 1-7, 1-10*, 1-14, 1-15*, 1-16*, 1-17 und 1-18 vorliegt (Referenzverbindungen sind mit * gekennzeichnet).

In allen Fällen werden mit den OLEDs gute Ergebnisse bzgl. Betriebsspannung und EQE erzielt (Tabelle 2a). Weiterhin weisen die erfindungsgemäßen OLEDs eine gute Lebensdauer auf.

Auch mit Verbindungen mit N-gebundenem Carbazol, wie beispielsweise der Verbindung 1-13, können OLEDs mit vergleichbaren Leistungsdaten wie in Tabelle 2a gezeigt erhalten werden.

| **Tabelle 1a: Aufbau der OLEDs** | | | | | | | |
|---|---|---|---|---|---|---|---|
| **Bsp.** | **HIL** | **HTL** | **EBL** | **EML** | **HBL** | **ETL** | **EIL** |
| V1 | HTM: F4TCNQ(5%) 20 nm | HTM 215 nm | HTMV1 10 nm | H1 (59%): H2(29%): TEG(12%) 30 nm | ETM 10 nm | ETM:LiQ(50%) 30 nm | LiQ 1 nm |
| E1 | s.o. | s.o. | 1-1 | s.o. | s.o. | s.o. | s.o. |
| | | | 10 nm | | | | |
| E2 | s.o. | s.o. | 1-2 | s.o. | s.o. | s.o. | s.o. |
| | | | 10 nm | | | | |
| E3 | s.o. | s.o. | 1-3 | s.o. | s.o. | s.o. | s.o. |
| | | | 10 nm | | | | |
| E4 | s.o. | s.o. | 1-4 | s.o. | s.o. | s.o. | s.o. |
| | | | 10 nm | | | | |
| E5 | s.o. | s.o. | 1-6 | s.o. | s.o. | s.o. | s.o. |
| | | | 10 nm | | | | |
| E6 | s.o. | s.o. | 1-7 | s.o. | s.o. | s.o. | s.o. |
| | | | 10 nm | | | | |
| E7 | s.o. | s.o. | 1-10 | s.o. | s.o. | s.o. | s.o. |
| | | | 10 nm | | | | |
| E8 | s.o. | s.o. | 1-14 | s.o. | s.o. | s.o. | s.o. |
| | | | 10 nm | | | | |
| E9 | s.o. | s.o. | 1-15 | s.o. | s.o. | s.o. | s.o. |
| | | | 10 nm | | | | |
| E10 | s.o. | s.o. | 1-16 | s.o. | s.o. | s.o. | s.o. |
| | | | 10 nm | | | | |
| E11 | s.o. | s.o. | 1-17 | s.o. | s.o. | s.o. | s.o. |
| | | | 10 nm | | | | |
| E12 | s.o. | s.o. | 1-18 | s.o. | s.o. | s.o. | s.o. |
| | | | 10 nm | | | | |

| **Tabelle 2a: Daten der OLEDs** | | |
|---|---|---|
| **Beispiel** | **U @ 1000 cd/m²** | **EQE @ 1000 cd/m²** |
| | [V] | % |
| V1 | 3.3 | 15.2 |
| E1 | 3.0 | 17.2 |
| E2 | 3.1 | 19.1 |
| E3 | 3.1 | 17.4 |
| E4 | 3.0 | 17.1 |
| E5 | 3.4 | 16.6 |
| E6 | 3.0 | 17.1 |
| E7 | 3.2 | 18.8 |
| E8 | 3.2 | 16.3 |
| E9 | 3.1 | 17.9 |
| E10 | 3.2 | 18.5 |
| E11 | 2.9 | 17.3 |
| E12 | 2.9 | 17.4 |

### Verwendung der Verbindungen in der HIL und HTL von blau fluoreszierenden OLEDs

OLED-Beispiele E13 bis E15 weisen den in Tabelle 1b gezeigten Schichtaufbau auf, wobei in den lochtransportierenden Schichten HIL und HTL jeweils eine der Verbindungen 1-15, 1-17 und 1-18 vorliegt.

In allen Fällen werden mit den OLEDs gute Ergebnisse bzgl. Betriebsspannung und EQE erzielt (Tabelle 2b). Weiterhin weisen die OLEDs eine gute Lebensdauer auf.

| **Tabelle 1b: Aufbau der OLEDs** | | | | | | |
|---|---|---|---|---|---|---|
| **Bsp.** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| E13 | 1-15: F4TCNQ (5%) | 1-15 | EBL 10 nm | H:SEB(5%) 20 nm | ETM:LiQ(50%) 30 nm | LiQ 1 nm |
| | 20 nm | 180 nm | | | | |
| E14 | 1-17: F4TCNQ (5%) | 1-17 | s.o. | s.o. | s.o. | s.o. |
| | 20 nm | 180 nm | | | | |
| E15 | 1-18: F4TCNQ (5%) | 1-18 | s.o. | s.o. | s.o. | s.o. |
| | 20 nm | 180 nm | | | | |

| **Tabelle 2b: Daten der OLEDs** | | |
|---|---|---|
| | **U @ 10 mA/cm²** | **EQE @ 10 mA/cm²** |
| | [V] | [%] |
| E13 | 4.8 | 9.0 |
| E14 | 4.5 | 8.1 |
| E15 | 4.4 | 8.5 |

### Verwendung der Verbindungen in der EBL von blau fluoreszierenden OLEDs

OLED-Beispiele E16 und E17 weisen den in Tabelle 1c gezeigten Schichtaufbau auf, wobei in der EBL jeweils eine der erfindungsgemäßen Verbindungen 1-15 und 1-16 vorliegt.

In allen Fällen werden mit den OLEDs gute Ergebnisse bzgl. Betriebsspannung und EQE erzielt (Tabelle 2c). Weiterhin weisen die OLEDs eine gute Lebensdauer auf.

| **Tabelle 1c: Aufbau der OLEDs** | | | | | | |
|---|---|---|---|---|---|---|
| **Bsp.** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| E-16 | HTM: F4TCNQ (5%) | HTM 180 nm | 1-15 | H:SEB(5%) 20 nm | ETM:LiQ(50%) 30 nm | LiQ 1 nm |
| | 20 nm | | 10 nm | | | |
| E-17 | s.o. | s.o. | 1-16 | s.o. | s.o. | s.o. |
| | | | 10 nm | | | |

| **Tabelle 2c: Daten der OLEDs** | | |
|---|---|---|
| | **U @ 10 mA/cm²** | **EQE @ 10 mA/cm²** |
| | [V] | [%] |
| E-16 | 3.8 | 8.5 |
| E-17 | 3.8 | 9.3 |

| **Tabelle 3: Strukturen der Materialien** | | |
|---|---|---|
| 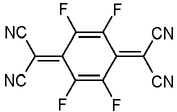 | 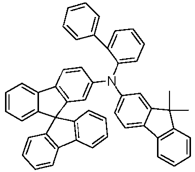 | 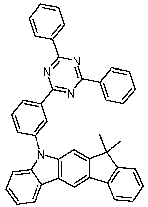 |
| F4TCNQ | HTM | H1 |
| 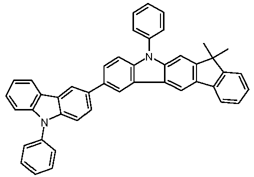 | 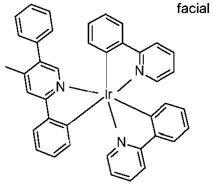 | 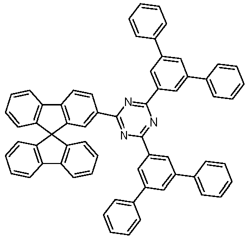 |
| H2 | TEG | ETM |
| 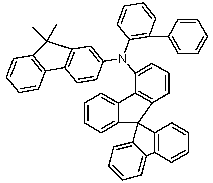 | 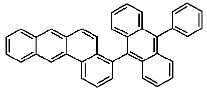 | 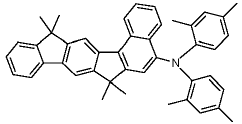 |
| EBL | H | SEB |
| 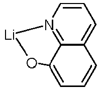 | 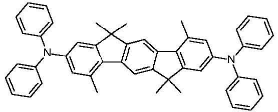 | 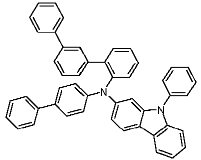 |
| LiQ | HTMV1 | 1-1 |
| 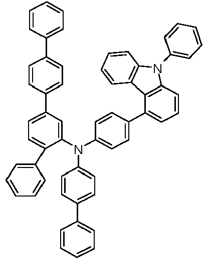 | 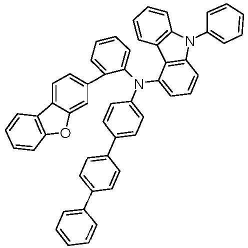 | 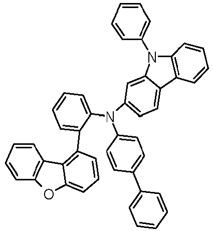 |
| 1-2 | 1-3 | 1-4 |
| 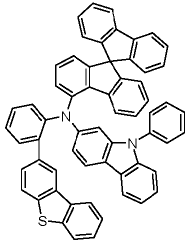 | 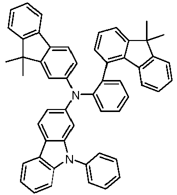 | 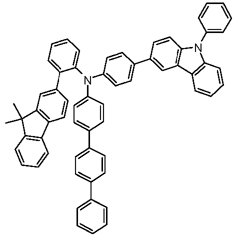 |
| 1-6 | 1-7 | 1-10 |
| 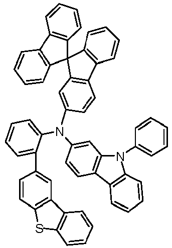 | 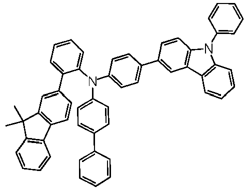 | 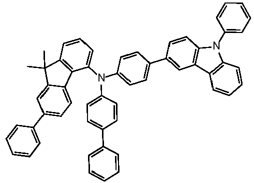 |
| 1-14 | 1-15 | 1-16 |
| 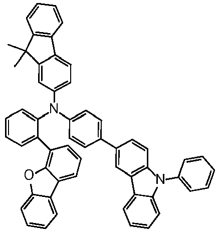 | 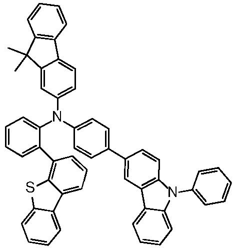 | |
| 1-17 | 1-18 | |

## Patentansprüche

1. Verbindung gemäß Formel (I) 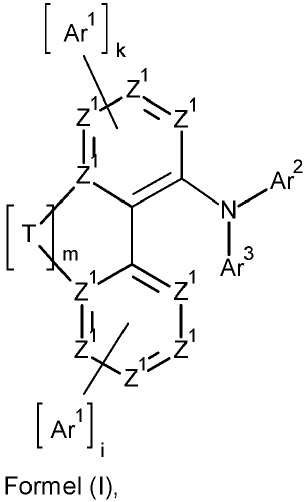 wobei die Untereinheit 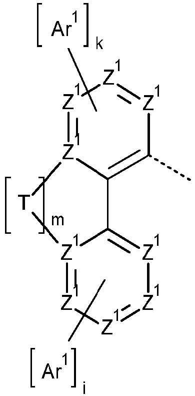
der Formel (I) gewählt ist aus den folgenden Strukturen:
| | | |
|---|---|---|
| 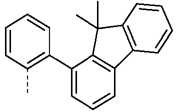 | | |
| Formel (I-A-45) | | |
| 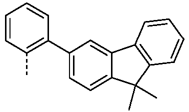 | 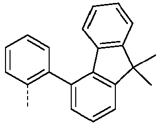 | |
| Formel (I-A-46) | Formel (I-A-47) | |
| 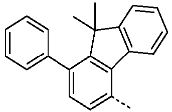 | 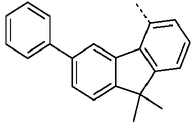 | 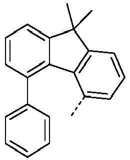 |
| Formel (I-A-49) | Formel (I-A-50) | Formel (I-A-51) |
| 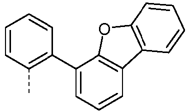 | | 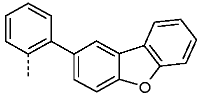 |
| Formel (I-A-53) | | Formel (I-A-54) |
| 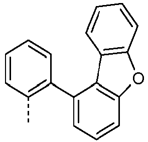 | | 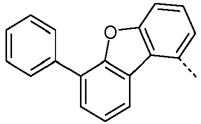 |
| Formel (I-A-55) | | Formel (I-A-57) |
| 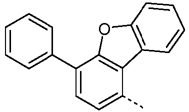 | 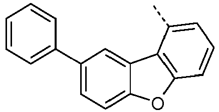 | 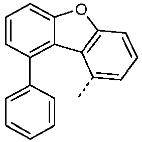 |
| Formel (I-A-58) | Formel (I-A-59) | Formel (I-A-60) |
| 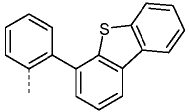 | | 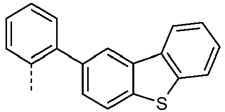 |
| Formel (I-A-62) | | Formel (I-A-63) |
| 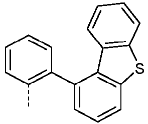 | | 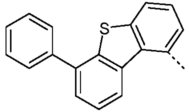 |
| Formel (I-A-64) | | Formel (I-A-66) |
| 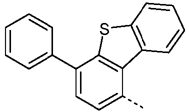 | 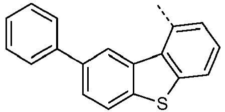 | 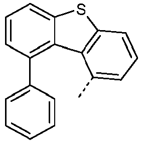 |
| Formel (I-A-67) | Formel (I-A-68) | Formel (I-A-69) |
| 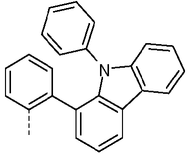 | | 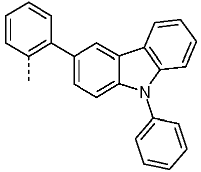 |
| Formel (I-A-71) | | Formel (I-A-72) |
| 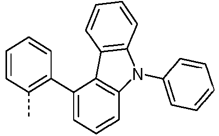 | | 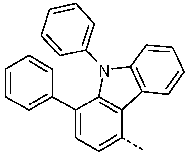 |
| Formel (I-A-73) | | Formel (I-A-75) |
| 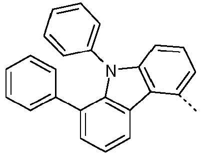 | 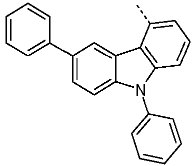 | 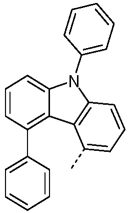 |
| Formel (I-A-76) | Formel (I-A-77) | Formel (I-A-78) |
| 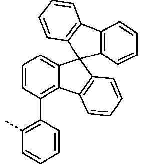 | | 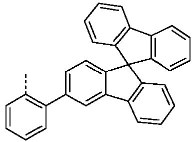 |
| Formel (I-A-80) | | Formel (I-A-81) |
| 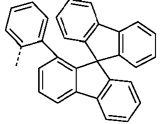 | | 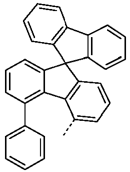 |
| Formel (I-A-82) | | Formel (I-A-84) |
| 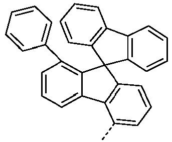 | 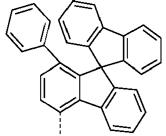 | 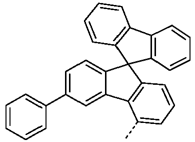 |
| Formel (I-A-85) | Formel (I-A-86) | Formel (I-A-87) |
| 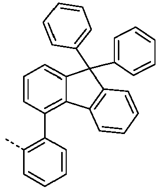 | | 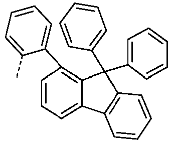 |
| Formel (I-A-88) | | Formel (I-A-90) |
| 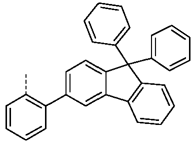 | | 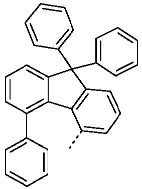 |
| Formel (I-A-91) | | Formel (I-A-93) |
| 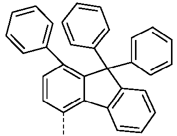 | 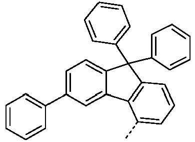 | 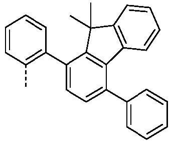 |
| Formel (I-A-94) | Formel (I-A-95) | Formel (I-A-96) |
| 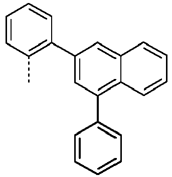 | | 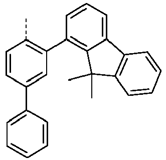 |
| Formel (I-A-109) | | Formel (I-A-111) |
| 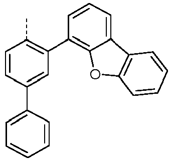 | 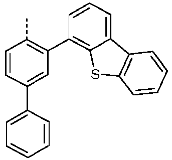 | 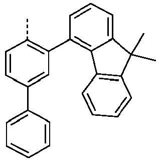 |
| Formel (I-A-112) | Formel (I-A-113) | Formel (I-A-114) |
| 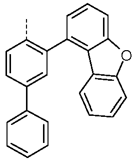 | 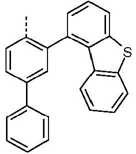 | |
| Formel (I-A-115) | Formel (I-A-116) | |
| 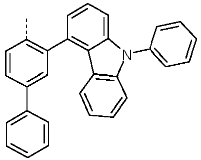 | 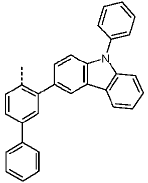 | |
| Formel (I-A-118) | Formel (I-A-119) | |
| 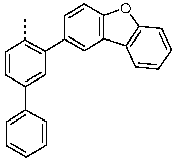 | 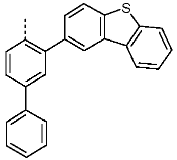 | |
| Formel (I-A-121) | Formel (I-A-122) | |
wobei die gestrichelte Linie die Bindung an das N-Atom in Formel (I) darstellt, und wobei für die auftretenden Variablen gilt:
Ar² entspricht einer Formel (A) oder (B) 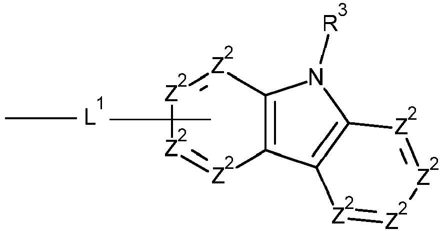 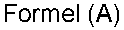 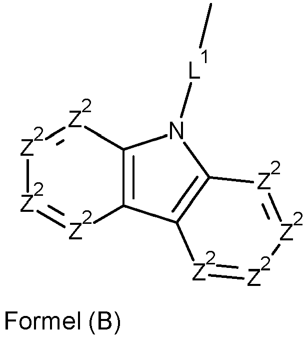
Z² ist bei jedem Auftreten gleich oder verschieden CR³ oder N, wobei Z² gleich C ist, wenn eine Gruppe L¹ daran gebunden ist;
L¹ ist eine Einfachbindung, oder ein aromatisches Ringsystem mit 6 bis 30 aromatischen Ringatomen, das mit einem oder mehreren Resten R³ substituiert sein kann, oder ein heteroaromatisches Ringsystem mit 5 bis 30 aromatischen Ringatomen, das mit einem oder mehreren Resten R³ substituiert sein kann;
Ar³ entspricht einer Formel (A), einer Formel (B) oder ist ein aromatisches Ringsystem mit 6 bis 30 aromatischen Ringatomen, das mit einem oder mehreren Resten R⁴ substituiert sein kann, oder ein heteroaromatisches Ringsystem mit 5 bis 30 aromatischen Ringatomen, das mit einem oder mehreren Resten R⁴ substituiert sein kann;
R³, R⁴ sind bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ bzw. R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch - R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R⁵ ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁶ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können.

2. Verbindung gemäß Anspruch 1, **dadurch gekennzeichnet, dass** Ar² gewählt ist aus Gruppen der folgenden Formeln
| | | |
|---|---|---|
| 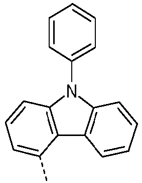 | 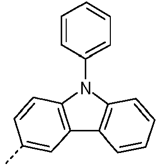 | 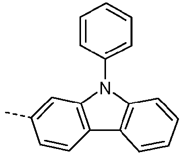 |
| Ar²-1 | Ar²-2 | Ar²-3 |
| 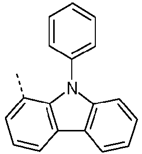 | 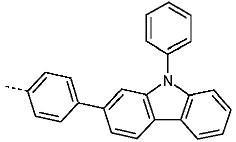 | 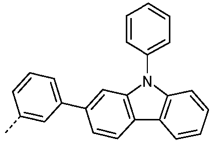 |
| Ar²-4 | Ar²-5 | Ar²-6 |
| 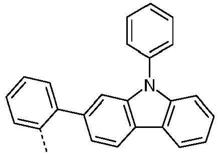 | 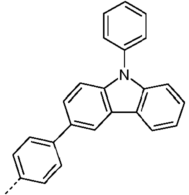 | 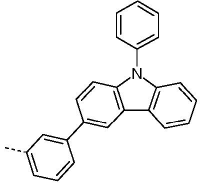 |
| Ar²-7 | Ar²-8 | Ar²-9 |
| 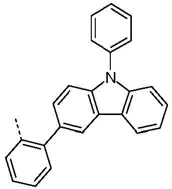 | 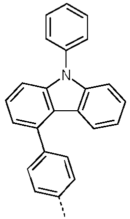 | 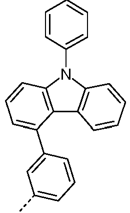 |
| Ar²-10 | Ar²-11 | Ar²-12 |
| 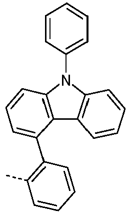 | 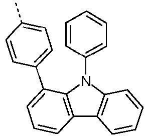 | 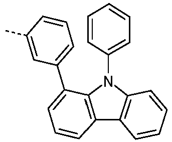 |
| Ar²-13 | Ar²-14 | Ar²-15 |
| 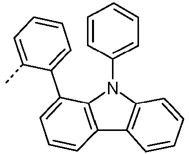 | 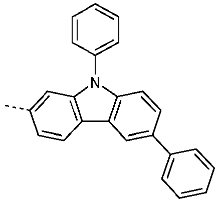 | 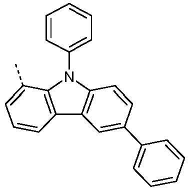 |
| Ar²-16 | Ar²-17 | Ar²-18 |
| 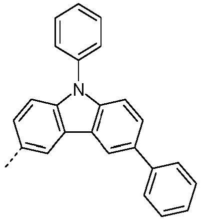 | 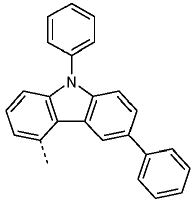 | 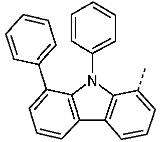 |
| Ar²-19 | Ar²-20 | Ar²-21 |
| 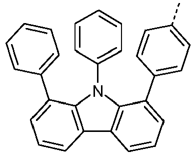 | 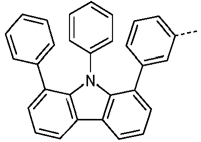 | 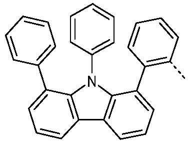 |
| Ar²-22 | Ar²-23 | Ar²-24 |
| 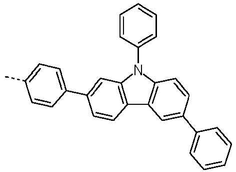 | 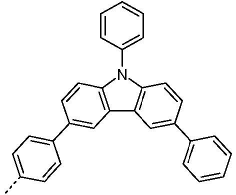 | 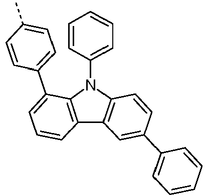 |
| Ar²-25 | Ar²-26 | Ar²-27 |
| 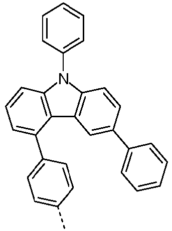 | 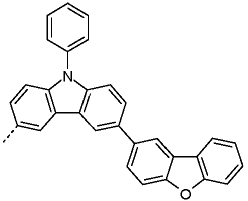 | 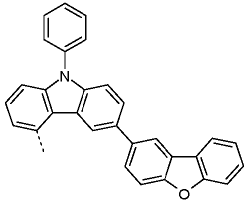 |
| Ar²-28 | Ar²-29 | Ar²-30 |
| 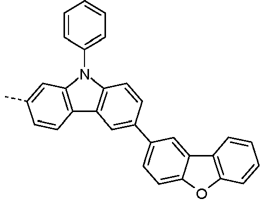 | 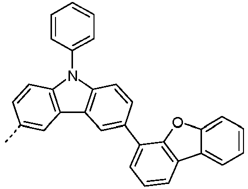 | 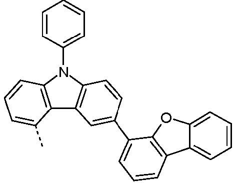 |
| Ar²-31 | Ar²-32 | Ar²-33 |
| 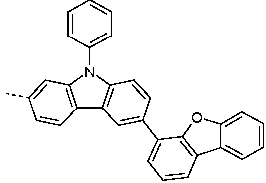 | 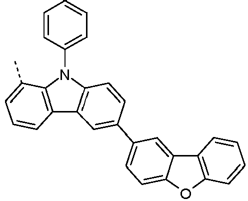 | 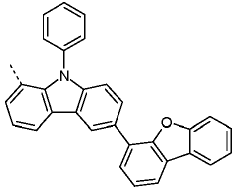 |
| Ar²-34 | Ar²-35 | Ar²-36 |
| 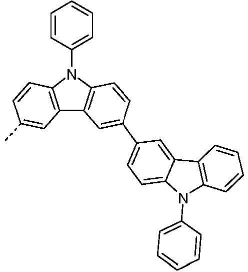 | 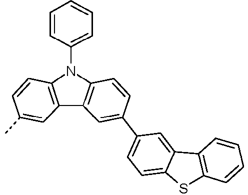 | 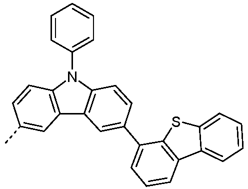 |
| Ar²-37 | Ar²-38 | Ar²-39 |
| 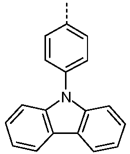 | 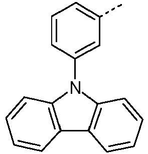 | 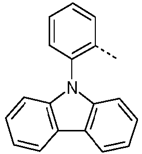 |
| Ar²-40 | Ar²-41 | Ar²-42 |
| 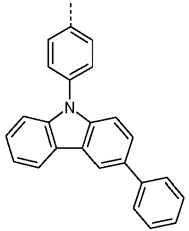 | 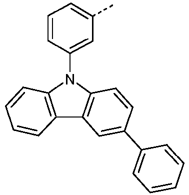 | 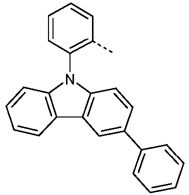 |
| Ar²-43 | Ar²-44 | Ar²-45 |
| 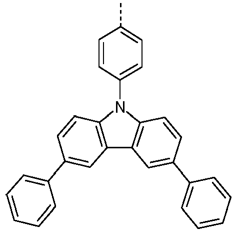 | 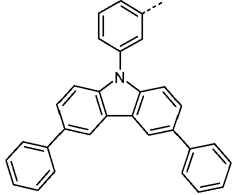 | 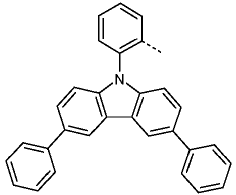 |
| Ar²-46 | Ar²-47 | Ar²-48 |
| 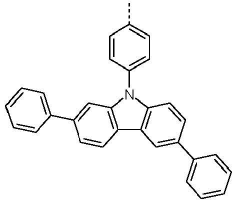 | 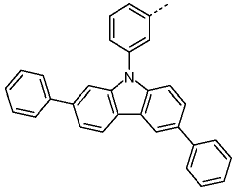 | 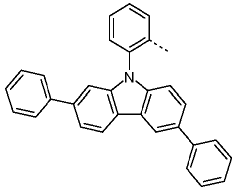 |
| Ar²-49 | Ar²-50 | Ar²-51 |
| 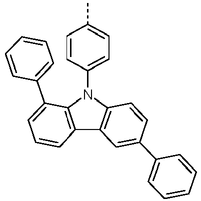 | 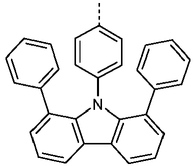 | 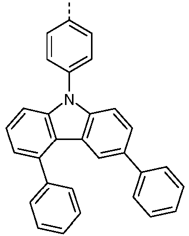 |
| Ar²-52 | Ar²-53 | Ar²-54 |
| 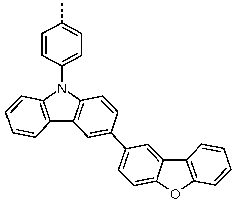 | 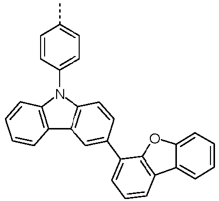 | 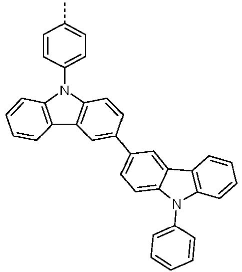 |
| Ar²-55 | Ar²-56 | Ar²-57 |
| 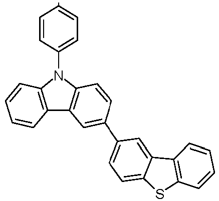 | 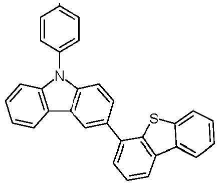 | 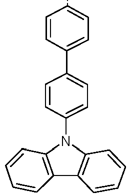 |
| Ar²-58 | Ar²-59 | Ar²-60 |
| 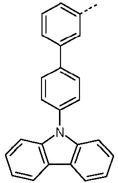 | 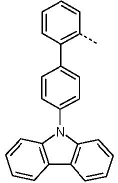 | 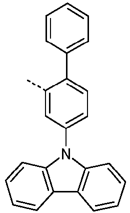 |
| Ar²-61 | Ar²-62 | Ar²-63 |
| 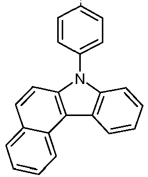 | 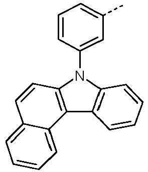 | 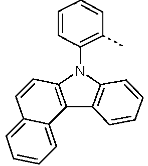 |
| Ar²-64 | Ar²-65 | Ar²-66 |
| 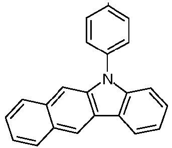 | 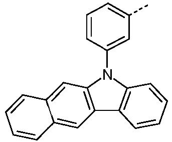 | 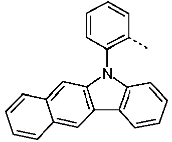 |
| Ar²-67 | Ar²-68 | Ar²-69 |
| 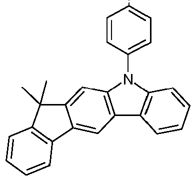 | 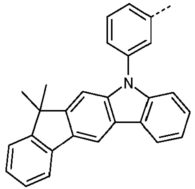 | 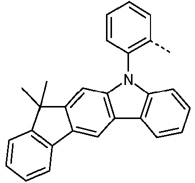 |
| Ar²-70 | Ar²-71 | Ar²-72 |
| 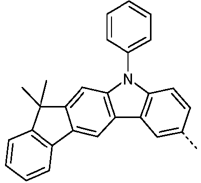 | 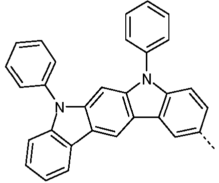 | 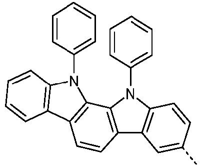 |
| Ar²-73 | Ar²-74 | Ar²-75 |
| 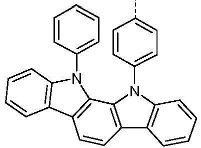 | | |
| Ar²-76 | | |

3. Verbindung gemäß Anspruch 1 oder 2, **dadurch gekennzeichnet, dass** Ar³ gewählt ist aus Phenyl, Biphenyl, Terphenyl, Fluorenyl, Fluorenyl-Phenyl, Naphthyl, Naphthyl-Phenyl, Spirobifluorenyl, Spirobifluorenyl-Phenyl, Pyridyl, Pyrimidyl, Triazinyl, Dibenzofuranyl, Dibenzofuranyl-Phenyl, benzo-kondensiertem Dibenzofuranyl, Dibenzothiophenyl, Dibenzothiophenyl-Phenyl, benzo-kondensiertem Dibenzothiophenyl, Carbazolyl, Carbazolyl-Phenyl und benzo-kondensiertem Carbazolyl, und Kombinationen aus zwei, drei oder vier dieser Gruppen, wobei die genannten Gruppen jeweils mit einem oder mehreren Resten R⁴ substituiert sein können.

4. Verbindung gemäß einem oder mehreren der Ansprüche 1 bis 3, **dadurch gekennzeichnet, dass** R³ und R⁴ bei jedem Auftreten gleich oder verschieden gewählt sind aus H, D, F, CN, Si(R⁵)₃, N(R⁵)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁵ substituiert sein können; und wobei in den genannten Alkylgruppen eine oder mehrere CH₂-Gruppen durch -C=C-, -R⁵C=CR⁵-, Si(R⁵)₂, C=O, C=NR⁵, -NR⁵-, -O-, -S-, -C(=O)O- oder -C(=O)NR⁵- ersetzt sein können.

5. Verfahren zur Herstellung einer Verbindung gemäß einem oder mehreren der Ansprüche 1 bis 4, **dadurch gekennzeichnet, dass** in einem ersten Schritt i) ein Biphenyl-Derivat, das mit reaktiven Gruppen X und Y substituiert ist, wobei Gruppe X in ortho-Position zur Bindung zwischen den beiden Phenylgruppen vorliegt, mit einem aromatischen oder heteroaromatischen Ringsystem, das mit einer Boronsäuregruppe substituiert ist, umgesetzt wird, so dass das aromatische oder heteroaromatische Ringsystem in der Position der Gruppe Y eingeführt wird, und dass in einem zweiten Schritt ii) das in Schritt i) erhaltene Intermediat mit einer Verbindung der Formel HNAr₂ umgesetzt wird, wobei Ar gewählt ist aus aromatischen Ringsystemen und heteroaromatischen Ringsystemen, wobei in dieser Umsetzung die Gruppe -NAr₂ in der Position der Gruppe X eingeführt wird.

6. Oligomer, Polymer oder Dendrimer, enthaltend eine oder mehrere Verbindungen nach einem oder mehreren der Ansprüche 1 bis 4, wobei die Bindung(en) zum Polymer, Oligomer oder Dendrimer an beliebigen, in Formel (I) mit R³ oder R⁴ substituierten Positionen lokalisiert sein können.

7. Formulierung, enthaltend mindestens eine Verbindung nach einem oder mehreren der Ansprüche 1 bis 4 sowie mindestens ein Lösungsmittel.

8. Elektronische Vorrichtung, enthaltend mindestens eine Verbindung nach einem oder mehreren der Ansprüche 1 bis 4.

9. Elektronische Vorrichtung nach Anspruch 8, **dadurch gekennzeichnet, dass** sie eine organische Elektrolumineszenzvorrichtung ist, enthaltend Anode, Kathode und mindestens eine emittierende Schicht, wobei mindestens eine organische Schicht der Vorrichtung, die eine emittierende Schicht oder eine lochtransportierende Schicht sein kann, die mindestens eine Verbindung enthält.

10. Vorrichtung nach Anspruch 9, **dadurch gekennzeichnet, dass** sie mindestens eine Elektronenblockierschicht enthält, die mindestens eine Verbindung der Formel (I) enthält.

11. Verwendung einer Verbindung nach einem oder mehreren der Ansprüche 1 bis 4 in einer elektronischen Vorrichtung.

## Claims

1. Compound of the formula (I) 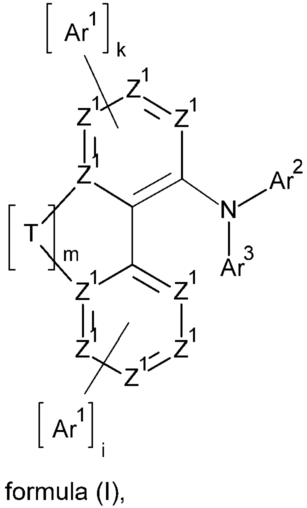 where the sub-unit 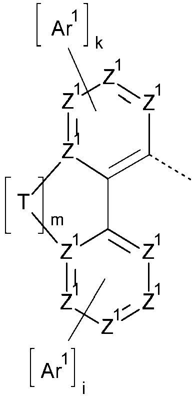
of the formula (I) is selected from the following structures:
| | | |
|---|---|---|
| 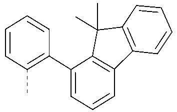 | | |
| formula (I-A-45) | | |
| 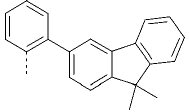 | 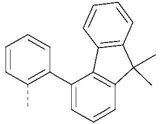 | |
| formula (I-A-46) | formula (I-A-47) | |
| 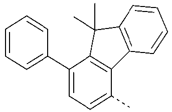 | 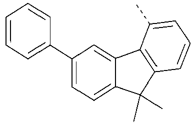 | 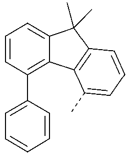 |
| formula (I-A-49) | formula (I-A-50) | formula (I-A-51) |
| 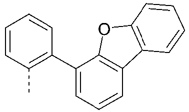 | | 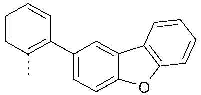 |
| formula (I-A-53) | | formula (I-A-54) |
| 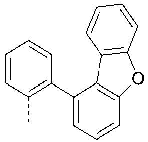 | | 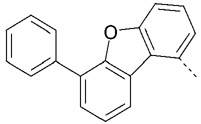 |
| formula (I-A-55) | | formula (I-A-57) |
| 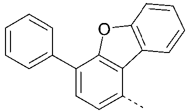 | 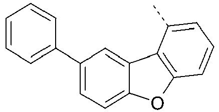 | 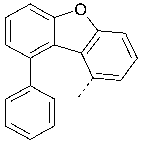 |
| formula (I-A-58) | formula (I-A-59) | formula (I-A-60) |
| 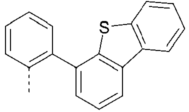 | | 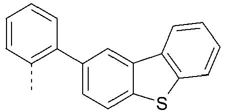 |
| formula (I-A-62) | | formula (I-A-63) |
| 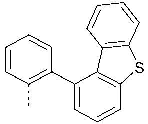 | | 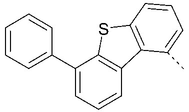 |
| formula (I-A-64) | | formula (I-A-66) |
| 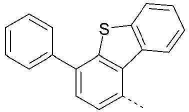 | 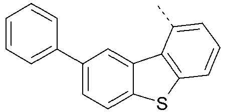 | 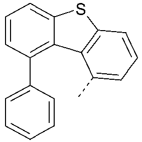 |
| formula (I-A-67) | formula (I-A-68) | formula (I-A-69) |
| 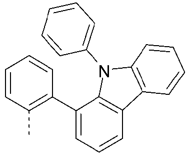 | | 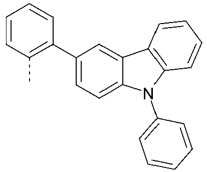 |
| formula (I-A-71) | | formula (I-A-72) |
| 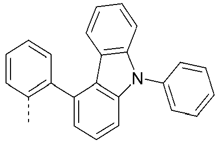 | | 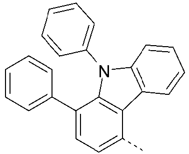 |
| formula (I-A-73) | | formula (I-A-75) |
| 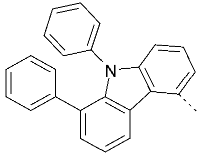 | 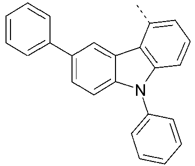 | 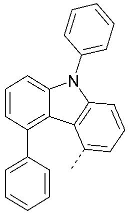 |
| formula (I-A-76) | formula (I-A-77) | formula (I-A-78) |
| 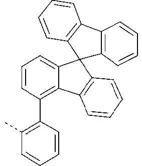 | | 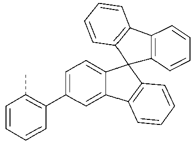 |
| formula (I-A-80) | | formula (I-A-81) |
| 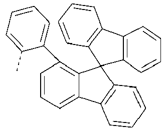 | | 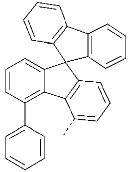 |
| formula (I-A-82) | | formula (I-A-84) |
| 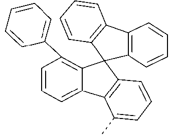 | 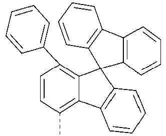 | 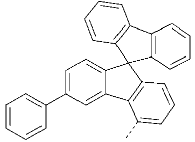 |
| formula (I-A-85) | formula (I-A-86) | formula (I-A-87) |
| 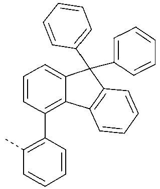 | | 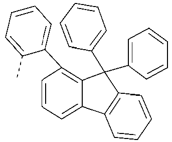 |
| formula (I-A-88) | | formula (I-A-90) |
| 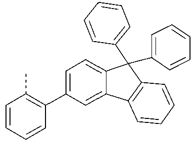 | | 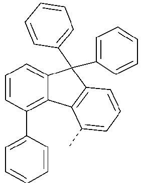 |
| formula (I-A-91) | | formula (I-A-93) |
| 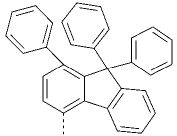 | 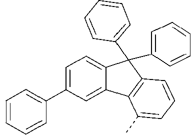 | 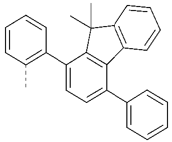 |
| formula (I-A-94) | formula (I-A-95) | formula (I-A-96) |
| 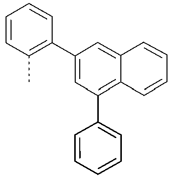 | | 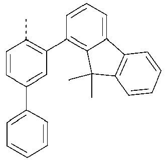 |
| formula (I-A-109) | | formula (I-A-111) |
| 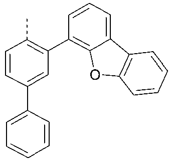 | 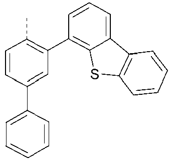 | 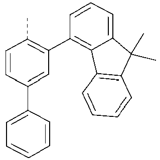 |
| formula (I-A-112) | formula (I-A-113) | formula (I-A-114) |
| 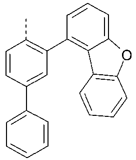 | 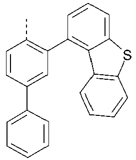 | |
| formula (I-A-115) | formula (I-A-116) | |
| 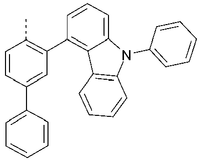 | 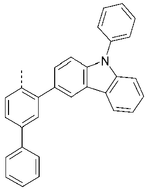 | |
| formula (I-A-118) | formula (I-A-119) | |
| 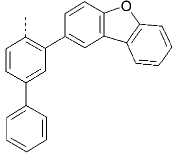 | 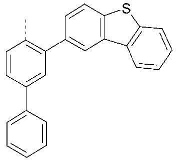 | |
| formula (I-A-121) | formula (I-A-122) | |
where the dashed line represents the bond to the N atom in formula (I), and where the following applies to the variables occurring:
Ar² corresponds to a formula (A) or (B) 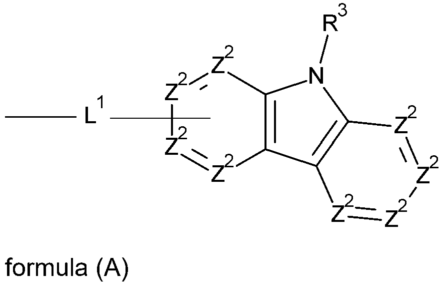 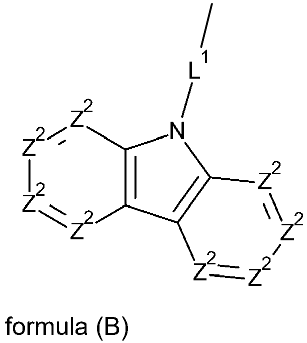
Z² is on each occurrence, identically or differently, CR³ or N, where Z² is equal to C if a group L¹ is bonded thereto;
L¹ is a single bond, or an aromatic ring system having 6 to 30 aromatic ring atoms, which may be substituted by one or more radicals R³, or a heteroaromatic ring system having 5 to 30 aromatic ring atoms, which may be substituted by one or more radicals R³;
Ar³ corresponds to a formula (A), a formula (B), or is an aromatic ring system having 6 to 30 aromatic ring atoms, which may be substituted by one or more radicals R⁴, or a heteroaromatic ring system having 5 to 30 aromatic ring atoms, which may be substituted by one or more radicals R⁴;
R³, R⁴ are selected on each occurrence, identically or differently, from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ and R⁴, respectively, may be linked to one another and may form a ring; where the said alkyl, alkoxy, alkenyl and alkynyl groups and the said aromatic ring systems and heteroaromatic ring systems may in each case be substituted by one or more radicals R⁵; and where one or more CH₂ groups in the said alkyl, alkoxy, alkenyl and alkynyl groups may be replaced by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂;
R⁵ is selected on each occurrence, identically or differently, from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁵ may be linked to one another and may form a ring; where the said alkyl, alkoxy, alkenyl and alkynyl groups and the said aromatic ring systems and heteroaromatic ring systems may in each case be substituted by one or more radicals R⁶; and where one or more CH₂ groups in the said alkyl, alkoxy, alkenyl and alkynyl groups may be replaced by -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO or SO₂;
R⁶ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁶ may be linked to one another and may form a ring; and where the said alkyl, alkoxy, alkenyl and alkynyl groups, aromatic ring systems and heteroaromatic ring systems may be substituted by F or CN.

2. Compound according to Claim 1, **characterised in that** Ar² is selected from groups of the following formulae
| | | |
|---|---|---|
| 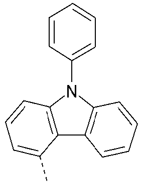 | 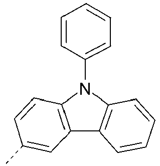 | 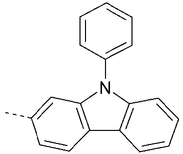 |
| Ar²-1 | Ar²-2 | Ar²-3 |
| 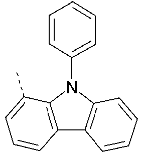 | 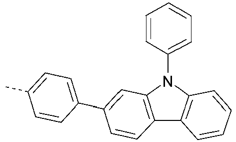 | 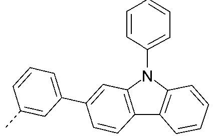 |
| Ar²-4 | Ar²-5 | Ar²-6 |
| 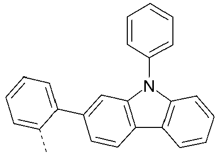 | 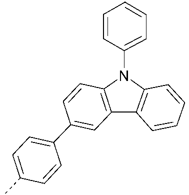 | 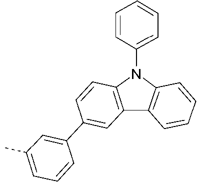 |
| Ar²-7 | Ar²-8 | Ar²-9 |
| 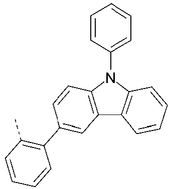 | 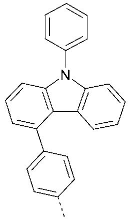 | 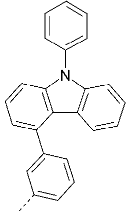 |
| Ar²-10 | Ar²-11 | Ar²-12 |
| 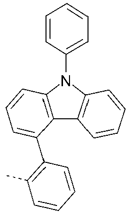 | 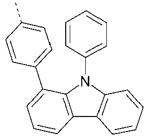 | 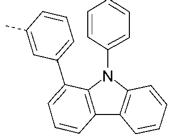 |
| Ar²-13 | Ar²-14 | Ar²-15 |
| 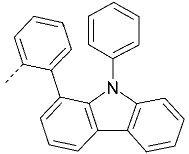 | 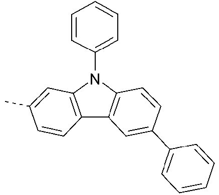 | 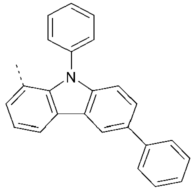 |
| Ar²-16 | Ar²-17 | Ar²-18 |
| 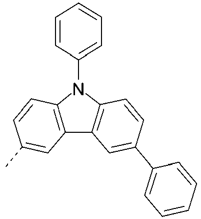 | 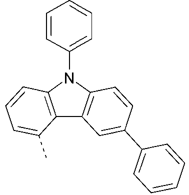 | 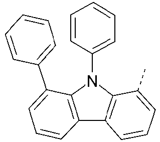 |
| Ar²-19 | Ar²-20 | Ar²-21 |
| 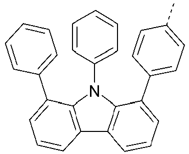 | 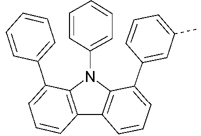 | 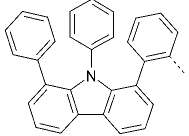 |
| Ar²-22 | Ar²-23 | Ar²-24 |
| 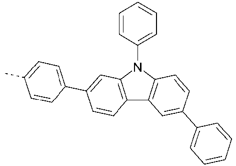 | 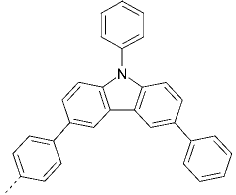 | 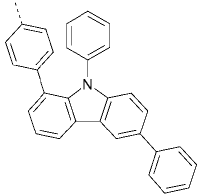 |
| Ar²-25 | Ar²-26 | Ar²-27 |
| 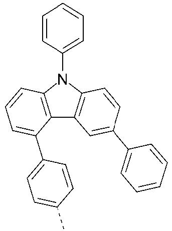 | 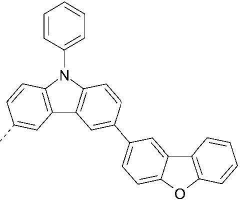 | 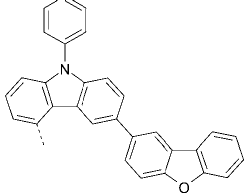 |
| Ar²-28 | Ar²-29 | Ar²-30 |
| 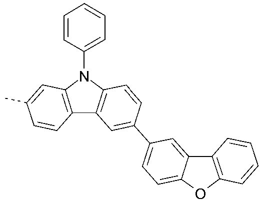 | 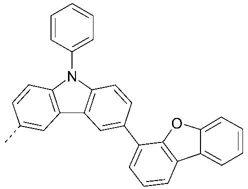 | 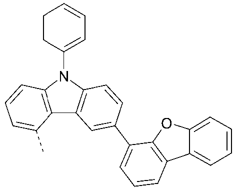 |
| Ar²-31 | Ar²-32 | Ar²-33 |
| 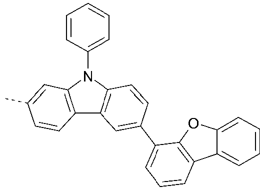 | 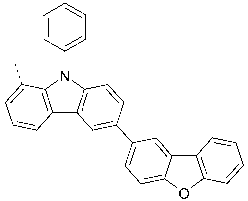 | 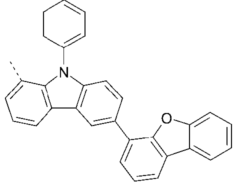 |
| Ar²-34 | Ar²-35 | Ar²-36 |
| 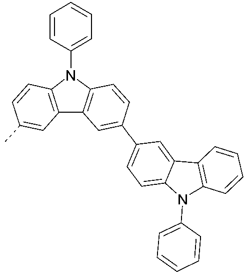 | 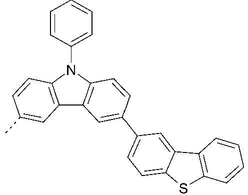 | 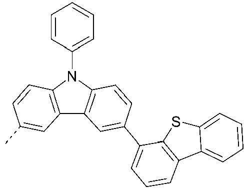 |
| Ar²-37 | Ar²-38 | Ar²-39 |
| 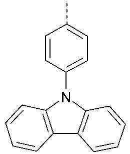 | 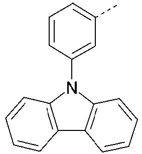 | 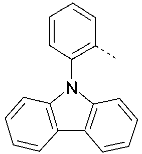 |
| Ar²-40 | Ar²-41 | Ar²-42 |
| 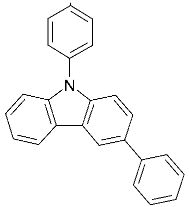 | 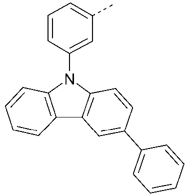 | 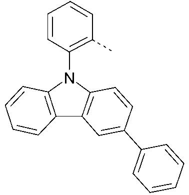 |
| Ar²-43 | Ar²-44 | Ar²-45 |
| 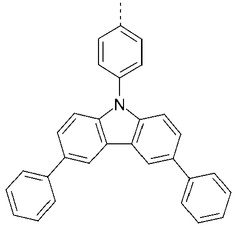 | 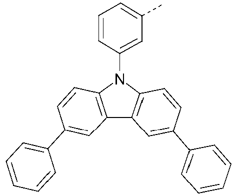 | 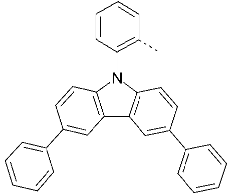 |
| Ar²-46 | Ar²-47 | Ar²-48 |
| 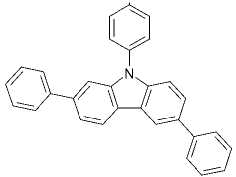 | 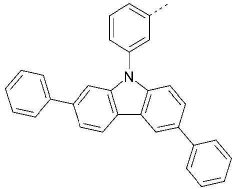 | 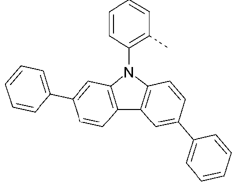 |
| Ar²-49 | Ar²-50 | Ar²-51 |
| 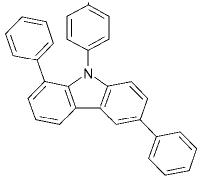 | 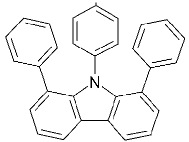 | 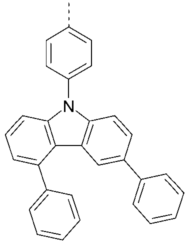 |
| Ar²-52 | Ar²-53 | Ar²-54 |
| 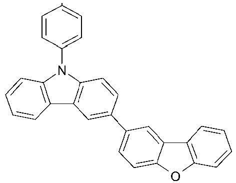 | 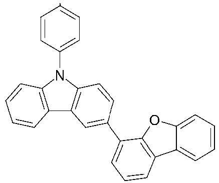 | 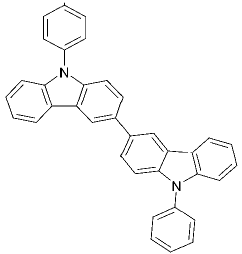 |
| Ar²-55 | Ar²-56 | Ar²-57 |
| 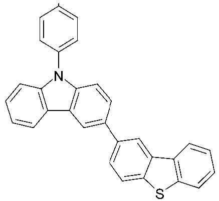 | 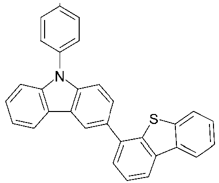 | 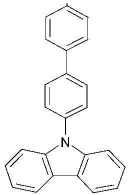 |
| Ar²-58 | Ar²-59 | Ar²-60 |
| 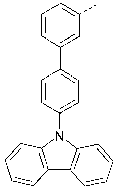 | 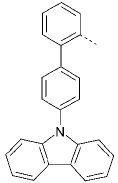 | 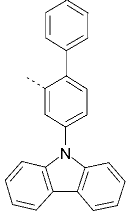 |
| Ar²-61 | Ar²-62 | Ar²-63 |
| 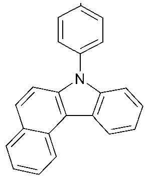 | 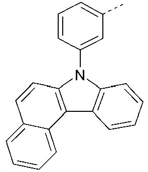 | 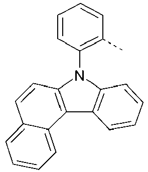 |
| Ar²-64 | Ar²-65 | Ar²-66 |
| 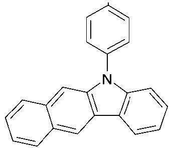 | 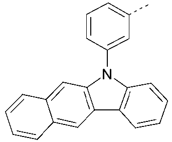 | 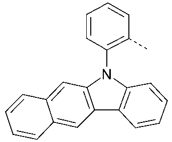 |
| Ar²-67 | Ar²-68 | Ar²-69 |
| 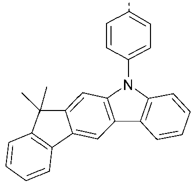 | 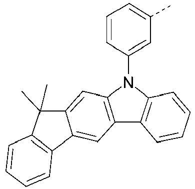 | 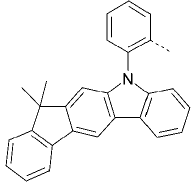 |
| Ar²-70 | Ar²-71 | Ar²-72 |
| 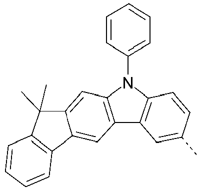 | 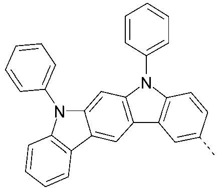 | 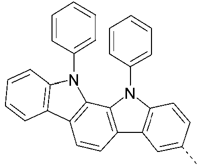 |
| Ar²-73 | Ar²-74 | Ar²-75 |
| 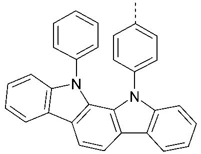 | | |
| Ar²-76 | | |

3. Compound according to Claim 1 or 2, **characterised in that** Ar³ is selected from phenyl, biphenyl, terphenyl, fluorenyl, fluorenyl-phenyl, naphthyl, naphthyl-phenyl, spirobifluorenyl, spirobifluorenyl-phenyl, pyridyl, pyrimidyl, triazinyl, dibenzofuranyl, dibenzofuranyl-phenyl, benzo-fused dibenzofuranyl, dibenzothiophenyl, dibenzothiophenyl-phenyl, benzo-fused dibenzothiophenyl, carbazolyl, carbazolyl-phenyl and benzo-fused carbazolyl, and combinations of two, three or four of these groups, where the said groups may in each case be substituted by one or more radicals R⁴.

4. Compound according to one or more of Claims 1 to 3, **characterised in that** R³ and R⁴ are selected on each occurrence, identically or differently, from H, D, F, CN, Si(R⁵)₃, N(R⁵)₂, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems may in each case be substituted by one or more radicals R⁵; and where one or more CH₂ groups in the said alkyl groups may be replaced by -C≡C-, -R⁵C=CR⁵-, Si(R⁵)₂, C=O, C=NR⁵, -NR⁵-, -O-, -S-, -C(=O)O- or -C(=O)NR⁵-.

5. Process for the preparation of a compound according to one or more of Claims 1 to 4, **characterised in that**, in a first step i), a biphenyl derivative which is substituted by reactive groups X and Y, where group X is present in the ortho position to the bond between the two phenyl groups, is reacted with an aromatic or heteroaromatic ring system which is substituted by a boronic acid group, so that the aromatic or heteroaromatic ring system is introduced in the position of group Y, and **in that**, in a second step ii), the intermediate obtained in step i) is reacted with a compound of the formula HNAr₂, where Ar is selected from aromatic ring systems and heteroaromatic ring systems, where the group -NAr₂ is introduced in the position of group X in this reaction.

6. Oligomer, polymer or dendrimer containing one or more compounds according to one or more of Claims 1 to 4, where the bond(s) to the polymer, oligomer or dendrimer can be localised at any desired positions in formula (I) that are substituted by R³ or R⁴.

7. Formulation comprising at least one compound according to one or more of Claims 1 to 4 and at least one solvent.

8. Electronic device containing at least one compound according to one or more of Claims 1 to 4.

9. Electronic device according to Claim 8, **characterised in that** it is an organic electroluminescent device comprising anode, cathode and at least one emitting layer, where at least one organic layer of the device, which can be an emitting layer or a hole-transporting layer, comprises the at least one compound.

10. Device according to Claim 9, **characterised in that** it comprises at least one electron-blocking layer which comprises at least one compound of the formula (I).

11. Use of a compound according to one or more of Claims 1 to 4 in an electronic device.

## Revendications

1. Composé de formule (I) 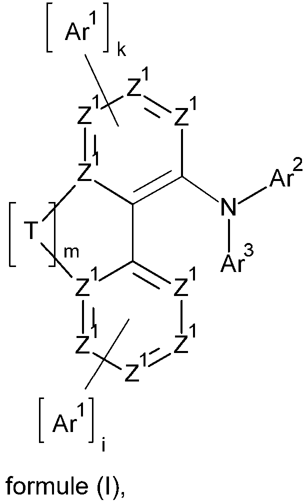 où la sous-unité 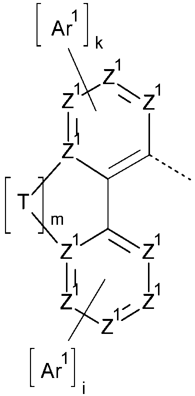
de formule (I) est choisie parmi les structures suivantes :
| | | |
|---|---|---|
| 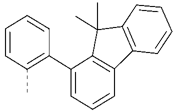 | | |
| formule (I-A-45) | | |
| 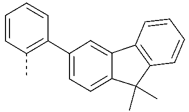 | 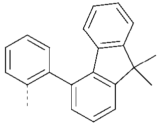 | |
| formule (I-A-46) | formule (I-A-47) | |
| 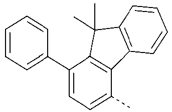 | 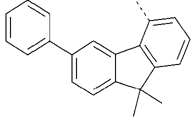 | 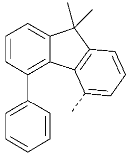 |
| formule (I-A-49) | formule (I-A-50) | formule (I-A-51) |
| 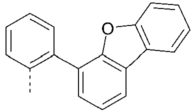 | | 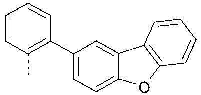 |
| formule (I-A-53) | | formule (I-A-54) |
| 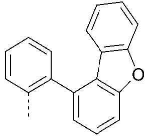 | | 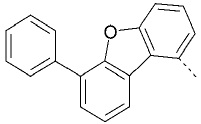 |
| formule (I-A-55) | | formule (I-A-57) |
| 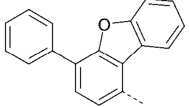 | 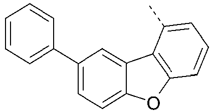 | 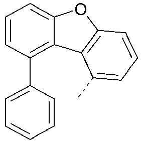 |
| formule (I-A-58) | formule (I-A-59) | formule (I-A-60) |
| 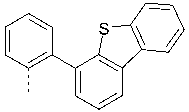 | | 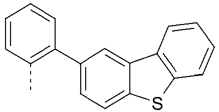 |
| formule (I-A-62) | | formule (I-A-63) |
| 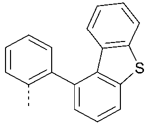 | | 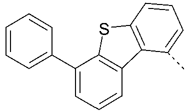 |
| formule (I-A-64) | | formule (I-A-66) |
| 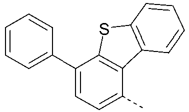 | 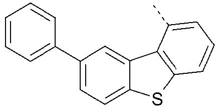 | 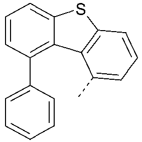 |
| formule (I-A-67) | formule (I-A-68) | formule (I-A-69) |
| 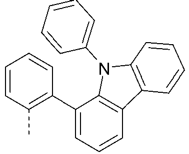 | | 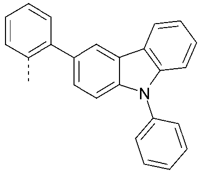 |
| formule (I-A-71) | | formule (I-A-72) |
| 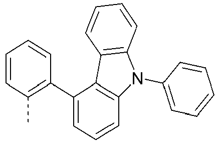 | | 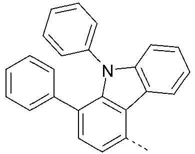 |
| formule (I-A-73) | | formule (I-A-75) |
| 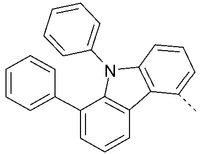 | 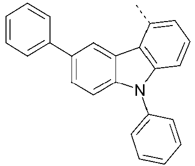 | 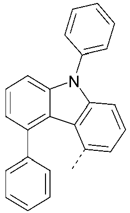 |
| formule (I-A-76) | formule (I-A-77) | formule (I-A-78) |
| 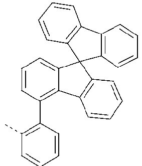 | | 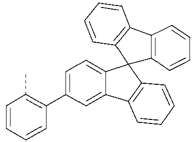 |
| formule (I-A-80) | | formule (I-A-81) |
| 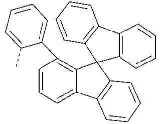 | | 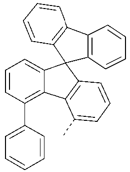 |
| formule (I-A-82) | | formule (I-A-84) |
| 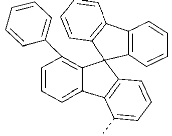 | 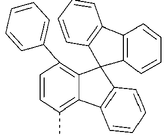 | 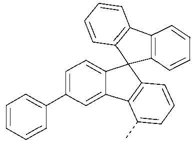 |
| formule (I-A-85) | formule (I-A-86) | formule (I-A-87) |
| 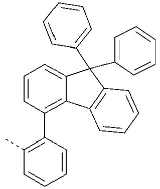 | | 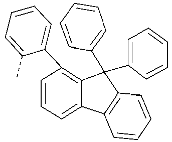 |
| formule (I-A-88) | | formule (I-A-90) |
| 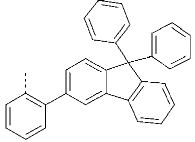 | | 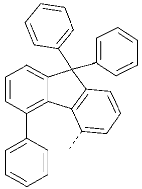 |
| formule (I-A-91) | | formule (I-A-93) |
| 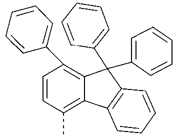 | 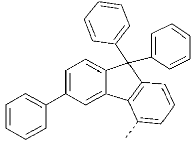 | 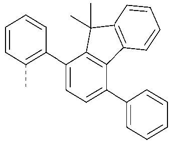 |
| formule (I-A-94) | formule (I-A-95) | formule (I-A-96) |
| 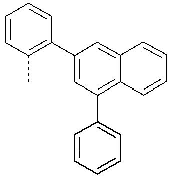 | | 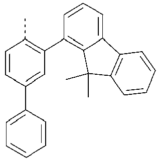 |
| formule (I-A-109) | | formule (I-A-111) |
| 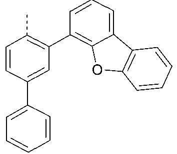 | 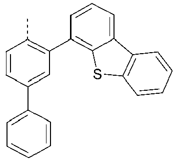 | 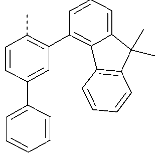 |
| formule (I-A-112) | formule (I-A-113) | formule (I-A-114) |
| 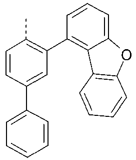 | 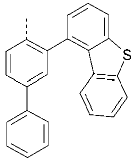 | |
| formule (I-A-115) | formule (I-A-116) | |
| 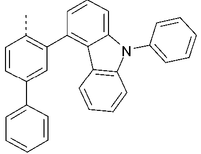 | 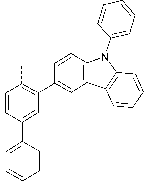 | |
| formule (I-A-118) | formule (I-A-119) | |
| 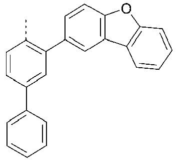 | 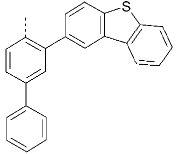 | |
| formule (I-A-121) | formule (I-A-122) | |
dans lesquelles la ligne en pointillés représente la liaison à l'atome de N dans la formule (I), et où ce qui suit s'applique aux variables présentes :
Ar² correspond à une formule (A) ou (B) 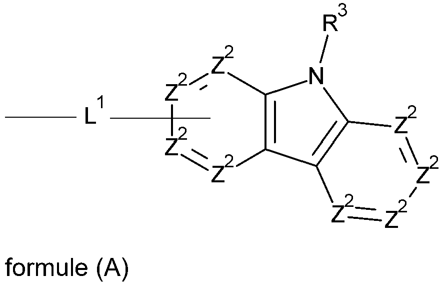 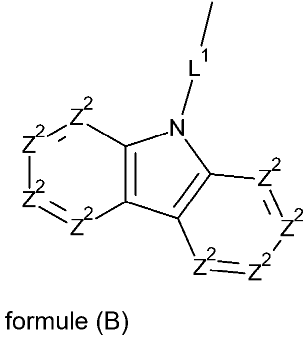
Z² est à chaque occurrence, de manière identique ou différente, CR³ ou N, où Z² est égal à C si un groupement L¹ y est lié ;
L¹ est une liaison simple, ou un noyau aromatique ayant de 6 à 30 atomes de cycle aromatique, qui peut être substitué par un ou plusieurs radicaux R³, ou un noyau hétéroaromatique ayant de 5 à 30 atomes de cycle aromatique, qui peut être substitué par un ou plusieurs radicaux R³ ;
Ar³ correspond à une formule (A), une formule (B), ou est un noyau aromatique ayant de 6 à 30 atomes de cycle aromatique, qui peut être substitué par un ou plusieurs radicaux R⁴, ou un noyau hétéroaromatique ayant de 5 à 30 atomes de cycle aromatique, qui peut être substitué par un ou plusieurs radicaux R⁴ ;
R³, R⁴ sont choisis à chaque occurrence, de manière identique ou différente, parmi H, D, F, C(=O)R⁵, CN, Si(R⁵)₃, N(R⁵)₂, P(=O)(R⁵)₂, OR⁵, S(=O)R⁵, S(=O)₂R⁵, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R³ et R⁴, respectivement, peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁵ ; et où un ou plusieurs groupements CH₂ dans lesdits groupements alkyle, alcoxy, alcényle et alcynyle peuvent être remplacés par -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO ou SO₂ ;
R⁵ est choisi à chaque occurrence, de manière identique ou différente, parmi H, D, F, C(=O)R⁶, CN, Si(R⁶)₃, N(R⁶)₂, P(=O)(R⁶)₂, OR⁶, S(=O)R⁶, S(=O)₂R⁶, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁵ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁶ ; et où un ou plusieurs groupements CH₂ dans lesdits groupements alkyle, alcoxy, alcényle et alcynyle peuvent être remplacés par -R⁶C=CR⁶-, -C=C-, Si(R⁶)₂, C=O, C=NR⁶, -C(=O)O-, - C(=O)NR⁶-, NR⁶, P(=O)(R⁶), -O-, -S-, SO ou SO₂ ;
R⁶ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁶ peuvent être liés les uns aux autres et peuvent former un cycle ; et où lesdits groupements alkyle, alcoxy, alcényle et alcynyle, noyaux aromatiques et noyaux hétéroaromatiques peuvent être substitués par F ou CN.

2. Composé selon la revendication 1, **caractérisé en ce que** Ar² est choisi parmi les groupements de formules suivantes
| | | |
|---|---|---|
| 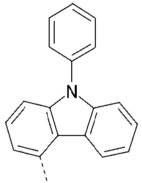 | 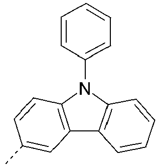 | 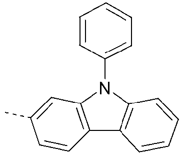 |
| Ar²-1 | Ar²-2 | Ar²-3 |
| 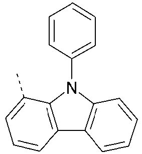 | 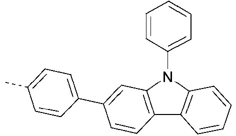 | 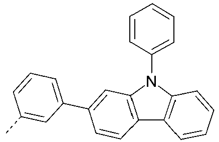 |
| Ar²-4 | Ar²-5 | Ar²-6 |
| 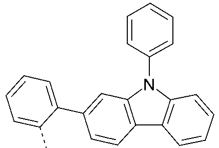 | 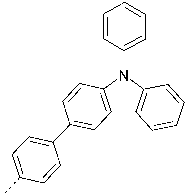 | 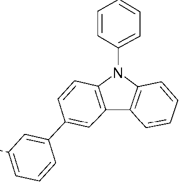 |
| Ar²-7 | Ar²-8 | Ar²-9 |
| 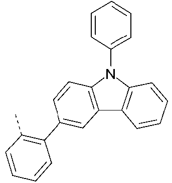 | 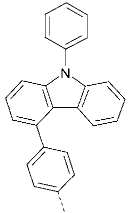 | 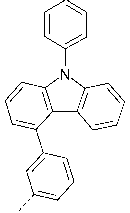 |
| Ar²-10 | Ar²-11 | Ar²-12 |
| 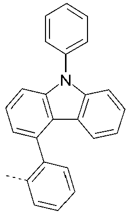 | 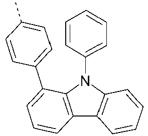 | 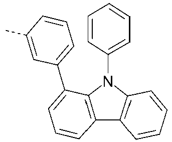 |
| Ar²-13 | Ar²-14 | Ar²-15 |
| 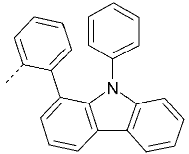 | 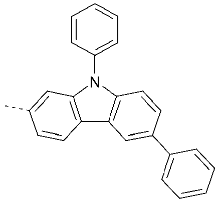 | 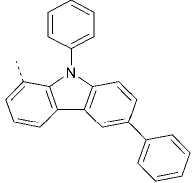 |
| Ar²-16 | Ar²-17 | Ar²-18 |
| 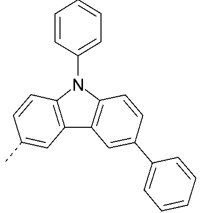 | 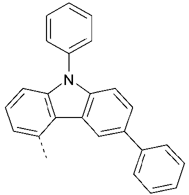 | 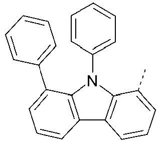 |
| Ar²-19 | Ar²-20 | Ar²-21 |
| 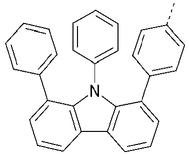 | 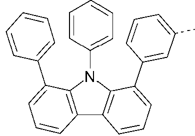 | 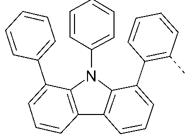 |
| Ar²-22 | Ar²-23 | Ar²-24 |
| 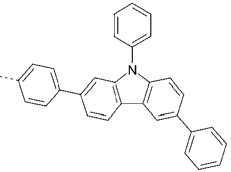 | 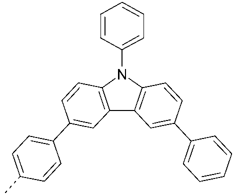 | 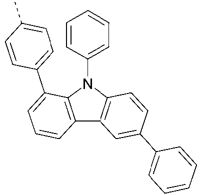 |
| Ar²-25 | Ar²-26 | Ar²-27 |
| 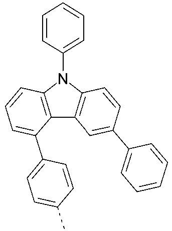 | 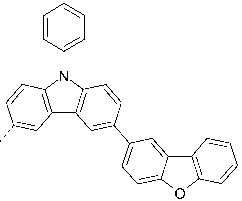 | 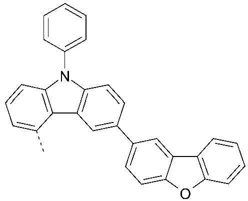 |
| Ar²-28 | Ar²-29 | Ar²-30 |
| 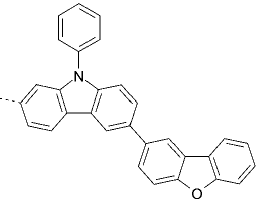 | 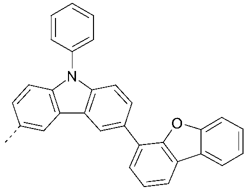 | 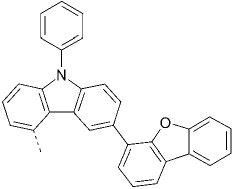 |
| Ar²-31 | Ar²-32 | Ar²-33 |
| 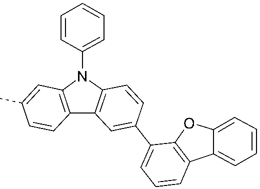 | 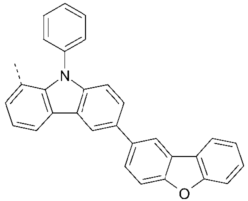 | 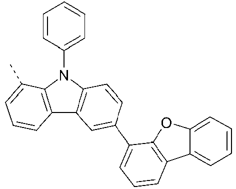 |
| Ar²-34 | Ar²-35 | Ar²-36 |
| 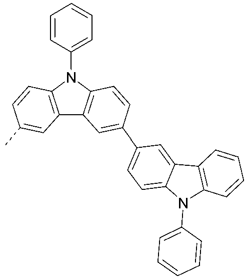 | 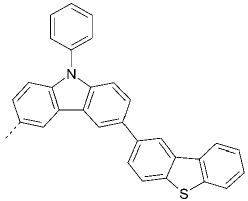 | 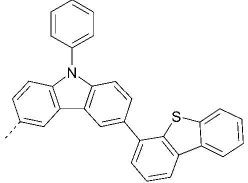 |
| Ar²-37 | Ar²-38 | Ar²-39 |
| 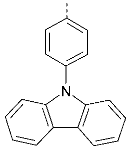 | 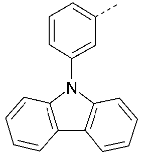 | 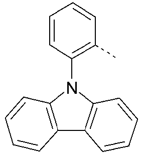 |
| Ar²-40 | Ar²-41 | Ar²-42 |
| 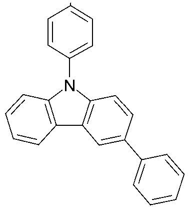 | 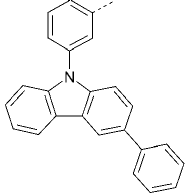 | 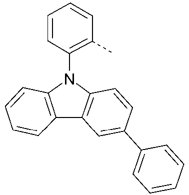 |
| Ar²-43 | Ar²-44 | Ar²-45 |
| 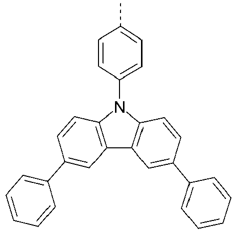 | 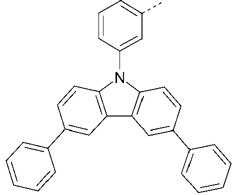 | 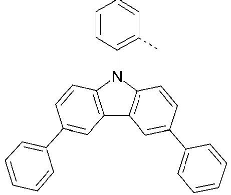 |
| Ar²-46 | Ar²-47 | Ar²-48 |
| 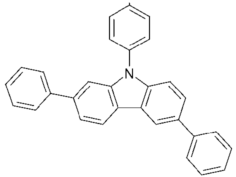 | 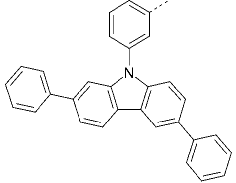 | 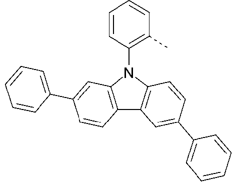 |
| Ar²-49 | Ar²-50 | Ar²-51 |
| 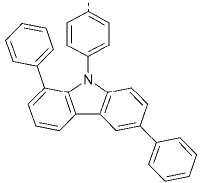 | 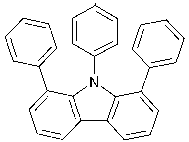 | 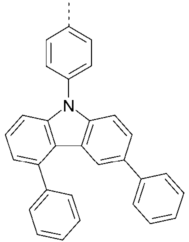 |
| Ar²-52 | Ar²-53 | Ar²-54 |
| 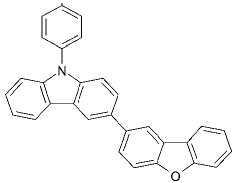 | 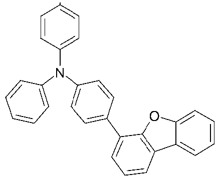 | 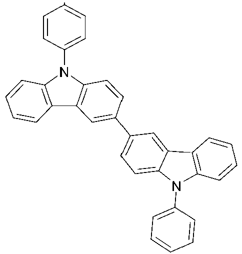 |
| Ar²-55 | Ar²-56 | Ar²-57 |
| 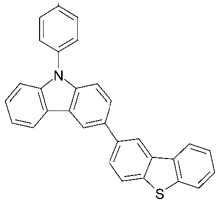 | 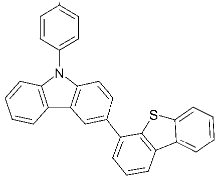 | 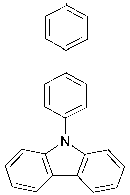 |
| Ar²-58 | Ar²-59 | Ar²-60 |
| 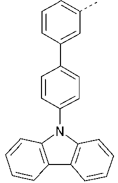 | 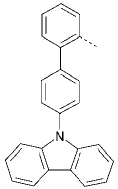 | 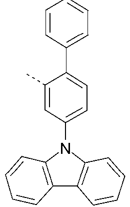 |
| Ar²-61 | Ar²-62 | Ar²-63 |
| 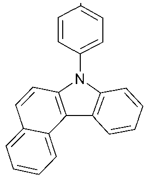 | 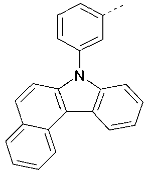 | 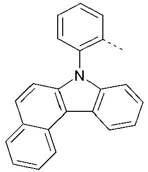 |
| Ar²-64 | Ar²-65 | Ar²-66 |
| 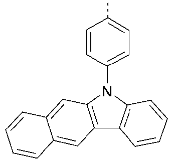 | 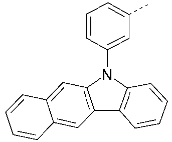 | 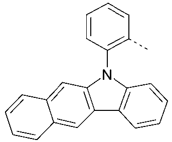 |
| Ar²-67 | Ar²-68 | Ar²-69 |
| 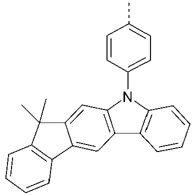 | 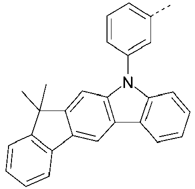 | 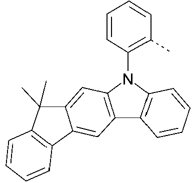 |
| Ar²-70 | Ar²-71 | Ar²-72 |
| 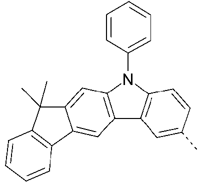 | 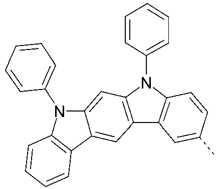 | 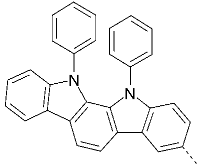 |
| Ar²-73 | Ar²-74 | Ar²-75 |
| 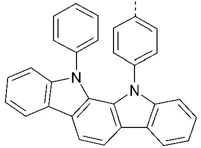 | | |
| Ar²-76 | | |

3. Composé selon la revendication 1 ou 2, **caractérisé en ce que** Ar³ est choisi parmi phényle, biphényle, terphényle, fluorényle, fluorényl-phényle, naphtyle, naphtyl-phényle, spirobifluorényle, spirobifluorényl-phényle, pyridyle, pyrimidyle, triazinyle, dibenzofuranyle, dibenzofuranyl-phényle, dibenzofuranyle benzo-condensé, dibenzothiophényle, dibenzothiophényl-phényle, dibenzothiophényle benzo-condensé, carbazolyle, carbazolyl-phényle et carbazolyle benzo-condensé, et des combinaisons de deux, trois ou quatre parmi ces groupements, où lesdits groupements peuvent dans chaque cas être substitués par un ou plusieurs radicaux R⁴.

4. Composé selon l'une ou plusieurs parmi les revendications 1 à 3, **caractérisé en ce que** R³ et R⁴ sont choisis à chaque occurrence, de manière identique ou différente, parmi H, D, F, CN, Si(R⁵)₃, N(R⁵)₂, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques peuvent dans chaque cas être substitués par un ou plusieurs radicaux R⁵ ; et où un ou plusieurs groupements CH₂ dans lesdits groupements alkyle peuvent être remplacés par -C≡C-, -R⁵C=CR⁵-, Si(R⁵)₂, C=O, C=NR⁵, -NR⁵-, -O-, -S-, -C(=O)O- ou -C(=O)NR⁵-.

5. Procédé de préparation d'un composé selon l'une ou plusieurs parmi les revendications 1 à 4, **caractérisé en ce que**, dans une première étape i), un dérivé biphényle qui est substitué par des groupements réactifs X et Y, où le groupement X est présent en position ortho par rapport à la liaison entre les deux groupements phényle, est réagi avec un noyau aromatique ou hétéroaromatique qui est substitué par un groupement acide boronique, de sorte que le noyau aromatique ou hétéroaromatique soit introduit dans la position du groupement Y, et **en ce que**, dans une deuxième étape ii), l'intermédiaire obtenu dans l'étape i) est réagi avec un composé de formule HNAr₂, où Ar est choisi parmi des noyaux aromatiques et des noyaux hétéroaromatiques, où le groupement -NAr₂ est introduit dans la position du groupement X dans cette réaction.

6. Oligomère, polymère ou dendrimère contenant un ou plusieurs composés selon l'une ou plusieurs parmi les revendications 1 à 4, la ou les liaisons au polymère, à l'oligomère ou au dendrimère pouvant être situées au niveau de positions désirées quelconques dans la formule (I) qui sont substituées par R³ ou R⁴.

7. Formulation comprenant au moins un composé selon l'une ou plusieurs parmi les revendications 1 à 4 et au moins un solvant.

8. Dispositif électronique contenant au moins un composé selon l'une ou plusieurs parmi les revendications 1 à 4.

9. Dispositif électronique selon la revendication 8, **caractérisé en ce qu'**il s'agit d'un dispositif électroluminescent organique comprenant une anode, une cathode et au moins une couche émettrice, où au moins une couche organique du dispositif, qui peut être une couche émettrice ou une couche de transport de trous, comprend le au moins un composé.

10. Dispositif selon la revendication 9, **caractérisé en ce qu'**il comprend au moins une couche de blocage d'électrons qui comprend au moins un composé de formule (I).

11. Utilisation d'un composé selon l'une ou plusieurs parmi les revendications 1 à 4, dans un dispositif électronique.
